(19) Europäisches Patentamt
European Patent Office
Office européen des brevets

(11) EP 4 014 958 A1

(12) EUROPEAN PATENT APPLICATION

(43) Date of publication:
**22.06.2022 Bulletin 2022/25**

(21) Application number: **22155607.9**

(22) Date of filing: **28.06.2019**

(51) International Patent Classification (IPC):
***A61K 8/64*** (2006.01) ***A61Q 5/12*** (2006.01)
***A61Q 19/00*** (2006.01) ***A61K 8/36*** (2006.01)
***A61K 8/362*** (2006.01) ***A61K 8/365*** (2006.01)
***A61Q 1/02*** (2006.01) ***A61Q 5/02*** (2006.01)
***A61Q 5/06*** (2006.01) ***A61Q 19/10*** (2006.01)
***C07K 5/06*** (2006.01) ***C09D 177/04*** (2006.01)

(52) Cooperative Patent Classification (CPC):
**A61K 8/362; A61K 8/361; A61K 8/365; A61K 8/64; A61Q 5/12; A61Q 19/00; C07K 5/06; C09D 177/04;**
A61Q 1/02; A61Q 5/02; A61Q 5/06; A61Q 19/10

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **29.06.2018 JP 2018125136**
**13.12.2018 JP 2018233469**

(62) Document number(s) of the earlier application(s) in accordance with Art. 76 EPC:
**19827474.8 / 3 808 329**

(71) Applicant: **Nissan Chemical Corporation**
**Tokyo 103-6119 (JP)**

(72) Inventors:
- **Imoto, Takayuki**
**Funabashi-shi, Chiba 274-0052 (JP)**
- **Sakata, Mizuki**
**Funabashi-shi, Chiba 274-0052 (JP)**
- **Katsuya, Mutsuhiro**
**Funabashi-shi, Chiba 274-0052 (JP)**
- **Shoji, Takeaki**
**Funabashi-shi, Chiba 274-0052 (JP)**

(74) Representative: **Hoffmann Eitle**
**Patent- und Rechtsanwälte PartmbB**
**Arabellastraße 30**
**81925 München (DE)**

Remarks:
This application was filed on 08.02.2022 as a divisional application to the application mentioned under INID code 62.

# (54) COATING FILM-FORMING COMPOSITION

(57) There is provided a composition that can more readily form a coating film exhibiting excellent moisture-retaining property.

A coating film-forming composition characterized by comprising at least one lipidic peptide compound composed of a low-molecular-weight lipidic peptide or a pharmaceutically usable salt thereof.

The lipidic peptide compound may preferably be a compound prepared by bonding of a peptide moiety having an amino acid repeating structure to a lipidic moiety having a $C_{9-23}$ aliphatic group.

EP 4 014 958 A1

**Description**

TECHNICAL FIELD

The present invention relates to a coating film-forming composition, and more particularly to a coating film-forming composition containing at least one lipidic peptide compound.

BACKGROUND ART

In the fields of cosmetic products and hairdressings, a coating film formed on the skin or the hair surface plays various important roles (e.g., suppression of transpiration of moisture from the skin or hair, and enhancement of retention of active ingredients) in personal care products, since the coating film provides the skin or the hair surface with an effective barrier.

In recent years, a demand has arisen for a cosmetic composition exhibiting a high moisturizing effect in association with increased awareness of health, in particular, increased awareness of dry skin. Examples of skincare products exhibiting a high moisturizing effect include coating film-forming cosmetic products based on a self-assembled structure, such as cosmetic products based on a lamellar $\alpha$-gel structure (Patent Document 1). Horny layer intercellular lipid present in a horny layer, which is the outermost skin layer, also has a self-assembled lamellar structure, and thus suppresses invasion of foreign substances into the skin or transpiration of moisture from the skin, thereby exhibiting moisture-retaining property or a function of maintaining the softness of skin.

There has been proposed a hair care preparation containing a polypeptide in order to reduce damage to hair caused by brushing or thermal treatment with, for example, a dryer (Patent Documents 2 and 3).

Prior Art Documents

Patent Documents

Patent Document 1: Japanese Unexamined Patent Application Publication No. 2016-6030 (JP 2016-6030 A)
Patent Document 2: Japanese Unexamined Patent Application Publication No. H10-77210 (JP 1998-77210 A)
Patent Document 3: Japanese Unexamined Patent Application Publication No. 2002-308756 (JP 2002-308756 A)

SUMMARY OF THE INVENTION

Problems to be Solved by the Invention

Formation of the aforementioned lamellar $\alpha$-gel structure requires mixing of a plurality of ingredients in specific proportions. Thus, when the lamellar $\alpha$ -gel structure is used in combination with an additional ingredient, a cumbersome process may be required for adjusting the mixing proportions of the plurality of ingredients and the additional ingredient.

The use of a polymer such as polypeptide in a coating film-forming agent results in skin tightness caused by the polymer. Thus, such an agent has been required to exhibit an improved sensation in use.

An object of the present invention is to provide a composition that can more readily form a coating film exhibiting excellent moisture-retaining property.

Another object of the present invention is to provide a composition that can form a coating film causing almost no skin tightness and exhibiting an improved sensation in use.

Means for Solving the Problems

The present inventors have conducted extensive studies for solving the aforementioned problems, and as a result have found that a coating film is formed on the skin or the hair surface only by using at least one lipidic peptide compound, and the resultant coating film achieves the following: 1. excellent moisture-retaining property; 2. suppression of adsorption of moisture; 3. promotion of permeation of an active ingredient into hair; 4. adsorption of a negatively charged ingredient; and 5. suppression of conjugation of oil ingredients. The present invention has been accomplished on the basis of this finding.

The present inventors have also found that the aforementioned coating film causes almost no skin tightness and exhibits a good sensation in use. The present invention has been accomplished also on the basis of this finding.

Accordingly, a first aspect of the present invention is a coating film-forming composition characterized by

comprising at least one lipidic peptide compound composed of a low-molecular-weight lipidic peptide or a pharmaceutically usable salt thereof.

A second aspect of the present invention is the coating film-forming composition according to the first aspect, wherein the lipidic peptide compound is a compound prepared by bonding of a peptide moiety having an amino acid repeating structure to a lipidic moiety having a $C_{9-23}$ aliphatic group.

A third aspect of the present invention is the coating film-forming composition according to the second aspect, characterized in that the lipidic peptide compound is composed of at least one of compounds of the following Formulae (1) to (3) or pharmaceutically usable salts of the compounds:

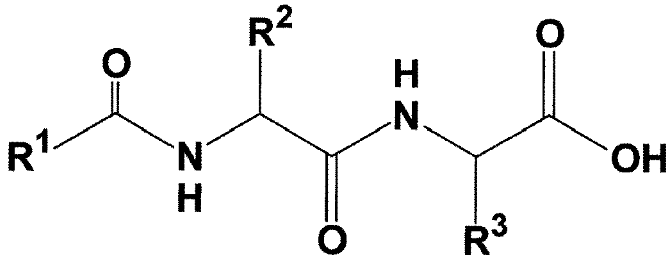

(1)

(wherein $R^1$ is a $C_{9-23}$ aliphatic group; $R^2$ is a hydrogen atom or a $C_{1-4}$ alkyl group optionally having a $C_1$ or $C_2$ branched chain; and $R^3$ is a $-(CH_2)_n-X$ group, wherein n is a number of 1 to 4, and X is an amino group, a guanidino group, a $-CONH_2$ group, or a 5-membered ring or a 6-membered ring optionally having one to three nitrogen atoms or a fused heterocyclic ring composed of the 5-membered ring and the 6-membered ring);

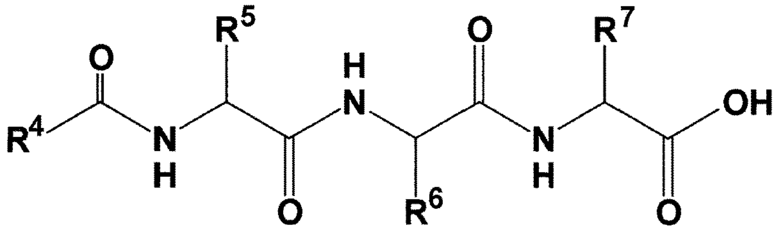

(2)

(wherein $R^4$ is a $C_{9-23}$ aliphatic group; and $R^5$ to $R^7$ are each independently a hydrogen atom, a $C_{1-4}$ alkyl group optionally having a $C_1$ or $C_2$ branched chain, or a $-(CH_2)_n-X$ group, wherein n is a number of 1 to 4, and X is an amino group, a guanidino group, a $-CONH_2$ group, or a 5-membered ring or a 6-membered ring optionally having one to three nitrogen atoms or a fused heterocyclic ring composed of the 5-membered ring and the 6-membered ring); and

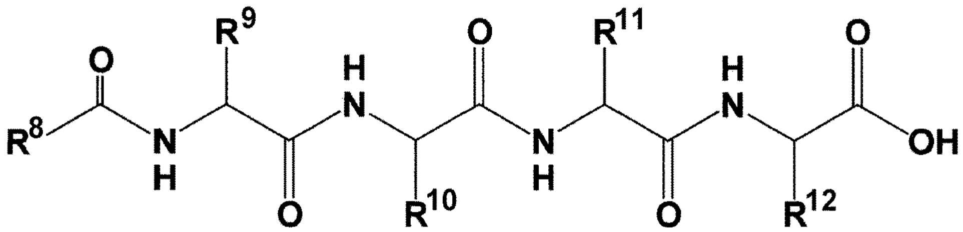

(3)

(wherein $R^8$ is a $C_{9-23}$ aliphatic group; and $R^9$ to $R^{12}$ are each independently a hydrogen atom, a $C_{1-4}$ alkyl group optionally having a $C_1$ or $C_2$ branched chain, or a $-(CH_2)_n-X$ group, wherein n is a number of 1 to 4, and X is an amino group, a guanidino group, a $-CONH_2$ group, or a 5-membered ring or a 6-membered ring optionally having one to three nitrogen atoms or a fused heterocyclic ring composed of the 5-membered ring and the 6-membered ring).

A fourth aspect of the present invention is a method for producing a coating film, the method being characterized by comprising using the coating film-forming composition according to any one of the first to third aspects.

A fifth aspect of the present invention is a coating film formed from the coating film-forming composition according to any one of the first to third aspects.

## Effects of the Invention

According to the present invention, the composition containing at least one lipidic peptide compound can form a coating film exhibiting excellent moisture-retaining property. Thus, since the proportions of ingredients in the composition are not required to be adjusted overly, a coating film exhibiting excellent moisture-retaining property can be more readily formed.

According to the present invention, incorporation of the composition into, for example, a cosmetic product can provide the cosmetic product with excellent moisture-retaining property.

Furthermore, the composition of the present invention can form a coating film causing almost no skin tightness and exhibiting a good sensation in use.

Also, the composition of the present invention can form a coating film exhibiting at least one effect selected from the following effects: an effect of reducing light reflection, a water repellent effect, an effect of achieving smooth finger passage or smooth combing, an effect of preventing makeup deterioration, an effect of imparting a gloss, and an effect of highly retaining an active ingredient (possibly containing a large amount of an active ingredient).

The lipidic peptide compound used in the present invention is a highly safe artificial low-molecular-weight compound that is composed of a lipid and a peptide only. Thus, the composition of the present invention has high biological safety, and is particularly useful in, for example, pharmaceutical products and cosmetic products.

## BRIEF DESCRIPTION OF THE DRAWINGS

[FIG. 1] FIG. 1 shows scanning electron microscope (SEM) images in Example 1 [(a) SEM image of a sample immediately after being set in the scanning electron microscope (numbers in the figure correspond to the positions of typical mineral oil droplets), and (b) SEM image of the sample after being dried (numbers in the figure correspond to the positions of the mineral oil droplets shown in (a))].

[FIG. 2] FIG. 2 shows appearance photographs in Example 3 and Comparative Example 3 [(a) appearance photograph in Example 3, and (b) appearance photograph in Comparative Example 3].

[FIG. 3] FIG. 3 shows scanning electron microscope (SEM) images in Example 3 and Comparative Example 3 [(a) SEM image in Example 3, and (b) SEM image in Comparative Example 3].

[FIG. 4] FIG. 4 shows the results of a moisturizing test of a shampoo (rate of moisture retention in hair) in Examples 4 to 8 and Comparative Example 4.

[FIG. 5] FIG. 5 shows the results of measurement of contact angle in Example 9 and Comparative Example 5 [(a) the results of measurement of contact angle in Example 9, and (b) the results of measurement of contact angle in Comparative Example 5].

[FIG. 6] FIG. 6 shows the results of a moisturizing test of a conditioner (rate of moisture retention in hair) in Example 10 and Comparative Example 6.

[FIG. 7] FIG. 7 shows scanning electron microscope (SEM) images of hair after the moisturizing test of the conditioner in Example 10 and Comparative Example 6 [(a) SEM image in Example 10, and (b) SEM image in Comparative Example 6].

[FIG. 8] FIG. 8 shows the results of measurement of frictional resistance in Example 11 and Comparative Example 7.

[FIG. 9] FIG. 9 shows the results of measurement of reflected light intensity in Example 13 and Comparative Example 9.

[FIG. 10] FIG. 10 shows appearance photographs in Example 14 and Comparative Example 10 [(a) appearance photograph in Comparative Example 10, and (b) appearance photograph in Example 14].

[FIG. 11] FIG. 11 shows the results of quantification of malic acid in Example 18 and Comparative Example 14.

[FIG. 12] FIG. 12 shows scanning electron microscope (SEM) images of hair after treatment with a conditioner in Example 19, Comparative Example 15, and damaged hair [(a) SEM image of damaged hair, (b) SEM image in Example 19, and (c) SEM image in Comparative Example 15].

[FIG. 13] FIG. 13 shows the results of measurement of dynamic friction coefficient in Example 21 and Comparative Example 17.

[FIG. 14] FIG. 14 shows the results of quantification of succinic acid in hair treated with a conditioner in Example 23 and Comparative Example 19.

[FIG. 15] FIG. 15 shows the results of evaluation of the amount of succinic acid and lipidic peptide permeating into human hair in Example 24 and Comparative Example 20.

[FIG. 16] FIG. 16 shows the results of evaluation of the hardness of the surface of hair treated with a conditioner in Example 26, Comparative Example 22, black hair, and damaged hair.

[FIG. 17] FIG. 17 shows the results of evaluation of adsorption or desorption of moisture after treatment with a

conditioner in Example 27, Comparative Example 23, and damaged hair.

[FIG. 18] FIG. 18 shows the results of observation of time-course change in shine by sebum floating in Example 28 and Comparative Example 24.

[FIG. 19] FIG. 19 shows the moisturizing effect of a beauty essence in Example 30 and Comparative Example 26.

[FIG. 20] FIG. 20 shows the results of quantification of keratin in hair treated with a conditioner in Example 33, Example 34, and Comparative Example 28.

[FIG. 21] FIG. 21 shows scanning electron microscope (SEM) images of hair after treatment with a conditioner in Example 36, Comparative Example 30, and damaged hair [(a) SEM image in Example 36, (b) SEM image in Comparative Example 30, and (c) SEM image of damaged hair].

MODES FOR CARRYING OUT THE INVENTION

The present invention is directed to a coating film-forming composition characterized by comprising at least one lipidic peptide compound composed of a low-molecular-weight lipidic peptide or a pharmaceutically usable salt thereof.

The coating film-forming composition according to the present invention generally refers to a composition that utilizes its coating film formability to provide industrial application values, and particularly refers to a composition for forming a coating film on a target; i.e., the surface of skin or hair. However, no other limitation is imposed on the application of the composition to, for example, paints, coating materials, sealers, primers, or correcting fluids.

The aforementioned lipidic peptide compound is preferably a compound prepared by bonding of a peptide moiety having an amino acid repeating structure to a lipidic moiety having a $C_{9-23}$ aliphatic group.

The aforementioned lipidic peptide compound is more preferably composed of at least one of compounds of the following Formulae (1) to (3) (lipidic peptides) or pharmaceutically usable salts of the compounds (low-molecular-weight compounds each having a lipidic moiety as a hydrophobic moiety and a peptide moiety as a hydrophilic moiety).

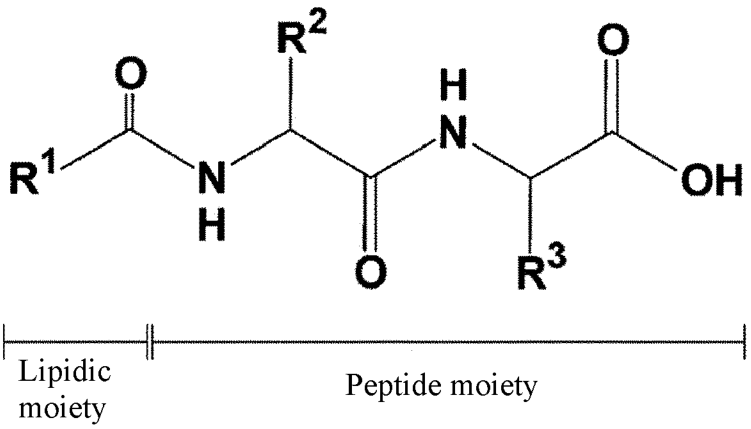


(1)

In Formula (1), $R^1$ is a $C_{9-23}$ aliphatic group. Preferably, $R^1$ is a linear aliphatic group having a carbon atom number of 11 to 23 and optionally having zero to two unsaturated bonds.

Specific examples of the lipidic moiety (acyl group) composed of $R^1$ and the adjacent carbonyl group include lauroyl group, dodecylcarbonyl group, myristoyl group, tetradecylcarbonyl group, palmitoyl group, margaroyl group, oleoyl group, elaidoyl group, linoleoyl group, stearoyl group, vaccenoyl group, octadecylcarbonyl group, arachidoyl group, eicosylcarbonyl group, behenoyl group, elkanoyl group, docosylcarbonyl group, lignoceroyl group, and nervonoyl group. Particularly preferred examples include lauroyl group, myristoyl group, palmitoyl group, margaroyl group, stearoyl group, oleoyl group, elaidoyl group, and behenoyl group.

In Formula (1), $R^2$ included in the peptide moiety is a hydrogen atom or a $C_{1-4}$ alkyl group optionally having a $C_1$ or $C_2$ branched chain.

The $C_{1-4}$ alkyl group optionally having a $C_1$ or $C_2$ branched chain refers to an alkyl group having a $C_{1-4}$ main chain and optionally having a $C_1$ or $C_2$ branched chain. Specific examples of the alkyl group include methyl group, ethyl group, n-propyl group, i-propyl group, n-butyl group, i-butyl group, sec-butyl group, and tert-butyl group.

$R^2$ is preferably a hydrogen atom or a $C_{1-3}$ alkyl group optionally having a $C_1$ branched chain, more preferably a hydrogen atom.

The $C_{1-3}$ alkyl group optionally having a $C_1$ branched chain refers to an alkyl group having a $C_{1-3}$ main chain and optionally having a $C_1$ branched chain. Specific examples of the alkyl group include methyl group, ethyl group, n-propyl group, i-propyl group, i-butyl group, and sec-butyl group. Preferred is methyl group, i-propyl group, i-butyl group,

or sec-butyl group.

In Formula (1), $R^3$ is a $-(CH_2)_n-X$ group. In the $-(CH_2)_n-X$ group, n is a number of 1 to 4, and X is an amino group, a guanidino group, a $-CONH_2$ group, or a 5-membered ring or a 6-membered ring optionally having one to three nitrogen atoms or a fused heterocyclic ring composed of the 5-membered ring and the 6-membered ring.

In the $-(CH_2)_n-X$ group represented by $R^3$, X is preferably an amino group, a guanidino group, a carbamoyl group ($-CONH_2$ group), a pyrrole group, an imidazole group, a pyrazole group, or an indole group, more preferably an imidazole group. In the $-(CH_2)_n-X$ group, n is preferably 1 or 2, more preferably 1.

Thus, the $-(CH_2)_n-$ group is preferably an aminomethyl group, a 2-aminoethyl group, a 3-aminopropyl group, a 4-aminobutyl group, a carbamoylmethyl group, a 2-carbamoylethyl group, a 3-carbamoylbutyl group, a 2-guanidinoethyl group, a 3-guanidinobutyl group, a pyrrolemethyl group, a 4-imidazolemethyl group, a pyrazolemethyl group, or a 3-indolemethyl group, more preferably a 4-aminobutyl group, a carbamoylmethyl group, a 2-carbamoylethyl group, a 3-guanidinobutyl group, a 4-imidazolemethyl group, or a 3-indolemethyl group, still more preferably a 4-imidazolemethyl group.

Particularly suitable lipidic peptide compounds of Formula (1) are the following compounds each being formed of a lipidic moiety and a peptide moiety (amino acid assembly). In the present specification, the amino acid abbreviations are alanine (Ala), asparagine (Asn), glutamine (Gln), glycine (Gly), histidine (His), isoleucine (Ile), leucine (Leu), lysine (Lys), tryptophan (Trp), and valine (Val). Specific examples of the compounds include lauroyl-Gly-His, lauroyl-Gly-Gln, lauroyl-Gly-Asn, lauroyl-Gly-Trp, lauroyl-Gly-Lys, lauroyl-Ala-His, lauroyl-Ala-Gln, lauroyl-Ala-Asn, lauroyl-Ala-Trp, and lauroyl-Ala-Lys; myristoyl-Gly-His, myristoyl-Gly-Gln, myristoyl-Gly-Asn, myristoyl-Gly-Trp, myristoyl-Gly-Lys, myristoyl-Ala-His, myristoyl-Ala-Gln, myristoyl-Ala-Asn, myristoyl-Ala-Trp, and myristoyl-Ala-Lys; palmitoyl-Gly-His, palmitoyl-Gly-Gln, palmitoyl-Gly-Asn, palmitoyl-Gly-Trp, palmitoyl-Gly-Lys, palmitoyl-Ala-His, palmitoyl-Ala-Gln, palmitoyl-Ala-Asn, palmitoyl-Ala-Trp, and palmitoyl-Ala-Lys; and stearoyl-Gly-His, stearoyl-Gly-Gln, stearoyl-Gly-Asn, stearoyl-Gly-Trp, stearoyl-Gly-Lys, stearoyl-Ala-His, stearoyl-Ala-Gln, stearoyl-Ala-Asn, stearoyl-Ala-Trp, and stearoyl-Ala-Lys.

Most preferred are lauroyl-Gly-His, lauroyl-Ala-His, myristoyl-Gly-His, myristoyl-Ala-His; palmitoyl-Gly-His, palmitoyl-Ala-His; stearoyl-Gly-His, and stearoyl-Ala-His.

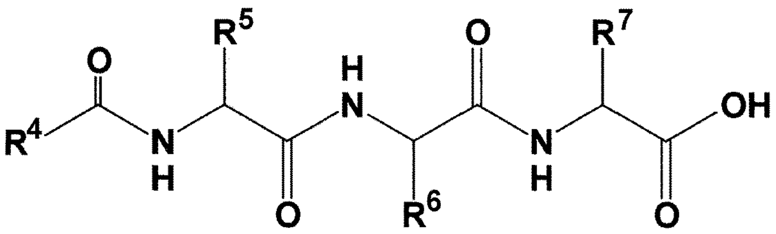

(2)

In Formula (2), $R^4$ is a $C_{9-23}$ aliphatic group. Preferred specific examples of $R^4$ include the same groups as those defined by $R^1$ above.

In Formula (2), $R^5$ to $R^7$ are each independently a hydrogen atom, a $C_{1-4}$ alkyl group optionally having a $C_1$ or $C_2$ branched chain, or a $-(CH_2)_n-X$ group, and at least one of $R^5$ to $R^7$ is a $-(CH_2)_n-X$ group. In the $-(CH_2)_n-X$ group, n is a number of 1 to 4, and X is an amino group, a guanidino group, a $-CONH_2$ group, or a 5-membered ring or a 6-membered ring optionally having one to three nitrogen atoms or a fused heterocyclic ring composed of the 5-membered ring and the 6-membered ring. Preferred specific examples of $R^5$ to $R^7$ include the same groups as those defined by $R^2$ and $R^3$ above.

Suitable lipidic peptide compounds of Formula (2) are the following compounds each being formed of a lipidic moiety and a peptide moiety (amino acid assembly). Specific examples of the compounds include lauroyl-Gly-Gly-His, myristoyl-Gly-Gly-His, myristoyl-Gly-Gly-Gln, myristoyl-Gly-Gly-Asn, myristoyl-Gly-Gly-Trp, myristoyl-Gly-Gly-Lys, myristoyl-Gly-Ala-His, myristoyl-Gly-Ala-Gln, myristoyl-Gly-Ala-Asn, myristoyl-Gly-Ala-Trp, myristoyl-Gly-Ala-Lys, myristoyl-Ala-Gly-His, myristoyl-Ala-Gly-Gln, myristoyl-Ala-Gly-Asn, myristoyl-Ala-Gly-Trp, myristoyl-Ala-Gly-Lys, myristoyl-Gly-His-Gly, myristoyl-His-Gly-Gly, palmitoyl-Gly-Gly-His, palmitoyl-Gly-Gly-Gln, palmitoyl-Gly-Gly-Asn, palmitoyl-Gly-Gly-Trp, palmitoyl-Gly-Gly-Lys, palmitoyl-Gly-Ala-His, palmitoyl-Gly-Ala-Gln, palmitoyl-Gly-Ala-Asn, palmitoyl-Gly-Ala-Trp, palmitoyl-Gly-Ala-Lys, palmitoyl-Ala-Gly-His, palmitoyl-Ala-Gly-Gln, palmitoyl-Ala-Gly-Asn, palmitoyl-Ala-Gly-Trp, palmitoyl-Ala-Gly-Lys, palmitoyl-Gly-His-Gly, palmitoyl-His-Gly-Gly, and stearoyl-Gly-Gly-His.

Of these, most preferred are lauroyl-Gly-Gly-His, myristoyl-Gly-Gly-His, palmitoyl-Gly-Gly-His, palmitoyl-Gly-His-Gly, palmitoyl-His-Gly-Gly, and stearoyl-Gly-Gly-His.

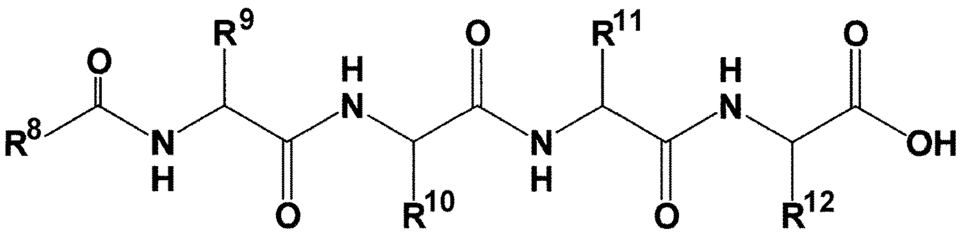 (3)

In Formula (3), $R^8$ is a $C_{9-23}$ aliphatic group. Preferred specific examples of $R^8$ include the same groups as those defined by $R^1$ above.

In Formula (3), $R^9$ to $R^{12}$ are each independently a hydrogen atom, a $C_{1-4}$ alkyl group optionally having a $C_1$ or $C_2$ branched chain, or a $-(CH_2)_n$-X group, and at least one of $R^9$ to $R^{12}$ is a $-(CH_2)_n$-X group. In the $-(CH_2)_n$-X group, n is a number of 1 to 4, and X is an amino group, a guanidino group, a $-CONH_2$ group, or a 5-membered ring or a 6-membered ring optionally having one to three nitrogen atoms or a fused heterocyclic ring composed of the 5-membered ring and the 6-membered ring. Preferred specific examples of $R^9$ to $R^{12}$ include the same groups as those defined by $R^2$ and $R^3$ above.

Suitable examples of the lipidic peptide compound of Formula (3) include lauroyl-Gly-Gly-Gly-His, myristoyl-Gly-Gly-Gly-His, palmitoyl-Gly-Gly-Gly-His, palmitoyl-Gly-Gly-His-Gly, palmitoyl-Gly-His-Gly-Gly, palmitoyl-His-Gly-Gly-Gly, and stearoyl-Gly-Gly-Gly-His.

In the present invention, the amount of the lipidic peptide compound contained in the coating film-forming composition is, for example, 0.01 to 30% by mass, preferably 0.02 to 10% by mass, more preferably 0.03 to 5% by mass, relative to the total mass of the composition.

In the present invention, the lipidic peptide compound is more preferably composed of at least one of compounds (lipidic peptides) of Formulae (1) to (3) or pharmaceutically usable salts of the compounds. These compounds may be used alone or in combination of two or more species.

The composition of the present invention may contain water, an alcohol, a polyhydric alcohol, or a mixed solution thereof in addition to at least one of the aforementioned lipidic peptide compounds.

Examples of the aforementioned water include clean water, purified water, hard water, soft water, natural water, deep ocean water, electrolytic alkaline ion water, electrolytic acidic ion water, ion water, and cluster water.

The aforementioned alcohol refers to a monohydric alcohol. Examples of the monohydric alcohol include $C_{1-6}$ alcohols that dissolve in water in any proportion, such as methanol, ethanol, 2-propanol, and i-butanol; and higher alcohols, such as oleyl alcohol and phenoxy alcohol.

The aforementioned polyhydric alcohol refers to a di- or more-valent alcohol. Examples of the polyhydric alcohol include propylene glycol, 1,3-butanediol, 2-ethyl-1,3-hexanediol, glycerin, isopentyldiol, ethylhexanediol, erythrulose, ozonated glycerin, caprylyl glycol, glycol, (C15-18) glycol, (C20-30) glycol, glycerin, diethylene glycol, diglycerin, dithiaoctanediol, DPG, thioglycerin, 1,10-decanediol, decylene glycol, triethylene glycol, trimethylhydroxymethylcyclohexanol, phytantriol, phenoxypropanediol, 1,2-butanediol, 2,3-butanediol, butylethylpropanediol, 1,2-hexanediol, hexylene glycol, pentylene glycol, methylpropanediol, menthanediol, lauryl glycol, and polypropylene glycol.

In the present invention, when the composition contains a polyhydric alcohol, the amount of the polyhydric alcohol may be, for example, 1% by mass to 60% by mass, preferably 1% by mass to 30% by mass, relative to the total mass of the composition.

In the present invention, when the composition contains a polyhydric alcohol, a single polyhydric alcohol may be used, or two or more polyhydric alcohols may be used in combination.

[Other Additives]

The composition of the present invention may optionally contain additives generally usable as additives for cosmetic products, quasi-drugs, and pharmaceutical products.

Examples of additive ingredients such as physiologically active substances and functional substances contained in external preparations for skin (e.g., cosmetic products, quasi-drugs, or pharmaceutical products) include pigments, oily bases, humectants, texture improvers, surfactants other than those described above, polymers, thickeners, gelators, solvents, antioxidants, reducing agents, oxidizers, preservatives, antimicrobial agents, antiseptics, chelating agents, pH adjusters, acids, alkalis, powders, inorganic salts, ultraviolet absorbers, whitening agents, vitamins and derivatives thereof, hair growth-promoting agents, blood circulation promoters, stimulants, hormones, anti-wrinkle agents, anti-aging agents, firming agents, cooling agents, warming agents, wound-healing promoters, abirritants, analgesics, cell activators, plant/animal/microbial extracts, antipruritics, exfoliates/keratolytic agents, antiperspirants, algefacients, astringents, enzymes, nucleic acids, perfumes, colors, coloring agents, dyes, antiphlogistics, anti-inflammatory agents, anti-asthmatic

agents, anti-chronic obstructive pulmonary disease agents, anti-allergic agents, immunomodulators, anti-infective agents, and antifungal agents.

The amount of such an additive contained in the composition may vary depending on the type of the additive. The amount of the additive may be, for example, about 0.001% by mass to 20% by mass or about 0.01% by mass to 10% by mass, relative to the total mass of the composition.

Preferred examples of pigments include inorganic white pigments, such as titanium dioxide and zinc oxide; inorganic red pigments, such as red iron oxide and iron titanate; inorganic brown pigments, such as $\gamma$-iron oxide; inorganic yellow pigments, such as yellow iron oxide and ocher; inorganic black pigments, such as black iron oxide and low-order titanium oxide; inorganic violet pigments, such as mango violet and cobalt violet; inorganic green pigments, such as chromium oxide, chromium hydroxide, and cobalt titanate; inorganic blue pigments, such as ultramarine and Prussian blue; pearl pigments, such as titanium oxide-coated mica, titanium oxide-coated bismuth oxychloride, titanium oxide-coated talc, colored titanium oxide-coated mica, bismuth oxychloride, and argentine; extender pigments, such as talc, sericite, mica, kaolin, calcium carbonate, magnesium carbonate, silicic anhydride, barium sulfate, and aluminum hydroxide; metal powder pigments, such as aluminum powder, copper powder, and gold; surface-treated inorganic and metal powder pigments; organic pigments, such as zirconium, barium, and aluminum lake; and surface-treated organic pigments.

Preferred examples of oily bases include higher (polyhydric) alcohols, such as oleyl alcohol, jojoba alcohol, chimyl alcohol, selachyl alcohol, batyl alcohol, hexyldecanol, isostearyl alcohol, 2-octyldodecanol, and dimer diols; aralkyl alcohols and derivatives thereof, such as benzyl alcohol; isostearic acid, behenic acid, undecylenic acid, 12-hydroxystearic acid, palmitoleic acid, oleic acid, linoleic acid, linolenic acid, erucic acid, docosahexaenoic acid, eicosapentaenoic acid, isohexadecanoic acid, anteisoheneicosanoic acid, long-chain branched fatty acids, dimer acids, and hydrogenated dimer acids; hydrocarbons, such as liquid paraffin (mineral oil), heavy liquid isoparaffin, light liquid isoparaffin, $\alpha$-olefin oligomers, polyisobutene, hydrogenated polyisobutene, polybutene, squalane, olive-derived squalane, squalene, vaseline, and solid paraffin; waxes, such as candelilla wax, carnauba wax, rice wax, Japan wax, beeswax, montan wax, ozokerite, ceresin, paraffin wax, microcrystalline wax, petrolatum, Fischer-Tropsch wax, polyethylene wax, and ethylene-propylene copolymers; vegetable oils and fats, such as coconut oil, palm oil, palm kernel oil, safflower oil, olive oil, castor oil, avocado oil, sesame oil, tea oil, evening primrose oil, wheat germ oil, macadamia nut oil, hazelnut oil, kukui nut oil, rose hip oil, meadowfoam oil, persic oil, tea tree oil, peppermint oil, corn oil, rapeseed oil, sunflower oil, wheat germ oil, linseed oil, cottonseed oil, soybean oil, peanut oil, rice bran oil, cacao butter, shea butter, hydrogenated coconut oil, hydrogenated castor oil, jojoba oil, hydrogenated jojoba oil, grape seed oil, apricot oil (apricot kernel oil), and camellia oil; animal oils and fats, such as beef tallow, milk fat, horse fat, egg-yolk oil, mink oil, and turtle oil; animal waxes, such as spermaceti, lanolin, and orange roughy oil; lanolins, such as liquid lanolin, reduced lanolin, adsorption-purified lanolin, acetylated lanolin, acetylated liquid lanolin, hydroxylated lanolin, polyoxyethylene lanolin, lanolin fatty acids, hard lanolin fatty acids, lanolin alcohol, acetylated lanolin alcohol, and acetylated (cetyl/lanolyl) ester; sterols, such as cholesterol, dihydrocholesterol, lanosterol, dihydrolanosterol, phytosterol, and cholic acid; sapogenins; saponins; sterol esters, such as cholesteryl acetate, cholesteryl nonanoate, cholesteryl stearate, cholesteryl isostearate, cholesteryl oleate, di(cholesteryl/behenyl/octyldodecyl) N-lauroyl-L-glutamate, di(cholesteryl/octyldodecyl) N-lauroyl-L-glutamate, di(phytosteryl/behenyl/octyldodecyl) N-lauroyl-L-glutamate, di(phytosteryl/octyldodecyl) N-lauroyl-L-glutamate, acyl sarcosine alkyl esters such as isopropyl N-lauroylsarcosinate, cholesteryl 12-hydroxystearate, cholesteryl macadamiate, phytosteryl macadamiate, phytosteryl isostearate, soft lanolin fatty acid cholesteryl esters, hard lanolin fatty acid cholesteryl esters, long-chain branched fatty acid cholesteryl esters, and long-chain $\alpha$-hydroxy fatty acid cholesteryl esters; lipidic complexes, such as phospholipid-cholesterol complexes and phospholipid-phytosterol complexes; monohydric alcohol esters of carboxylic acids, such as octyldodecyl myristate, hexyldecyl myristate, octyldodecyl isostearate, cetyl palmitate, octyldodecyl palmitate, cetyl octanoate, hexyldecyl octanoate, isotridecyl isononanoate, isononyl isononanoate, octyl isononanoate, isodecyl neopentanoate, isotridecyl neopentanoate, isostearyl neopentanoate, octyldodecyl neodecanoate, oleyl oleate, octyldodecyl oleate, octyldodecyl ricinoleate, octyldodecyl lanolate, hexyldecyl dimethyloctanoate, octyldodecyl erucate, hydrogenated castor oil isostearate, ethyl oleate, ethyl avocadate, isopropyl myristate, isopropyl palmitate, octyl palmitate, isopropyl isostearate, isopropyl lanolate, diethyl sebacate, diisopropyl sebacate, dioctyl sebacate, diisopropyl adipate, dibutyloctyl sebacate, diisobutyl adipate, dioctyl succinate, and triethyl citrate; oxyacid esters, such as cetyl lactate, diisostearyl malate, and hydrogenated castor oil monoisostearate; polyhydric alcohol esters of fatty acids, such as glyceryl trioctanoate (glyceryl tri-2-ethylhexanoate), glyceryl trioleate, glyceryl triisostearate, glyceryl diisostearate, glyceryl tri(caprylate/caprate), glyceryl tri(caprylate/caprate/myristate/stearate), hydrogenated rosin triglyceride (hydrogenated ester gum), rosin triglyceride (ester gum), glyceryl behenate eicosanedioate, trimethylolpropane trioctanoate, trimethylolpropane triisostearate, neopentyl glycol dioctanoate, neopentyl glycol dicaprate, 2-butyl-2-ethyl-1,3-propanediol dioctanoate, propylene glycol dioleate, pentaerythrityl tetraoctanoate, hydrogenated rosin pentaerythrityl ester, ditrimethylolpropane triethylhexanoate, ditrimethylolpropane (isostearate/sebacate), pentaerythrityl triethylhexanoate, dipentaerythrityl (hydroxystearate/stearate/rosinate), diglyceryl diisostearate, polyglyceryl tetraisostearate, polyglyceryl-10 nonaisostearate, polyglyceryl-8 deca(erucate/isostearate/ricinoleate), (hexyldecanoic acid/sebacic acid) diglyceryl

oligoester, glycol distearate (ethylene glycol distearate), 3-methyl-1,5-pentanediol dineopentanoate, and 2,4-diethyl-1,5-pentanediol dineopentanoate; dimer acid or dimer diol derivatives, such as diisopropyl dimer dilinoleate, diisostearyl dimer dilinoleate, di(isostearyl/phytosteryl) dimer dilinoleate, (phytosteryl/behenyl) dimer dilinoleate, (phytosteryl/isostearyl/cetyl/stearyl/behenyl) dimer dilinoleate, dimer dilinoleyl dimer dilinoleate, dimer dilinoleyl diisostearate, dimer dilinoleyl hydrogenated rosin condensates, hydrogenated castor oil dimer dilinoleate, and hydroxyalkyl dimer dilinoleyl ether; fatty acid alkanolamides, such as coconut oil fatty acid monoethanolamide (cocamide MEA), coconut oil fatty acid diethanolamide (cocamide DEA), lauric acid monoethanolamide (lauramide MEA), lauric acid diethanolamide (lauramide DEA), lauric acid monoisopropanolamide (lauramide MIPA), palmitic acid monoethanolamide (palmitamide MEA), palmitic acid diethanolamide (palmitamide DEA), and coconut oil fatty acid methylethanolamide (cocamide methyl MEA); silicones, such as dimethicone (dimethylpolysiloxane), highly polymerized dimethicone (highly polymerized dimethylpolysiloxane), cyclomethicone (cyclic dimethylsiloxane, decamethylcyclopentasiloxane (which may also be referred to simply as "cyclopentasiloxane")), phenyl trimethicone, diphenyl dimethicone, phenyl dimethicone, stearoxypropyldimethylamine, (aminoethylaminopropyl methicone/dimethicone) copolymers, dimethiconol, dimethiconol crosspolymers, silicone resin, silicone rubber, amino-modified silicones such as aminopropyl dimethicone and amodimethicone, cation-modified silicones, polyether-modified silicones such as dimethicone copolyols, polyglycerin-modified silicones, sugar-modified silicones, carboxylic acid-modified silicones, phosphoric acid-modified silicones, sulfuric acid-modified silicones, alkyl-modified silicones, fatty acid-modified silicones, alkyl ether-modified silicones, amino acid-modified silicones, peptide-modified silicones, fluorine-modified silicones, cation-modified and polyether-modified silicones, amino-modified and polyether-modified silicones, alkyl-modified and polyether-modified silicones, and polysiloxane-oxyalkylene copolymers; and fluorine oils, such as perfluorodecane, perfluorooctane, and perfluoropolyether.

Preferred examples of humectants and texture improvers include polyols and polymers thereof, such as glycerin, trimethylolpropane, pentaerythritol, hexylene glycol, diglycerin, polyglycerin, diethylene glycol, dipropylene glycol, polypropylene glycol, and ethylene glycol-propylene glycol copolymers; glycol alkyl ethers, such as diethylene glycol monoethyl ether (ethoxydiglycol), ethylene glycol monoethyl ether, ethylene glycol monobutyl ether, and diethylene glycol dibutyl ether; water-soluble esters, such as polyglyceryl-10 (eicosanedioate/tetradecanedioate) and polyglyceryl-10 tetradecanedioate; sugar alcohols, such as sorbitol, xylitol, erythritol, mannitol, and maltitol; saccharides and derivatives thereof, such as glucose, fructose, galactose, mannose, threose, xylose, arabinose, fucose, ribose, deoxyribose, maltose, trehalose, lactose, raffinose, gluconic acid, glucuronic acid, cyclodextrins ($\alpha$-, $\beta$-, and $\gamma$-cyclodextrins, and modified cyclodextrins such as maltosyl cyclodextrin and hydroxyalkyl cyclodextrin), $\beta$-glucan, chitin, chitosan, heparin and derivatives thereof, pectin, arabinogalactan, dextrin, dextran, glycogen, ethyl glucoside, and glucosylethyl methacrylate polymer or copolymer; hyaluronic acid and sodium hyaluronate; sodium chondroitin sulfate; mucoitin sulfate, charonin sulfate, keratosulfate, and dermatan sulfate; Tremella fuciformis extract and Tremella fuciformis polysaccharides; fucoidan; tuberose polysaccharides or naturally occurring polysaccharides; organic acids such as citric acid, tartaric acid, and lactic acid, and salts thereof; urea and derivatives thereof; 2-pyrrolidone-5-carboxylic acid and salts thereof, such as sodium salt; amino acids, such as betaine (trimethylglycine), proline, hydroxyproline, arginine, lysine, serine, glycine, alanine, phenylalanine, tyrosine, $\beta$-alanine, threonine, glutamic acid, glutamine, asparagine, aspartic acid, cysteine, cysteine, methionine, leucine, isoleucine, valine, tryptophan, histidine, and taurine, and salts thereof; protein peptides and derivatives thereof, such as collagen, fish-derived collagen, atelocollagen, gelatin, elastin, peptides derived from degraded collagen, hydrolyzed collagen, hydroxypropylammonium chloride hydrolyzed collagen, peptides derived from degraded elastin, peptides derived from degraded keratin, hydrolyzed keratin, peptides derived from degraded conchiolin, hydrolyzed conchiolin, peptides derived from degraded silk protein, hydrolyzed silk, sodium lauroyl hydrolyzed silk, peptides derived from degraded soy protein, peptides derived from degraded wheat protein, hydrolyzed wheat protein, peptides derived from degraded casein, and acylated peptides; acylated peptides, such as palmitoyl oligopeptide, palmitoyl pentapeptide, and palmitoyl tetrapeptide; silylated peptides; lactic acid bacteria culture, yeast extracts, eggshell membrane protein, bovine submaxillary mucin, hypotaurine, sesame lignan glycosides, glutathione, albumin, and whey; choline chloride and phosphorylcholine; animal and plant extract ingredients, such as placenta extract, elastin, collagen, aloe extract, Hamamelis virginiana water, Luffa cylindrica water, Chamomilla recutita extract, licorice extract, comfrey extract, silk extract, Rosa roxburghii extract, Achillea millefolium extract, Eucalyptus globulus extract, and melilot extract; and ceramides, such as natural ceramides (types 1, 2, 3, 4, 5, and 6), hydroxyceramide, pseudoceramide, sphingoglycolipid, ceramide-containing extract, and glucosylceramide-containing extract.

Preferred examples of surfactants include anionic surfactants, nonionic surfactants, cationic surfactants, amphoteric surfactants, and polymer surfactants. Preferred examples of anionic surfactants include fatty acid salts, such as potassium laurate and potassium myristate; alkyl sulfates, such as sodium lauryl sulfate, triethanolamine lauryl sulfate, and ammonium lauryl sulfate; polyoxyethylene alkyl sulfates, such as sodium laureth sulfate and triethanolamine laureth sulfate; acyl N-methylamino acid salts, such as sodium cocoyl methyl taurate, potassium cocoyl methyl taurate, sodium lauroyl methyl taurate, sodium myristoyl methyl taurate, sodium lauroyl methyl alaninate, sodium lauroyl sarcosinate, triethanolamine lauroyl sarcosinate, and sodium lauroyl glutamate methyl alaninate; acyl amino acid salts, such as sodium cocoyl glutamate, triethanolamine cocoyl glutamate, sodium lauroyl glutamate, sodium myristoyl glutamate,

sodium stearoyl glutamate, ditriethanolamine palmitoyl aspartate, and triethanolamine cocoyl alaninate; polyoxyethylene alkyl ether acetates, such as sodium laureth acetate; succinates, such as sodium lauroyl monoethanolamide succinate; fatty acid alkanolamide ether carboxylates; acyl lactates; polyoxyethylene fatty amine sulfates; fatty acid alkanolamide sulfates; fatty acid glyceride sulfates, such as sodium hydrogenated coconut oil fatty acid glycerin sulfates; alkylbenzene polyoxyethylene sulfates; olefin sulfonates, such as sodium $\alpha$-olefin sulfonates; alkyl sulfosuccinates, such as disodium lauryl sulfosuccinate and sodium dioctyl sulfosuccinate; alkyl ether sulfosuccinates, such as disodium laureth sulfosuccinate, sodium monolauroyl monoethanolamide polyoxyethylene sulfosuccinate, and sodium lauryl polypropylene glycol sulfosuccinate; alkylbenzene sulfonates, such as sodium tetradecylbenzene sulfonate and triethanolamine tetradecylbenzene sulfonate; alkyl naphthalene sulfonates; alkane sulfonates; $\alpha$-sulfofatty acid methyl ester salts; acyl isethionates; alkyl glycidyl ether sulfonates; alkyl sulfoacetates; alkyl ether phosphates, such as sodium laureth phosphate, sodium dilaureth phosphate, sodium trilaureth phosphate, and sodium monooreth phosphate; alkyl phosphates, such as potassium lauryl phosphate; sodium caseinate; alkyl aryl ether phosphates; fatty acid amide ether phosphates; phospholipids, such as phosphatidylglycerol, phosphatidylinositol, and phosphatidic acid; and silicone anionic surfactants, such as carboxylic acid-modified silicones, phosphoric acid-modified silicones, and sulfuric acid-modified silicones. Preferred examples of nonionic surfactants include polyoxyethylene alkyl ethers with various numbers of polyoxyethylene units, such as laureths (polyoxyethylene lauryl ethers), ceteths (polyoxyethylene cetyl ethers), steareths (polyoxyethylene stearyl ethers), beheneths (polyoxyethylene behenyl ethers), isosteareths (polyoxyethylene isostearyl ethers), and octyldodeceths (polyoxyethylene octyldodecyl ethers); polyoxyethylene alkyl phenyl ethers; castor oil derivatives and hydrogenated castor oil derivatives, such as polyoxyethylene hydrogenated castor oil, polyoxyethylene castor oil, polyoxyethylene hydrogenated castor oil monoisostearate, polyoxyethylene hydrogenated castor oil triisostearate, polyoxyethylene hydrogenated castor oil monopyroglutamate monoisostearate diester, and polyoxyethylene hydrogenated castor oil maleate; polyoxyethylene phytosterol; polyoxyethylene cholesterol; polyoxyethylene cholestanol; polyoxyethylene lanolin; polyoxyethylene reduced lanolin; polyoxyethylene-polyoxypropylene alkyl ethers, such as polyoxyethylene-polyoxypropylene cetyl ether, polyoxyethylene-polyoxypropylene 2-decyltetradecyl ether, polyoxyethylene-polyoxypropylene monobutyl ether, polyoxyethylene-polyoxypropylene hydrogenated lanolin, and polyoxyethylene-polyoxypropylene glycerin ether; polyoxyethylene-polyoxypropylene glycol; (poly)glycerin polyoxypropylene glycols, such as PPG-9 diglyceryl; glycerin fatty acid partial esters, such as glyceryl stearate, glyceryl isostearate, glyceryl palmitate, glyceryl myristate, glyceryl oleate, glyceryl cocoate, glycerin mono-cottonseed oil fatty acid esters, glycerin monoerucate, glycerin sesquioleate, glycerin $\alpha,\alpha'$-oleate pyroglutamate, and glycerin monostearate malate; polyglycerin fatty acid esters, such as polyglyceryl-2 stearate, polyglyceryl-3 stearate, polyglyceryl-4 stearate, polyglyceryl-5 stearate, polyglyceryl-6 stearate, polyglyceryl-8 stearate, polyglyceryl-10 stearate, polyglyceryl-6 distearate, polyglyceryl-10 distearate, polyglyceryl-2 tristearate, polyglyceryl-10 decastearate, polyglyceryl-2 isostearate, polyglyceryl-3 isostearate, polyglyceryl-4 isostearate, polyglyceryl-5 isostearate, polyglyceryl-6 isostearate, polyglyceryl-8 isostearate, polyglyceryl-10 isostearate, polyglyceryl-2 diisostearate (diglyceryl diisostearate), polyglyceryl-3 diisostearate, polyglyceryl-10 diisostearate, polyglyceryl-2 triisostearate, polyglyceryl-2 tetraisostearate, polyglyceryl-10 decaisostearate, polyglyceryl-2 oleate, polyglyceryl-3 oleate, polyglyceryl-4 oleate, polyglyceryl-5 oleate, polyglyceryl-6 oleate, polyglyceryl-8 oleate, polyglyceryl-10 oleate, polyglyceryl-6 dioleate, polyglyceryl-2 trioleate, and polyglyceryl-10 decaoleate; ethylene glycol mono-fatty acid esters, such as ethylene glycol monostearate; propylene glycol mono-fatty acid esters, such as propylene glycol monostearate; pentaerythritol fatty acid partial esters; sorbitol fatty acid partial esters; maltitol fatty acid partial esters; maltitol ethers; sorbitan fatty acid esters, such as sorbitan monooleate, sorbitan monoisostearate, sorbitan monolaurate, sorbitan monopalmitate, sorbitan monostearate, sorbitan sesquioleate, sorbitan trioleate, diglycerol sorbitan penta-2-ethylhexylate, and diglycerol sorbitan tetra-2-ethylhexylate; sugar derivative partial esters, such as sucrose fatty acid esters, methyl glucoside fatty acid esters, and trehalose undecylenoate; alkyl glucosides, such as caprylyl glucoside; alkyl polyglycosides; lanolin alcohol; reduced lanolin; polyoxyethylene fatty acid mono- and di-esters, such as polyoxyethylene distearate, polyethylene glycol diisostearate, polyoxyethylene monooleate, and polyoxyethylene dioleate; polyoxyethylene-propylene glycol fatty acid esters; polyoxyethylene glycerin fatty acid esters, such as polyoxyethylene monooleates, for example, polyoxyethylene glycerin monostearate, polyoxyethylene glycerin monoisostearate, and polyoxyethylene glycerin triisostearate; polyoxyethylene sorbitan fatty acid esters, such as polyoxyethylene sorbitan monooleate, polyoxyethylene sorbitan monostearate, polyoxyethylene sorbitan monooleate, and polyoxyethylene sorbitan tetraoleate; polyoxyethylene sorbitol fatty acid esters, such as polyoxyethylene sorbitol monolaurate, polyoxyethylene sorbitol monooleate, polyoxyethylene sorbitol pentaoleate, and polyoxyethylene sorbitol monostearate; polyoxyethylene methyl glucoside fatty acid esters; polyoxyethylene alkyl ether fatty acid esters; polyoxyethylene animal and vegetable fats and oils, such as polyoxyethylene sorbitol beeswax; alkyl glyceryl ethers, such as isostearyl glyceryl ether, chimyl alcohol, selachyl alcohol, and batyl alcohol; polyhydric alcohol alkyl ethers; polyoxyethylene alkylamines; tetrapolyoxyethylene/tetrapolyoxypropylene-ethylenediamine condensates; natural surfactants, such as saponin and sophorolipid; polyoxyethylene fatty acid amides; fatty acid alkanolamides, such as coconut oil fatty acid monoethanolamide (cocamide MEA), coconut oil fatty acid diethanolamide (cocamide DEA), lauric acid monoethanolamide (lauramide MEA), lauric acid diethanolamide (lauramide DEA), lauric acid monoisopropanolamide (lauramide MIPA), palmitic acid monoethanolamide (palmitamide

MEA), palmitic acid diethanolamide (palmitamide DEA), and coconut oil fatty acid methylethanolamides (cocamide methyl MEA); alkyl dimethylamine oxides, such as lauramine oxide, cocamine oxide, stearamine oxide, and behenamine oxide; alkyl ethoxydimethylamine oxides; polyoxyethylene alkyl mercaptans; and silicone nonionic surfactants, for example, polyether-modified silicones such as dimethicone copolyols, polysiloxane-oxyalkylene copolymers, polyglycerin-modified silicones, and sugar-modified silicones. Preferred examples of cationic surfactants include alkyl trimethylammonium chlorides, such as behentrimonium chloride, steartrimonium chloride, cetrimonium chloride, and lauryltrimonium chloride; alkyl trimethylammonium bromides, such as steartrimonium bromide; dialkyl dimethylammonium chlorides, such as distearyldimonium chloride and dicocodimonium chloride; fatty acid amide amines, such as stearamidopropyl dimethylamine and stearamidoethyl diethylamine, and salts thereof; alkyl ether amines, such as stearoxypropyldimethylamine and salts or quaternary salts thereof; fatty acid amide quaternary ammonium salts, such as long-chain branched fatty acid (12 to 31) aminopropylethyldimethylammonium ethyl sulfates and lanolin fatty acid aminopropylethyldimethylammonium ethyl sulfates; polyoxyethylene alkylamines and salts or quaternary salts thereof; alkylamine salts; fatty acid amide guanidium salts; alkyl ether amine ammonium salts; alkyl trialkylene glycol ammonium salts; benzalkonium salts; benzethonium salts; pyridinium salts, such as cetylpyridinium chloride; imidazolinium salts; alkyl isoquinolinium salts; dialkyl morpholinium salts; polyamine fatty acid derivatives; and silicone cationic surfactants, such as amino-modified silicones such as aminopropyl dimethicone and amodimethicone, cation-modified silicones, cation-modified and polyether-modified silicones, and amino-modified and polyether-modified silicones. Preferred examples of amphoteric surfactants include N-alkyl-N,N-dimethylamino acid betaines, such as lauryl betaine (lauryl dimethylaminoacetic acid betaine); fatty acid amidoalkyl-N,N-dimethylamino acid betaines, such as cocamidopropyl betaine and lauramidopropyl betaine; imidazoline betaines, such as sodium cocoamphoacetate and sodium lauroamphoacetate; alkyl sulfobetaines, such as alkyl dimethyltaurine; betaine sulfates, such as alkyl dimethylaminoethanol sulfate; betaine phosphates, such as alkyl dimethylaminoethanol phosphates; phospholipids, such as phosphatidylcholine, phosphatidylethanolamine, phosphatidylserine, sphingophospholipids such as sphingomyelin, lysolecithin, hydrogenated soybean phospholipid, partially hydrogenated soybean phospholipid, hydrogenated egg yolk phospholipid, partially hydrogenated egg yolk phospholipid, and hydroxylated lecithin; and silicone amphoteric surfactants. Preferred examples of polymer surfactants include polyvinyl alcohol, sodium alginate, starch derivatives, tragacanth gum, and acrylic acid-alkyl methacrylate copolymers; and various silicone surfactants.

Preferred examples of polymers, thickeners, and gelators include guar gum, locust bean gum, quince seed, carrageenan, galactan, gum arabic, tara gum, tamarind, furcellaran, karaya gum, Abelmoschus manihot, cara gum, tragacanth gum, pectin, pectic acid and salts thereof, such as sodium salt, alginic acid and salts thereof, such as sodium salt, and mannan; starches, such as rice starch, corn starch, potato starch, and wheat starch; xanthan gum, dextran, succinoglucan, curdlan, hyaluronic acid and salts thereof, xanthan gum, pullulan, gellan gum, chitin, chitosan, agar, brown algae extract, chondroitin sulfate, casein, collagen, gelatin, and albumin; celluloses and derivatives thereof, such as methyl cellulose, ethyl cellulose, hydroxyethyl cellulose, hydroxypropyl cellulose, hydroxypropyl methylcellulose, carboxymethyl cellulose and salts thereof, such as sodium salt, methylhydroxypropyl cellulose, sodium cellulose sulfate, dialkyldimethylammonium cellulose sulfate, crystalline cellulose, and cellulose powder; starch derivatives, such as soluble starch, starch polymers such as carboxymethyl starch, methylhydroxypropyl starch, and methyl starch, starch hydroxypropyltrimonium chloride, and aluminum corn starch octenylsuccinate; alginic acid derivatives, such as sodium alginate and propylene glycol alginate; polyvinylpyrrolidone (PVP), polyvinyl alcohol (PVA), vinylpyrrolidone-vinyl alcohol copolymers, and polyvinyl methyl ether; polyethylene glycol, polypropylene glycol, and polyoxyethylene-polyoxypropylene copolymers; amphoteric methacrylic acid ester copolymers, such as (methacryloyloxyethylcarboxybetaine/alkyl methacrylate) copolymers and (acrylate/stearyl acrylate/ethylamine oxide methacrylate) copolymers; (dimethicone/vinyl dimethicone) crosspolymers, (alkyl acrylate/diacetone acrylamide) copolymers, and (alkyl acrylate/diacetone acrylamide) copolymers AMP; partially saponified polyvinyl acetate and maleic acid copolymers; vinylpyrrolidone-dialkylaminoalkyl methacrylate copolymers; acrylic resin alkanolamine; polyester and water-dispersible polyester; polyacrylamide; copolymers of polyacrylic esters such as polyethyl acrylate, carboxy vinyl polymers, polyacrylic acid and salts thereof, such as sodium salt, and acrylic acid-methacrylic acid ester copolymers; acrylic acid-alkyl methacrylate copolymers; cationized celluloses such as polyquaternium-10, diallyldimethylammonium chloride-acrylamide copolymers, such as polyquaternium-7, acrylic acid-diallyldimethylammonium chloride copolymers, such as polyquaternium-22, acrylic acid-diallyldimethylammonium chloride-acrylamide copolymers, such as polyquaternium-39, acrylic acid-cationized methacrylic acid ester copolymers, acrylic acid-cationized methacrylic acid amide copolymers, acrylic acid-methyl acrylate-methacrylamidopropyltrimethylammonium chloride copolymers, such as polyquaternium-47, and methacryloyl chloride choline ester polymers; cationized polysaccharides, such as cationized oligosaccharides, cationized dextran, and guar hydroxypropyltrimonium chloride; polyethyleneimine; cationic polymers; copolymers of 2-methacryloyloxyethyl phosphorylcholine and butyl methacrylate, such as polyquaternium-51; polymer emulsions, such as acrylic resin emulsions, polyethyl acrylate emulsions, polyacrylalkyl ester emulsions, polyvinyl acetate resin emulsions, natural rubber latex, and synthetic latex; nitrocellulose; polyurethanes and various copolymers thereof; various silicones; various silicone copolymers, such as acrylic-silicone graft copolymers; various fluorine polymers; 12-hydroxystearic acid and salts thereof; dextrin fatty

acid esters, such as dextrin palmitate and dextrin myristate; and silicic anhydride, fumed silica (silicic anhydride ultrafine particles), magnesium aluminum silicate, magnesium sodium silicate, metallic soaps, dialkyl phosphate metal salts, bentonite, hectorite, organic-modified clay minerals, sucrose fatty acid esters, and fructooligosaccharide fatty acid esters. Among the aforementioned examples, preferred are cellulose and derivatives thereof, alginic acid and salts thereof, polyvinyl alcohol, hyaluronic acid and salts thereof, or collagen.

Preferred examples of solvents include lower alcohols, such as ethanol, 2-propanol (isopropyl alcohol), butanol, and isobutyl alcohol; glycols, such as propylene glycol, diethylene glycol, dipropylene glycol, and isopentyldiol; glycol ethers, such as diethylene glycol monoethyl ether (ethoxydiglycol), ethylene glycol monoethyl ether, ethylene glycol monobutyl ether, triethylene glycol monoethyl ether, diethylene glycol diethyl ether, diethylene glycol dibutyl ether, propylene glycol monoethyl ether, and dipropylene glycol monoethyl ether; glycol ether esters, such as ethylene glycol monoethyl ether acetate, diethylene glycol monoethyl ether acetate, and propylene glycol monoethyl ether acetate; glycol esters, such as diethoxyethyl succinate and ethylene glycol disuccinate; benzyl alcohol, benzyloxyethanol, propylene carbonate, dialkyl carbonate, acetone, ethyl acetate, and N-methylpyrrolidone; and toluene.

Preferred examples of antioxidants include tocopherol (vitamin E) and tocopherol derivatives, such as tocopherol acetate; BHT and BHA; gallic acid derivatives, such as propyl gallate; vitamin C (ascorbic acid) and/or derivatives thereof; erythorbic acid and derivatives thereof; sulfites, such as sodium sulfite; hydrogen sulfites, such as sodium hydrogen sulfite; thiosulfates, such as sodium thiosulfate; metabisulfites; thiotaurine and hypotaurine; and thioglycerol, thiourea, thioglycolic acid, and cysteine hydrochloride.

Preferred examples of reducing agents include thioglycolic acid, cysteine, and cysteamine.

Preferred examples of oxidizers include hydrogen peroxide solution, ammonium persulfate, sodium bromate, and percarbonic acid.

Preferred examples of preservatives, antimicrobial agents, and antiseptics include hydroxybenzoic acids and salts or esters thereof, such as methylparaben, ethylparaben, propylparaben, and butylparaben; salicylic acid; sodium benzoate; phenoxyethanol; isothiazolinone derivatives, such as methylchloroisothiazolinone and methylisothiazolinone; imidazolinium urea; dehydroacetic acid and salts thereof; phenols; halogenated bisphenols, such as triclosan, acid amides thereof, and quaternary ammonium salts thereof; trichlorocarbanide, zinc pyrithione, benzalkonium chloride, benzethonium chloride, sorbic acid, chlorhexidine, chlorhexidine gluconate, halocarban, hexachlorophene, and hinokitiol; other phenols, such as phenol, isopropylphenol, cresol, thymol, parachlorohenol, phenylphenol, and sodium phenylphenate; phenyl ethyl alcohol, photosensitizers, antibacterial zeolite, and silver ions.

Preferred examples of chelating agents include edetates (ethylenediamine tetraacetates), such as EDTA, EDTA 2Na, EDTA 3Na, and EDTA 4Na; hydroxyethylethylenediamine triacetates, such as HEDTA 3Na; pentetates (diethylenetriamine pentaacetate); phytic acid; phosphonic acids, such as etidronic acid, and salts thereof, such as sodium salt; polyamino acids, such as polyaspartic acid and polyglutamic acid; sodium polyphosphate, sodium metaphosphate, and phosphoric acid; and sodium citrate, citric acid, alanine, dihydroxyethylglycine, gluconic acid, ascorbic acid, succinic acid, and tartaric acid.

Preferred examples of pH adjusters, acids, and alkalis include ascorbic acid, citric acid, sodium citrate, lactic acid, sodium lactate, potassium lactate, glycolic acid, succinic acid, acetic acid, sodium acetate, malic acid, tartaric acid, fumaric acid, phosphoric acid, hydrochloric acid, sulfuric acid, monoethanolamine, diethanolamine, triethanolamine, isopropanolamine, triisopropanolamine, 2-amino-2-methyl-1,3-propanediol, 2-amino-2-hydroxymethyl-1,3-propanediol, arginine, sodium hydroxide, potassium hydroxide, aqueous ammonia, guanidine carbonate, and ammonium carbonate.

Preferred examples of powders include inorganic powders having various sizes and shapes, such as mica, talc, kaolin, sericite, montmorillonite, kaolinite, mica, muscovite, phlogopite, synthetic mica, lepidolite, biotite, vermiculite, magnesium carbonate, calcium carbonate, aluminum silicate, barium silicate, calcium silicate, magnesium silicate, strontium silicate, metal tungstates, magnesium, zeolite, barium sulfate, baked calcium sulfate, calcium phosphate (e.g., tricalcium phosphate), fluorapatite, hydroxyapatite, ceramic powder, bentonite, smectite, clay, mud, metallic soaps (e.g., zinc myristate, calcium palmitate, and aluminum stearate), calcium carbonate, red iron oxide, yellow iron oxide, black iron oxide, ultramarine, Prussian blue, carbon black, titanium oxide, titanium oxide fine particles and titanium oxide ultrafine particles, zinc oxide, zinc oxide fine particles and zinc oxide ultrafine particles, alumina, silica, fumed silica (silicic anhydride ultrafine particles), titanated mica, argentine, boron nitride, photochromic pigments, synthetic fluorophlogopite, fine particulate composite powders, gold, silver, platinum, and aluminum; inorganic powders, such as hydrophobic or hydrophilic powders prepared by treating the aforementioned powders with various surface-treating agents such as silicones (e.g., hydrogen silicone and cyclic hydrogen silicone) or other silanes or titanium coupling agents; organic powders having various sizes and shapes, such as starch, cellulose, nylon powder, polyethylene powder, polymethyl methacrylate powder, polystyrene powder, styrene-acrylic acid copolymer resin powder, polyester powder, benzoguanamine resin powder, polyethylene terephthalate-polymethyl methacrylate laminated powder, polyethylene terephthalate-aluminum-epoxy laminated powder, urethane powder, silicone powder, and Teflon (registered trademark) powder; surface-treated organic powders; and organic-inorganic composite powders.

Preferred examples of inorganic salts include sodium chloride-containing salts, such as common salt, regular

salt, rock salt, sea salt, and natural salt; potassium chloride, aluminum chloride, calcium chloride, magnesium chloride, bittern, zinc chloride, and ammonium chloride; sodium sulfate, aluminum sulfate, aluminum potassium sulfate (alum), aluminum ammonium sulfate, barium sulfate, calcium sulfate, potassium sulfate, magnesium sulfate, zinc sulfate, iron sulfate, and copper sulfate; and sodium phosphates, such as mono-, di-, and tri-sodium phosphates, potassium phosphates, calcium phosphates, and magnesium phosphates.

Preferred examples of ultraviolet absorbers include benzoic acid ultraviolet absorbers, such as p-aminobenzoic acid, p-aminobenzoic acid monoglycerin ester, N,N-dipropoxy-p-aminobenzoic acid ethyl ester, N,N-diethoxy-p-aminobenzoic acid ethyl ester, N,N-dimethyl-p-aminobenzoic acid ethyl ester, N,N-dimethyl-p-aminobenzoic acid butyl ester, and N,N-dimethyl-p-aminobenzoic acid methyl ester; anthranilic acid ultraviolet absorbers, such as homomenthyl-N-acetylanthranilate; salicylic acid ultraviolet absorbers, such as salicylic acid and sodium salt thereof, amyl salicylate, menthyl salicylate, homomenthyl salicylate, octyl salicylate, phenyl salicylate, benzyl salicylate, and p-isopropanolphenyl salicylate; cinnamic acid ultraviolet absorbers, such as octyl cinnamate, ethyl-4-isopropylcinnamate, methyl-2,5-diisopropylcinnamate, ethyl-2,4-diisopropylcinnamate, methyl-2,4-diisopropylcinnamate, propyl-p-methoxycinnamate, isopropyl-p-methoxycinnamate, isoamyl-p-methoxycinnamate, 2-ethylhexyl-p-methoxycinnamate (octyl p-methoxycinnamate), 2-ethoxyethyl-p-methoxycinnamate (cinoxate), cyclohexyl-p-methoxycinnamate, ethyl-α-cyano-β-phenylcinnamate, 2-ethylhexyl-α-cyano-β-phenylcinnamate (octocrylene), glyceryl mono-2-ethylhexanoyl-di-p-methoxycinnamate, ferulic acid, and derivatives thereof; benzophenone ultraviolet absorbers, such as 2,4-dihydroxybenzophenone, 2,2'-dihydroxy-4-methoxybenzophenone, 2,2'-dihydroxy-4,4'-dimethoxybenzophenone, 2,2',4,4'-tetrahydroxybenzophenone, 2-hydroxy-4-methoxybenzophenone (oxybenzone-3), 2-hydroxy-4-methoxy-4'-methylbenzophenone, 2-hydroxy-4-methoxybenzophenone-5-sulfonate, 4-phenylbenzophenone, 2-ethylhexyl-4'-phenyl-benzophenone-2-carboxylate, 2-hydroxy-4-n-octoxybenzophenone, and 4-hydroxy-3-carboxybenzophenone; 3-(4'-methylbenzylidene)-d,1-camphor and 3-benzylidene-d,1-camphor; 2-phenyl-5-methylbenzoxazole; 2,2'-hydroxy-5-methylphenylbenzotriazole; 2-(2'-hydroxy-5'-t-octylphenyl)benzotriazole; 2-(2'-hydroxy-5'-methylphenyl)benzotriazole; dibenzalazine; dianisoylmethane; 5-(3,3-dimethyl-2-norbornylidene)-3-pentan-2-one; dibenzoylmethane derivatives, such as 4-t-butylmethoxydibenzoylmethane; octyl triazone; urocanic acid and urocanic acid derivatives, such as ethyl urocanate; and 2-(2'-hydroxy-5'-methylphenyl)benzotriazole, 1-(3,4-dimethoxyphenyl)-4,4-dimethyl-1,3-pentanedione, hydantoin derivatives, such as 2-ethylhexyl dimethoxybenzylidene dioxoimidazolidine propionate, phenylbenzimidazole sulfonic acid, terephthalylidene dicamphor sulfonic acid, drometrizole trisiloxane, methyl anthranilate, rutin and derivatives thereof, and oryzanol and derivatives thereof.

Preferred examples of whitening agents include hydroquinone glycosides, such as arbutin and α-arbutin, and esters thereof; ascorbic acid, and ascorbic acid derivatives, for example, ascorbyl phosphates, such as sodium ascorbyl phosphate and magnesium ascorbyl phosphate, ascorbyl fatty acid esters, such as ascorbyl tetraisopalmitate, ascorbic acid alkyl ethers, such as ascorbic acid ethyl ether, ascorbic acid glucosides, such as ascorbic acid 2-glucoside and fatty acid esters thereof, ascorbyl sulfate, and tocopheryl ascorbyl phosphate; kojic acid, ellagic acid, tranexamic acid and derivatives thereof; ferulic acid and derivatives thereof; placenta extract, glutathione, oryzanol, butylresorecinol, and plant extracts, such as oil-soluble chamomilla extract, oil-soluble licorice extract, Tamarix chinensis extract, and Saxifraga stolonifera extract.

Preferred examples of vitamins and derivatives thereof include forms of vitamin A, such as retinol, retinol acetate, and retinol palmitate; forms of vitamin B, such as thiamine hydrochloride, thiamine sulfate, riboflavin, riboflavin acetate, pyridoxine hydrochloride, pyridoxine dioctanoate, pyridoxine dipalmitate, flavin adenine dinucleotide, cyanocobalamin, folic acid products, nicotinic acid products, such as nicotinamide and benzyl nicotinate, and choline products; forms of vitamin C, such as ascorbic acid and salts thereof, such as sodium salt; vitamin D; forms of vitamin E, such as α-, β-, γ-, and δ-tocopherols; other vitamins, such as pantothenic acid and biotin; ascorbic acid derivatives, for example, ascorbyl phosphates, such as sodium ascorbyl phosphate and magnesium ascorbyl phosphate, ascorbyl fatty acid esters, such as ascorbyl tetraisopalmitate, ascorbyl stearate, ascorbyl palmitate, and ascorbyl dipalmitate, ascorbic acid alkyl ethers, such as ascorbic acid ethyl ether, ascorbic acid glucosides, such as ascorbic acid-2-glucoside and fatty acid esters thereof, and tocopheryl ascorbyl phosphate; vitamin derivatives, for example, tocopherol derivatives, such as tocopherol nicotinate, tocopherol acetate, tocopherol linoleate, tocopherol ferulate, and tocopherol phosphate, tocotrienol, and other various vitamin derivatives.

Preferred examples of hair growth-promoting agents, blood circulation promoters, and stimulants include plant extracts and tinctures, such as swertia herb extract, capsicum tincture, ginger tincture, ginger extract, and cantharis tincture; capsaicin, nonylic acid vanillylamide, zingerone, ichthammol, tannic acid, borneol, cyclandelate, cinnarizine, tolazoline, acetylcholine, verapamil, cepharanthine, γ-oryzanol, vitamin E and derivatives thereof, such as tocopherol nicotinate and tocopherol acetate, γ-oryzanol, nicotinic acid and derivatives thereof, such as nicotinamide, benzyl nicotinate, inositol hexanicotinate, and nicotinic alcohol, allantoin, photosensitizer 301, photosensitizer 401, carpronium chloride, pentadecanoic acid monoglyceride, flavanonol derivatives, stigmasterol or stigmastanol and glycosides thereof, and minoxidil.

Preferred examples of hormones include estradiol, estrone, ethinylestradiol, cortisone, hydrocortisone, and

prednisone.

Preferred examples of other medicinal agents, such as anti-wrinkle agents, anti-aging agents, firming agents, cooling agents, warming agents, wound-healing promoters, abirritants, analgesics, and cell activators include retinol products, retinoic acid products, and tocopheryl retinoate; lactic acid, glycolic acid, gluconic acid, fruit acid, salicylic acid, and derivatives thereof, such as glycosides and esters, and $\alpha$- or $\beta$-hydroxy acids and derivatives thereof, such as hydroxycapric acid, long-chain $\alpha$-hydroxy fatty acids, and long-chain $\alpha$-hydroxy fatty acid cholesteryl esters; $\gamma$-aminobutyric acid and $\gamma$-amino-$\beta$-hydroxybutyric acid; carnitine; carnocin; creatine; ceramides and sphingosines; caffeine, xanthine, and derivatives thereof; antioxidants and active oxygen scavengers, such as coenzyme Q10, carotene, lycopene, astaxanthin, lutein, $\alpha$-lipoic acid, platinum nanocolloid, and fullerenes; catechins; flavones, such as quercetin; isoflavones; gallic acid and sugar ester derivatives thereof; polyphenols, such as tannin, sesamin, proanthocyanidin, chlorogenic acid, and apple polyphenols; rutin and derivatives thereof, such as glycosides; hesperidin and derivatives thereof, such as glycosides; lignan glycosides; licorice extract-related substances, such as glabridin, glabrene, liquiritin, and isoliquiritin; lactoferrin; shogaol and gingerol; perfume substances, such as menthol and cedrol, and derivatives thereof; capsaicin, vanillin, and derivatives thereof; insect repellents, such as diethyltoluamide; and complexes of physiologically active substances and cyclodextrins.

Preferred examples of plant, animal, and microbial extracts include iris extract, Angelica keiskei extract, Thujopsis dolabrata extract, asparagus extract, avocado extract, Hydrangea serrata leaf extract, almond extract, Althea officinalis root extract, Arnica montana extract, aloe extract, apricot extract, apricot kernel extract, ginkgo extract, Artemisia capillaris flower extract, fennel fruit extract, turmeric root extract, oolong tea extract, uva-ursi extract, rose fruit extract, Echinacea angustifolia leaf extract, Isodonis japonicus extract, Scutellaria root extract, Phellodendron bark extract, Coptis rhizome extract, barley extract, Panax ginseng extract, Hypericum perforatum extract, Lamium album extract, Ononis spinosa extract, Nasturtium officinale extract, orange extract, dried sea water residues, seaweed extract, Japanese persimmon leaf extract, Pyracantha fortuneana fruit extract, hydrolyzed elastin, hydrolyzed wheat powder, hydrolyzed silk, Pueraria root extract, Chamomilla recutita extract, oil-soluble Chamomilla recutita extract, carrot extract, Artemisia capillaris extract, Avena fatua extract, Hibiscus sabdariffa extract, licorice extract, oil-soluble licorice extract, kiwi fruit extract, kiou extract, Auricularia auricula-judae extract, Cinchona bark extract, cucumber extract, Paulownia tomentosa leaf extract, guanosine, guava extract, Sophora root extract, Gardenia jasminoides extract, Sasa veitchii extract, Sophora flavescens extract, walnut extract, chestnut extract, grapefruit extract, Clematis vitalba extract, black rice extract, black sugar extract, black vinegar, chlorella extract, mulberry extract, gentian extract, geranium herb extract, black tea extract, yeast extract, magnolia bark extract, coffee extract, burdock root extract, rice extract, fermented rice extract, fermented rice bran extract, rice germ oil, comfrey extract, collagen, bilberry extract, Asiasarum root extract, Bupleurum root extract, umbilical cord extract, saffron extract, salvia extract, Saponaria officinalis extract, bamboo grass extract, Crataegus cuneata fruit extract, Bombyx mori excrementum extract, Zanthoxylum piperitum peel extract, shiitake mushroom extract, Rehmannia glutinosa root extract, Lithospermum root extract, Perilla frutescens extract, Tilia japonica extract, Filipendula multijuga extract, jatoba extract, peony root extract, ginger extract, Acorus calamus root extract, Betula alba extract, Tremella fuciformis extract, Equisetum arvense extract, stevia extract, stevia fermentation product, Tamarix chinensis extract, Hedera helix extract, Crataegus oxycantha extract, Sambucus nigra extract, Achillea millefolium extract, Mentha piperita extract, sage extract, mallow extract, Cnidium rhizome extract, swertia herb extract, mulberry bark extract, rhubarb extract, soybean extract, jujubi extract, thyme extract, dandelion extract, lichens extract, tea extract, clove extract, Imperata cylindrica extract, Citrus unshiu peel extract, tea tree oil, Rubus suavissimus extract, capsicum extract, Angelica acutiloba root extract, Calendula officinalis extract, peach kernel extract, bitter orange peel extract, Houttuynia cordata extract, tomato extract, natto extract, carrot extract, garlic extract, Rosa canina fruit extract, hibiscus extract, Ophiopogon tuber extract, lotus extract, parsley extract, birch extract, honey, Hamamelis virginiana extract, Parietaria officinalis extract, Rabdosia japonica extract, bisabolol, cypress extract, Bifidobacterium extract, Eriobotiya japonica extract, Tussilago farfara extract, Japanese butterbur flower-bud extract, Poria cocos sclerotium extract, butcher's broom extract, grape extract, grape seed extract, propolis, Luffa cylindrica extract, safflower extract, peppermint extract, Tilia miqueliaria extract, Paeonia suffruticosa extract, hop extract, Rosa rugosa extract, pine extract, Aesculus hippocastanum extract, Lysichiton camtschatcense extract, Sapindus mukurossi extract, Melissa officinalis extract, Nemacystus decipiens extract, peach extract, cornflower extract, Eucalyptus globulus extract, Saxifraga stolonifera extract, Citrus junos extract, lily extract, coix seed extract, Artemisia princeps extract, lavender extract, green tea extract, egg shell membrane extract, apple extract, rooibos tea extract, Litchi chinensis extract, lettuce extract, lemon extract, Forsythia fruit extract, Astragalus sinicus extract, rose extract, rosemary extract, Anthemis nobilis extract, royal jelly extract, and Sanguisorba officinalis extract.

Examples of antipruritics include diphenhydramine hydrochloride, chlorpheniramine maleate, camphor, and substance-P inhibitors.

Examples of exfoliates/keratolytic agents include salicylic acid, sulfur, resorcin, selenium sulfide, and pyridoxine.

Examples of antiperspirants include aluminum chlorohydrate, aluminum chloride, zinc oxide, and zinc p-phenolsulfonate.

Examples of algefacients include menthol and methyl salicylate.

Examples of astringents include citric acid, tartaric acid, lactic acid, aluminum potassium sulfate, and tannic acid.

Examples of enzymes include superoxide dismutase, catalase, lysozyme chloride, lipase, papain, pancreatin, and protease.

Preferred examples of nucleic acids include ribonucleic acids and salts thereof, deoxyribonucleic acids and salts thereof, and adenosine triphosphate disodium.

Preferred examples of perfumes include synthetic and natural perfumes and various compound perfumes, such as acetyl cedrene, amylcinnamaldehyde, allylamyl glycolate, β-ionone, Iso E Super, isobutylquinoline, iris oil, irone, indole, ylang ylang oil, undecanal, undecenal, γ-undecalactone, estragole, eugenol, oakmoss, opoponax resinoid, orange oil, eugenol, aurantiol, galaxolide, carvacrol, L-carvone, camphor, canon, carrot seed oil, clove oil, methyl cinnamate, geraniol, geranyl nitrile, isobornyl acetate, geranyl acetate, dimethylbenzylcarbinyl acetate, styralyl acetate, cedryl acetate, terpinyl acetate, p-t-butylcyclohexyl acetate, vetiveryl acetate, benzyl acetate, linalyl acetate, isopentyl salicylate, benzyl salicylate, sandalwood oil, santalol, cyclamen aldehyde, cyclopentadecanolide, methyl dihydroj asmonate, dihydromyrcenol, jasmine absolute, jasmine lactone, cis-jasmone, citral, citronellol, citronellal, cinnamon bark oil, 1,8-cineole, cinnamaldehyde, styrax resinoid, cedarwood oil, cedrene, cedrol, celery seed oil, thyme oil, damascone, damascenone, thymol, tuberose absolute, decanal, decalactone, terpineol, γ-terpinen, triplal, nerol, nonanal, 2,6-nonadienol, nonalactone, patchouli alcohol, vanilla absolute, vanillin, basil oil, patchouli oil, hydroxycitronellal, α-pinene, piperitone, phenethyl alcohol, phenylacetaldehyde, petitgrain oil, hexylcinnamaldehyde, cis-3-hexenol, Peru balsam, vetiver oil, vetiverol, peppermint oil, pepper oil, heliotropin, bergamot oil, benzyl benzoate, borneol, myrrh resinoid, musk ketone, methylnonylacetaldehyde, γ-methylionone, menthol, L-menthol, L-menthone, eucalyptus oil, β-ionone, lime oil, lavender oil, D-limonene, linalool, lyral, lilial, lemon oil, rose absolute, rose oxide, rose oil, rosemary oil, and various essential oils.

Preferred examples of colors, coloring agents, and dyes include certified colors, such as Brown No. 201, Black No. 401, Violet No. 201, Violet No. 401, Blue No. 1, Blue No. 2, Blue No. 201, Blue No. 202, Blue No. 203, Blue No. 204, Blue No. 205, Blue No. 403, Blue No. 404, Green No. 201, Green No. 202, Green. No. 204, Green No. 205, Green No. 3, Green No. 401, Green No. 402, Red No. 102, Red No. 104-1, Red No. 105-1, Red No. 106, Red No. 2, Red No. 201, Red No. 202, Red No. 203, Red No. 204, Red No. 205, Red No. 206, Red No. 207, Red No. 208, Red No. 213, Red No. 214, Red No. 215, Red No. 218, Red No. 219, Red No. 220, Red No. 221, Red No. 223, Red No. 225, Red No. 226, Red No. 227, Red No. 228, Red No. 230-1, Red No. 230-2, Red No. 231, Red No. 232, Red No. 3, Red No. 401, Red No. 404, Red No. 405, Red No. 501, Red No. 502, Red No. 503, Red No. 504, Red No. 505, Red No. 506, Orange No. 201, Orange No. 203, Orange No. 204, Orange No. 205, Orange No. 206, Orange No. 207, Orange No. 401, Orange No. 402, Orange No. 403, Yellow No. 201, Yellow No. 202-1, Yellow No. 202-2, Yellow No. 203, Yellow No. 204, Yellow No. 205, Yellow No. 4, Yellow No. 401, Yellow No. 402, Yellow No. 403-1, Yellow No. 404, Yellow No. 405, Yellow No. 406, Yellow No. 407, and Yellow No. 5; other acid dyes, such as Acid Red 14; basic dyes, such as Arianor Sienna Brown, Arianor Madder Red, Arianor Steel Blue, and Arianor Straw Yellow; nitro dyes, such as HC Yellow 2, HC Yellow 5, HC Red 3, 4-hydroxypropylamino-3-nitrophenol, N,N'-bis(2-hydroxyethyl)-2-nitro-p-phenylenediamine, HC Blue 2, and Basic Blue 26; disperse dyes; natural colors and dyes, for example, anthraquinones, such as astaxanthin and alizarin, naphthoquinones, such as anthocyanidin, β-carotene, catenal, capsanthin, chalcone, carthamin, quercetin, crocin, chlorophyll, curcumin, cochineal, and shikonin, bixin, flavones, betacyanidine, henna, hemoglobin, lycopene, riboflavin, and rutin; oxidation dye intermediates and couplers, such as p-phenylenediamine, toluene-2,5-diamine, o-, m-, or p-aminophenol, m-phenylenediamine, 5-amino-2-methylphenol, resorcin, 1-naphthol, and 2,6-diaminopyridine, and salts thereof; autoxidation dyes, such as indoline; and dihydroxyacetone.

Preferred examples of antiphlogistics and anti-inflammatory agents include glycyrrhizic acid and derivatives thereof, glycyrrhetic acid derivatives, salicylic acid derivatives, hinokitiol, guaiazulene, allantoin, indomethacin, ketoprofen, ibuprofen, diclofenac, loxoprofen, celecoxib, infliximab, etanercept, zinc oxide, hydrocortisone acetate, prednisone, diphedramine hydrochloride, and chlorpheniramine maleate; and plant extracts, such as peach leaf extract and Artemisia princeps leaf extract.

Preferred examples of anti-asthmatic agents, anti-chronic obstructive pulmonary disease agents, anti-allergic agents, and immunomodulators include aminophylline, theophyllines, steroids (e.g., fluticasone and beclomethasone), leukotriene antagonists, thromboxane inhibitors, Intal, β2 agonists (e.g., formoterol, salmeterol, albuterol, tulobuterol, clenbuterol, and epinephrine), tiotropium, ipratropium, dextromethorphan, dimemorfan, bromhexine, tranilast, ketotifen, azelastine, cetirizine, chlorpheniramine, mequitazine, tacrolimus, ciclosporin, sirolimus, methotrexate, cytokine modulators, interferon, omalizumab, and protein/antibody preparations.

Preferred examples of anti-infective agents and antifungal agents include oseltamivir, zanamivir, and itraconazole. The composition may contain, besides the aforementioned ingredients, known cosmetic ingredients, known pharmaceutical ingredients, and known food ingredients, such as ingredients described in Japanese Standards of Cosmetic Ingredients, Japanese Cosmetic Ingredients Codex, List of Cosmetics Ingredients Japanese Labeling Names issued by the Japan Cosmetic Industry Association, INCI dictionary (The International Cosmetic Ingredient Dictionary and Handbook), Japanese Standards of Quasi-drug Ingredients, Japanese Pharmacopoeia, Japanese Pharmaceutical Excipients,

Japan's Specifications and Standards for Food Additives, and other standards, and ingredients described in Japanese and foreign patent publications and patent application publications (including Japanese translations of PCT international application publications and re-publications of PCT international publications) classified as International Patent Classification IPC classes A61K7 and A61K8, in known combinations and in known proportions and amounts.

[Method for Producing Coating Film-Forming Composition]

The coating film-forming composition of the present invention can be produced by, for example, mixing at least one lipidic peptide compound, water, and optionally other ingredients under heating, stirring the resultant mixture, and then allowing the mixture to stand still to cool.

[Method for Producing Coating Film]

The present invention is also directed to a method for producing a coating film, the method being characterized by comprising using the aforementioned coating film-forming composition.

No particular limitation is imposed on the coating film production method, so long as the method can produce a coating film. For example, the method involves application of the aforementioned coating film-forming composition to a target, and subsequent drying of the applied composition by, for example, natural drying or heat drying.

Examples

The present invention will next be described in more detail by way of examples. However, the present invention should not be construed as being limited to the following examples.

The commercially available reagents described below were used in Synthesis Examples. Synthesized compounds were analyzed with analytical instruments described below.

<Reagents>

Methanol: available from Kanto Chemical Co., Inc. (special grade)
i-Propanol: available from Kanto Chemical Co., Inc. (first grade)
Toluene: available from Kanto Chemical Co., Inc. (first grade)
Acetic acid: available from Kanto Chemical Co., Inc. (first grade)
Glycine methyl ester hydrochloride: available from Tokyo Chemical Industry Co., Ltd.
L-Histidine: available from Tokyo Chemical Industry Co., Ltd. and KYOWA HAKKO BIO Co., Ltd.
Sodium methoxide 28% methanol solution: available from Wako Pure Chemical Industries, Ltd. (28% Sodium Methoxide Methanol Solution)
Sodium carbonate: available from JUNSEI CHEMICAL CO., LTD. (first grade)
Hydrochloric acid: available from Kanto Chemical Co., Inc. (first grade)
Acetonitrile: available from Kanto Chemical Co., Inc. (special grade)
ISTA: 2-(4,4-dimethylpentan-2-yl)-5,7,7-trimethyloctanoic acid [FINEOXOCOL (registered trademark) ISOSTEARIC ACID, available from Nissan Chemical Corporation]
2-Heptylundecanoic acid: [available from Nissan Chemical Corporation]
Thionyl chloride: available from Tokyo Chemical Industry Co., Ltd.
Acetone: available from Kanto Chemical Co., Inc. (first grade)
Tetrahydrofuran: available from Kanto Chemical Co., Inc. (first grade)
Ethyl acetate: available from Kanto Chemical Co., Inc. (first grade)
N,N-dimethylformamide (DMF): available from Kanto Chemical Co., Inc. (first grade)

<Analytical Instruments>

NMR: JNM-ECP300 (available from JEOL Ltd.)
LC-MS: Waters ACQUITY
pH Meter: available from METTLER TOLEDO

[Synthesis Example 1: Synthesis of Lipidic Peptide Compound (N-Palmitoyl-Gly-His)]

A 500 mL four-necked flask was charged with 14.2 g (91.6 mmol) of L-histidine, 30.0 g (91.6 mmol) of N-palmitoyl-Gly-methyl, and 300 g of toluene, and 35.3 g (183.2 mmol) of a 28% methanol solution of sodium methoxide serving as a base was added to the flask. The mixture was heated to 60°C in an oil bath and continuously stirred for one hour. Thereafter, the oil bath was removed, and the mixture was allowed to cool to 25°C. To the resultant solution was added 600 g of acetone for reprecipitation, and the precipitate was separated by filtration. The resultant solid was dissolved in a mixed solution of 600 g of water and 750 g of methanol. To the solution was added 30.5 mL (183.2 mmol) of 6N hydrochloric acid to thereby neutralize the solution and precipitate a solid, and the solid was filtered. Subsequently, the resultant solid was dissolved in a mixed solution of 120 g of tetrahydrofuran and 30 g of water at 60°C, and 150 g of ethyl acetate was added to the solution. The resultant mixture was cooled from 60°C to 30°C, and then the precipitated solid was filtered. The resultant solid was dissolved in a solvent mixture of 120 g of tetrahydrofuran and 60 g of acetonitrile. The solution was heated to 60°C, stirred for one hour, and then cooled, followed by filtration. The resultant solid was washed with 120 g of water, filtered, and then dried under reduced pressure, to thereby produce 26.9 g of a free form of N-palmitoyl-Gly-His (hereinafter may be referred to simply as "Pal-GH") as white crystals (yield: 65%).

[Synthesis Example 2: Synthesis of Lipidic Peptide Compound (2-(4,4-Dimethylpentan-2-yl)-5,7,7-trimethyloctanoylglycylhistidine)]

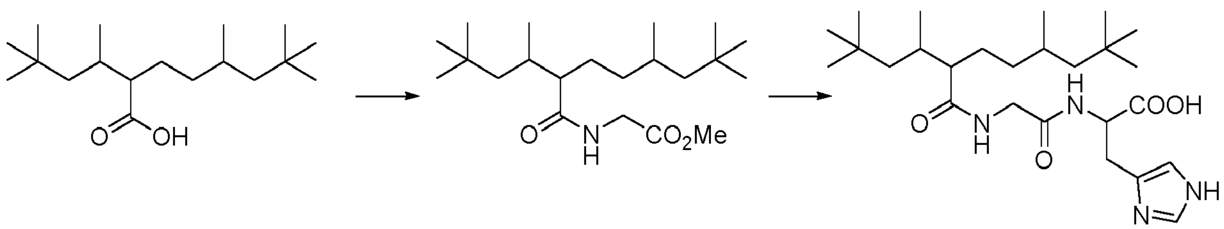

(1) Synthesis of 2-(4,4-Dimethylpentan-2-yl)-5,7,7-trimethyloctanoylglycine methyl ester

A 100 mL four-necked flask was charged with 20.0 g (70.3 mmol) of ISTA, 154 mg of DMF, and 20.0 g of toluene, and the temperature was adjusted to 0°C. Subsequently, 8.78 g (73.8 mmol) of thionyl chloride was added dropwise to the flask over 15 minutes, and then the resultant mixture was gradually heated to 30°C. The mixture was stirred at 30°C for two hours, and then 20.0 g of toluene was added to the mixture. The resultant mixture was heated to 50°C, and 20 g of toluene was distilled off under reduced pressure, to thereby remove thionyl chloride. This operation was repeated twice (acid chloride solution).

Separately from the aforementioned reaction container, a 500 mL four-necked flask was charged with 40.0 g of ion-exchange water, 11.5 g (91.4 mmol) of glycine methyl ester hydrochloride, and 80.0 g of toluene, and 8.6 g (80.9 mmol) of sodium carbonate serving as a base and 60.0 g of ion-exchange water were added to the flask, followed by stirring. Thereafter, the aforementioned acid chloride solution was added dropwise to the resultant mixture at a reaction temperature of 25 $\pm$ 5°C over one hour, and the mixture was stirred at 25°C for three hours. Subsequently, 200.0 g of 10% saline water was added to the mixture, and then phase separation was performed at 60°C. To the resultant organic phase was added 160.0 g of toluene, and the mixture was subjected to azeotropic dehydration, to thereby prepare 24.5 g of 2-(4,4-dimethylpentan-2-yl)-5,7,7-trimethyloctanoylglycine methyl ester (yield: 98%).

(2) Synthesis of 2-(4,4-Dimethylpentan-2-yl)-5,7,7-trimethyloctanoylglycylhistidine

A 500 mL four-necked flask was charged with 10.7 g (68.9 mmol) of L-histidine and 49.0 g of toluene, and 12.6 g (65.5 mmol) of a 28% methanol solution of sodium methoxide serving as a base was added dropwise to the flask. To the resultant mixture were added 24.5 g (68.9 mmol) of 2-(4,4-dimethylpentan-2-yl)-5,7,7-trimethyloctanoylglycine methyl ester prepared in (1) of Synthesis Example 2 and 9.8 g of methanol, and the mixture was heated to 70°C. Thereafter, dropwise addition of 10.0 g (51.7 mmol) of a 28% methanol solution of sodium methoxide serving as a base was initiated, and stirring was continued at about 70°C for three hours. After completion of the reaction, 5.0 g (82.6 mmol) of acetic acid was added to adjust the pH of the reaction mixture to 7, and 122.5 g of ion-exchange water and 49.0 g of i-propanol were added to the mixture, followed by phase separation at 60°C. To the resultant aqueous phase was added 13.8 g (82.7 mmol) of 6N hydrochloric acid, and 24.5 g of methanol was added to the mixture. Subsequently, the resultant

mixture was added to 489.8 g of acetonitrile for reprecipitation. The resultant slurry was filtered, and the wet product was dissolved in methanol. Thereafter, 4.9 g of activated carbon (TOKUSEI SHIRASAGI) was added to the solution, and the mixture was stirred for one hour. Subsequently, the activated carbon was separated by filtration, and the resultant product was added to 489.8 g of acetonitrile for reprecipitation. The resultant wet product was dried at 60°C under reduced pressure, to thereby prepare 26.6 g of 2-(4,4-dimethylpentan-2-yl)-5,7,7-trimethyloctanoylglycylhistidine (hereinafter may be referred to simply as "FO-GH") (yield: 86%).

$^1$H-NMR (300MHz, DMSO-$d_6$, δ ppm): 8.02 (2H, m), 7.55 (1H, s), 6.80 (1H, s), 4.38 (1H, m), 3.68 (2H, m), 2.88 (2H, m), 1.94 (1H, m), 1.51 (4H, m), 1.12 (3H, m), 0.87 (27H, m)

MS (ESI) m/z: 479.29 ($M+H^+$)

[Synthesis Example 3: Synthesis of Lipidic Peptide Compound (2-heptylundecanoylglycylhistidine)]

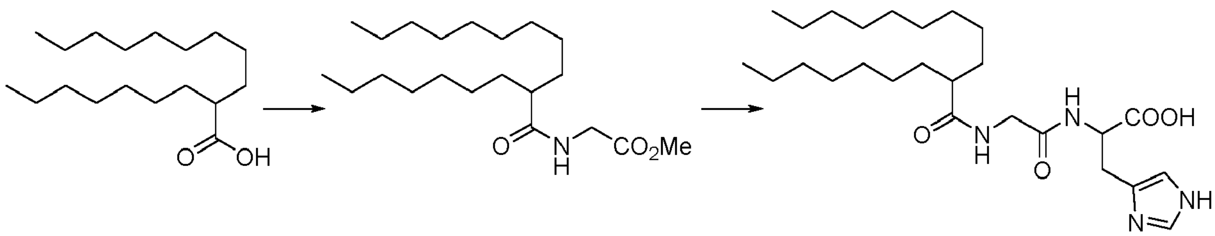

(1) Synthesis of 2-heptylundecanoylglycine methyl ester

The same procedure as in (1) of Synthesis Example 2 was performed, except that 2-heptylundecanoic acid was used as a raw material, to thereby prepare 24.1 g of 2-heptylundecanoic acid glycine methyl ester (yield: 96%)

(2) Synthesis of 2-heptylundecanoylglycylhistidine

The same procedure as in (2) of Synthesis Example 2 was performed, except that 2-(4,4-dimethylpentan-2-yl)-5,7,7-trimethyloctanoylglycine methyl ester was replaced with 2-heptylundecanoic acid glycine methyl ester, to thereby prepare 23.1 g of 2-heptylundecanoylglycylhistidine (hereinafter may be referred to simply as "IS-Ste-GH") (yield: 86%).

$^1$H-NMR (300 MHz, DMSO-$d_6$, δ ppm): 8.07 (1H, t, J = 6.0 Hz), 8.04 (1H, d, J = 7.5 Hz), 7.57 (1H, s), 6.82 (1H, s), 4.39 (1H, q, J = 7.5 Hz), 3.68 (2H, m), 2.28 (2H, m), 2.16 (1H, m), 1.42 (2H, m), 1.21 (26H, m), 0.85 (3H, m)

MS (ESI) m/z: 479.18 ($M+H^+$)

[Preparation Example 1: Preparation of Premix]

A 300 mL beaker was charged with Pal-GH and, as additives, stearic acid, 1,2-hexanediol, polyoxyethylene lauryl ether (available from Nikko Chemicals Co., Ltd.), and water in proportions shown in Table 1 below, and the mixture was stirred at 200 rpm under heating at a liquid temperature of 80°C, to thereby prepare a homogeneous solution. The solution was cooled while being stirred. After the liquid temperature reached 60°C, the solution was allowed to stand still to cool, to thereby prepare ES-01 premix.

Table 1

| Composition of ES-01 Premix | |
|---|---|
| Ingredient | Composition (wt%) |
| Pal-GH | 10.0 |
| Stearic acid*1 | 1.0 |
| 1,2-Hexanediol*2 | 4.0 |
| Polyoxyethylene lauryl ether*3 | 8.0 |

(continued)

| Composition of ES-01 Premix | |
|---|---|
| Ingredient | Composition (wt%) |
| Pure water | 77.0 |
| *1: available from Kao Corporation [trade name: S-98]<br>*2: available from ITO Inc.<br>*3: available from Nikko Chemicals Co., Ltd.: POE (4.2) lauryl ether | |

[Example 1] Gelation Test and Observation of Gel Form

A 300 mL tall beaker was charged with propylene glycol alginate, POE 60 hydrogenated castor oil, mineral oil, and pure water as shown in Table 2, and the mixture was stirred under heating at 80°C. The stirring was performed at 200 rpm with LABORATORY HIGH MIXER available from AS ONE CORPORATION. Subsequently, ES-01 premix heated to 80°C was added to the mixture, and the resultant mixture was further stirred under heating for five minutes. After completion of the thermal stirring, the mixture was cooled with stirring until the liquid temperature reached about 50°C, and then gel formation was determined. Gel formation was determined by the test tube inversion method. Specifically, the liquid was determined to undergo "gelation" in the case where the liquid (i.e., dispersion) lost its fluidity, and the liquid did not flow down even when the tall beaker was inverted.

The resultant gel was applied onto BIOSKIN (available from Beaulax Co., Ltd.) so as to achieve 10 mg/cm$^2$, and the form of the applied gel was observed with a scanning electron microscope (SEM) [Miniscope (registered trademark) TM3000 (available from Hitachi High-Technologies Corporation)]. FIG. 1 shows an image of the sample observed immediately after being set in the scanning electron microscope, and an image of the sample observed after being set and dried in the scanning electron microscope for a predetermined period of time.

[Comparative Example 1] Gelation Test

A 300 mL tall beaker was charged with propylene glycol alginate, POE 60 hydrogenated castor oil, mineral oil, and pure water as shown in Table 2, and the mixture was stirred under heating at 80°C. The stirring was performed at 200 rpm with LABORATORY HIGH MIXER available from AS ONE CORPORATION. After completion of the thermal stirring, the mixture was cooled with stirring until the liquid temperature reached about 50°C.

Table 2

| Ingredient | Example 1 | Comparative Example 1 |
|---|---|---|
| Propylene glycol alginate*4 | 0.2 g | 0.2 g |
| POE 60 hydrogenated castor oil*5 | 2.5 g | 2.5 g |
| Mineral oil*6 | 5.0 g | 5.0 g |
| Pure water | 82.3 g | 92.3 g |
| ES-01 premix | 10.0 g | None |
| Total amount | 100.0 g | 100.0 g |
| Form of composition | Gel | Liquid |
| *4: available from KIMICA Corporation, HV grade<br>*5: available from Nikko Chemicals Co., Ltd.<br>*6: available from PINOA Co., Ltd. | | |

[Example 2]

A 300 mL tall beaker was charged with propylene glycol alginate, POE 60 hydrogenated castor oil, and pure water as shown in Table 3, and the mixture was stirred under heating at 80°C. The stirring was performed at 200 rpm with LABORATORY HIGH MIXER available from AS ONE CORPORATION. Subsequently, ES-01 premix heated to 80°C was added to the mixture, and the resultant mixture was further stirred under heating for five minutes. After completion of the thermal stirring, the mixture was cooled with stirring until the liquid temperature reached about 50°C,

and then gel formation was determined. Gel formation was determined by the test tube inversion method. Specifically, the liquid was determined to undergo "gelation" in the case where the liquid (i.e., dispersion) lost its fluidity, and the liquid did not flow down even when the tall beaker was inverted.

[Comparative Example 2]

A 300 mL tall beaker was charged with propylene glycol alginate, POE 60 hydrogenated castor oil, and pure water as shown in Table 3, and the mixture was stirred under heating at 80°C. The stirring was performed at 200 rpm with LABORATORY HIGH MIXER available from AS ONE CORPORATION. After completion of the thermal stirring, the mixture was cooled with stirring until the liquid temperature reached about 50°C.

Table 3

| Ingredient | Example 2 | Comparative Example 2 |
|---|---|---|
| Propylene glycol alginate*4 | 0.2 g | 0.2 g |
| POE 60 hydrogenated castor oil*5 | 2.5 g | 2.5 g |
| Pure water | 87.3 g | 97.3 g |
| ES-01 premix | 10.0 g | None |
| Total amount | 100.0 g | 100.0 g |
| Form of composition | Gel | Liquid |
| *4: available from KIMICA Corporation, HV grade<br>*5: available from Nikko Chemicals Co., Ltd. | | |

[Example 3 and Comparative Example 3] Measurement of Reflected Light Intensity

The gel obtained in Example 2 or the liquid of Comparative Example 2 was weighed as shown in Table 4, and squalane was added thereto. After addition of squalane, a homogeneous thickened product or liquid was prepared by use of a spatula.

Subsequently, the thickened product of Example 3 or the liquid of Comparative Example 3 shown in Table 4 was applied onto BIOSKIN (available from Beaulax Co., Ltd.) so as to achieve 10 $mg/cm^2$, followed by drying in a thermostatic chamber at 35°C. The reflected light intensity of the resultant coating film at 30° (incident light: -30°) was measured with Goniophotometer GP-5 (available from MURAKAMI COLOR

RESEARCH LABORATORY).

FIG. 2 and FIG. 3 respectively show appearance photographs and SEM images in Example 3 and Comparative Example 3.

Table 4

| Ingredient | Example 3 | Comparative Example 3 |
|---|---|---|
| Example 2 | 0.5 g | |
| Comparative Example 2 | | 0.5 g |
| Squalane*7 | 0.1 g | 0.1 g |
| Appearance of composition | Thickened product | Liquid |
| Reflective light intensity at -30° | 47.0 | 95.8 |
| *7: available from Nikko Chemicals Co., Ltd. | | |

According to the results shown in Table 4, the coating film formed from the composition of the present invention (Example 3) exhibited a reflective light intensity about half that of the coating film formed from the composition containing no lipidic peptide compound (Comparative Example 3).

Thus, the coating film-forming composition of the present invention successfully formed a coating film capable

of reducing light reflection.

[Example 4 and Comparative Example 4] Moisturizing Test of Shampoo

As shown in Table 5, phase A was weighed in a 300 mL tall beaker. Separately, ES-01 premix was weighed and dissolved in purified water heated to 70°C, to thereby prepare phase B. Phase B was added to phase A heated to 70°C, and then the mixture was cooled with stirring until the liquid temperature reached about 40°C.

Table 5

| Phase | Ingredient | Example 4 | Comparative Example 4 |
|---|---|---|---|
| A | Purified water | 44.75% | 44.75% |
| | Cocoyl glutamate Na*8 | 7.30% | 7.30% |
| | Cocoyl glycine K*9 | 2.40% | 2.40% |
| | Cocoyl taurine Na*10 | 4.20% | 4.20% |
| | Cocamidopropyl betaine*11 | 12.00% | 12.00% |
| | Coconut oil fatty acid diethanolamide*12 | 4.00% | 4.00% |
| | 1,3-BG*13 | 4.00% | 4.00% |
| | Citric acid*14 | 0.35% | 0.35% |
| | Polyquaternium-7*15 | 0.50% | 0.50% |
| | Phenoxyethanol*16 | 0.50% | 0.50% |
| B | Purified water | 19.50% | 20.00% |
| | ES-01 premix | 0.50% | |
| Total | | 100.00% | 100.00% |

*8: Amisoft CS-11 available from Ajinomoto Co., Inc.
*9: Amilite GCK-11 available from Ajinomoto Co., Inc.
*10: Neoscoap CTS-25
*11: Anpholex
*12: Amycol CDE-1
*13: 1,3-butylene glycol available from ITO Inc.
*14: available from JUNSEI CHEMICAL CO., LTD.
*15:MERQUAT(TM) 550 POLYMER available from The Lubrizol Corporation
*16: available from Tokyo Chemical Industry Co., Ltd.

A human hair bundle (black hair, 1 g, 10 cm) (available from Ketabaya) was rubbed 10 times with Kikulon sponge, and then the surface that was not brought into contact with the Kikulon sponge was rubbed 10 times similarly. A 50 mL sample vial was charged with 10 g of the composition of Example 4 or Comparative Example 4, and the above-treated hair bundle was added to the sample vial, followed by stirring with a mix rotor for three minutes. A 300 mL tall beaker was charged with about 300 mL of pure water, and the hair bundle immersed in the shampoo was added to the beaker and washed with the pure water for 30 seconds. Subsequently, the hair bundle was washed again with about 300 mL of pure water separately provided in a 300 mL tall beaker. The hair bundle wetted with water was placed on Kojin wiper for five seconds, and then the surface that was not brought into contact with the Kojin wiper was placed on the wiper for five seconds. The rate of moisture retention was calculated by use of the following formula. The results are shown in FIG. 4.

$$\text{Rate of moisture retention (\%)} = (\text{The mass of hair after the elapse of a predetermined period of time} - \text{the mass of hair before immersion in shampoo})/\text{the mass of hair before immersion in shampoo} \times 100\%$$

[Examples 5 to 8] Moisturizing Test of Shampoo

As shown in Table 6, ingredients were weighed in a 300 mL tall beaker. The mixture was heated to 70°C to thereby prepare a homogeneous solution. Thereafter, the solution was cooled with stirring until the liquid temperature reached about 40°C. Subsequently, the rate of moisture retention was determined in the same manner as in Example 4. The results are shown in FIG. 4. The term "Pal-H" refers to palmitoyl histidine.

Table 6

| Ingredient | Example 5 | Example 6 | Example 7 | Example 8 |
|---|---|---|---|---|
| Purified water | 44.75% | 44.75% | 44.75% | 44.75% |
| Cocoyl glutamate Na*8 | 7.30% | 7.30% | 7.30% | 7.30% |
| Cocoyl glycine K*9 | 2.40% | 2.40% | 2.40% | 2.40% |
| Cocoyl taurine Na*10 | 4.20% | 4.20% | 4.20% | 4.20% |
| Cocamidopropyl betaine*11 | 12.00% | 12.00% | 12.00% | 12.00% |
| Coconut oil fatty acid diethanolamide*12 | 4.00% | 4.00% | 4.00% | 4.00% |
| 1,3-BG*13 | 4.00% | 4.00% | 4.00% | 4.00% |
| Citric acid*14 | 0.35% | 0.35% | 0.35% | 0.35% |
| Polyquaternium-7*15 | 0.50% | 0.50% | 0.50% | 0.50% |
| Phenoxyethanol*16 | 0.50% | 0.50% | 0.50% | 0.50% |
| Purified water | 19.95% | 19.95% | 19.95% | 19.95% |
| Pal-GH | 0.05% | | | |
| IS-Ste-GH | | 0.05% | | |
| FO-GH | | | 0.05% | |
| Pal-H | | | | 0.05% |
| Total | 100.00% | 100.00% | 100.00% | 100.00% |

According to the results shown in FIG. 4, the shampoo containing a lipidic peptide compound (Examples 4 to 8) exhibited a rate of moisture retention in hair higher than that of the shampoo containing no lipidic peptide compound (Comparative Example 4). In the case where the lipidic peptide compound was Pal-GH (Examples 4 and 5), IS-Ste-GH (Example 6), and FO-GH (Example 7), the rate of moisture retention in hair was particularly improved, and a superior moisturizing effect was achieved.

[Example 9 and Comparative Example 5]

As shown in Table 7, phase B and phase C were dissolved under heating. Phase D was heated and subjected to treatment with a homomixer (5,000 rpm, three minutes). Thereafter, the above-heated phase B and phase C were added to phase D, and the resultant mixture was subjected to treatment with the homomixer (5,000 rpm, three minutes). After the treatment with the homomixer, phase A was slowly added to the mixture, and the mixture was then subjected to treatment with the homomixer (5,000 rpm, three minutes). Thereafter, the mixture was cooled with stirring until the liquid temperature reached about 40°C.

Table 7

| Phase | Ingredient | Example 9 | Comparative Example 5 |
|---|---|---|---|
| A | NIKKOL Nikkomulese LC*17 | 4.00 | 4.00 |
| | Kalcohl*18 | 1.00 | 1.00 |
| | Squalane oil*19 | 5.00 | 5.00 |
| | Silicone oil*20 | 5.00 | 5.00 |

(continued)

| Phase | Ingredient | Example 9 | Comparative Example 5 |
|---|---|---|---|
| B | Xanthan gum*21 | 0.15 | 0.15 |
| | EDTA-2Na*22 | 0.30 | 0.30 |
| | Methylparaben*23 | 0.05 | 0.05 |
| | Purified water | 71.05 | 71.35 |
| C | Arginine*24 | 0.05 | 0.05 |
| | Purified water | 3.00 | 3.00 |
| | ES-01 premix | 0.30 | |
| D | BG*25 | 4.00 | 4.00 |
| | Glycerin*26 | 1.00 | 1.00 |
| | Titanium oxide*27 | 5.00 | 5.00 |
| | Red titanium oxide*28 | 0.10 | 0.10 |
| Total | | 100.00 | 100.00 |

*17: available from Nikko Chemicals Co., Ltd.
*18: Kalcohl 6098 available from Kao Corporation
*19: available from Nikko Chemicals Co., Ltd.
*20: KF-96A-100CS available from Shin-Etsu Chemical Co., Ltd.
*21: KELCOGEL-CF-LA available from Sansho Co., Ltd.
*22: available from JUNSEI CHEMICAL CO., LTD.
*23: available from Tokyo Chemical Industry Co., Ltd.
*24: available from Tokyo Chemical Industry Co., Ltd.
*25: 1,3-butylene glycol available from ITO Inc.
*26: available from ITO Inc.
*27: available from Orangeflower
*28: available from Orangeflower

Firstly, 100 μL of the composition of Example 9 or Comparative Example 5 was uniformly applied to a cut artificial leather piece having dimensions of 2 cm × 2 cm, and the applied composition was dried at room temperature for 30 minutes. Thereafter, contact angle was evaluated with a contact angle meter Drop Master DMC-MCS (available from KYOWA INDUSTRIAL CO., LTD.). The results are shown in FIG. 5.

According to the results shown in FIG. 5, the coating film formed from the composition of the present invention (Example 9) exhibited a contact angle higher than that of the coating film formed from the composition containing no lipidic peptide compound (Comparative Example 5).

Thus, the coating film-forming composition of the present invention successfully formed a coating film exhibiting good water repellency.

[Example 10 and Comparative Example 6] Moisturizing Test of Conditioner

As shown in Table 8, phase A was weighed and heated to 75°C to thereby prepare a homogeneous solution. Separately, ingredients of phase B (except for ES-01 premix) were heated to 75°C, and ES-01 premix heated to 75°C was added to the heated mixture. Subsequently, phase B was added to phase A heated to 75°C, and the resultant mixture was cooled with stirring to room temperature or thereabouts.

Table 8

| Phase | Ingredient | Example 10 | Comparative Example 6 |
|---|---|---|---|
| A | NIKKOL CA-2580*29 | 2.40% | 2.40% |
| | NIKKOL CA-3475V*30 | 0.80% | 0.80% |
| | Kalcohl 6098*31 | 5.00% | 5.00% |
| | Oleyl alcohol VP*32 | 1.00% | 1.00% |
| | NIKKOL MGS-BV2*33 | 1.00% | 1.00% |
| | NIKKOL Nikkowax LM*34 | 1.00% | 1.00% |
| | NIKKOL Olive Oil*35 | 2.00% | 2.00% |
| | NOMCORT W*36 | 8.00% | 8.00% |
| | KF-96A-100CS*37 | 1.00% | 1.00% |
| | KF-96-1000CS*38 | 0.50% | 0.50% |
| | Riken E Oil 700*39 | 0.50% | 0.50% |
| B | Concentrated glycerin for cosmetics*40 | 6.00% | 6.00% |
| | Phenoxyethanol*41 | 0.50% | 0.50% |
| | ES-01 premix | 0.50% | |
| | Purified water*42 | 69.80% | 70.30% |
| Total | | 100.00% | 100.00% |

*29: available from Nikko Chemicals Co., Ltd.
*30: available from Nikko Chemicals Co., Ltd.
*31: available from Kao Corporation
*32: available from KOKYU ALCOHOL KOGYO CO., LTD.
*33: available from Nikko Chemicals Co., Ltd.
*34: available from Nikko Chemicals Co., Ltd.
*35: available from Nikko Chemicals Co., Ltd.
*36: available from The Nisshin OilliO Group, Ltd.
*37: available from Shin-Etsu Chemical Co., Ltd.
*38: available from Shin-Etsu Chemical Co., Ltd.
*39: available from RIKEN VITAMIN CO., LTD.
*40: available from Sakamoto Yakuhin Kogyo Co., Ltd.
*41: available from Tokyo Chemical Industry Co., Ltd.
*42: available from Otsuka Pharmaceutical Co., Ltd.

A human hair bundle (black hair, 1 g, 10 cm) (available from Ketabaya) was rubbed 10 times with Kikulon sponge, and then the surface that was not brought into contact with the Kikulon sponge was rubbed 10 times similarly. Subsequently, 4.0 g of the composition (conditioner) of Example 10 or Comparative Example 6 was applied to the above-treated hair bundle, and the composition was spread over the hair bundle with hands. Thereafter, the hair bundle was immersed and shaken in about 300 mL of pure water charged in a 300 mL tall beaker, to thereby wash the hair bundle for 30 seconds. Subsequently, the hair bundle was washed again with about 300 mL of pure water separately provided in a 300 mL tall beaker. The hair bundle wetted with water was placed on Kojin wiper for five seconds, and then the surface that was not brought into contact with the Kojin wiper was placed on the wiper for five seconds. The rate of moisture retention was appropriately calculated by use of the following formula. The results are shown in FIG. 6.

After completion of the test, the resultant hair was observed with a scanning electron microscope (SEM). The results are shown in FIG. 7.

$$\text{Rate of moisture retention (\%)} = (\text{The mass of hair after the elapse of a predetermined period of time} - \text{the mass of hair before application of conditioner})/\text{the mass of hair before application of conditioner} \times 100\%$$

According to the results shown in FIG. 6, the conditioner containing a lipidic peptide compound (Example 10) exhibited a rate of moisture retention in hair higher than that of the conditioner containing no lipidic peptide compound (Comparative Example 6); i.e., the conditioner of Example 10 exhibited a superior moisturizing effect.

As shown in SEM images of FIG. 7, the conditioner containing a lipidic peptide compound formed a coating film on hair, whereas the conditioner containing no lipidic peptide compound did not form a coating film on hair.

[Formulation Example 1] Oil-Free Cleansing Product

According to compositions shown in Table 9, A, B, C, and D were prepared and mixed to thereby formulate oil-free cleansing products.

Table 9

| Ingredient | | (1) | (2) | (3) |
|---|---|---|---|---|
| A | PEG-7 glyceryl cocoate | 10.00% | 10.00% | 10.00% |
| | PEG-20 glyceryl triisostearate | 4.50% | 4.50% | 4.50% |
| | PEG-20 sorbitan isostearate | 3.50% | 3.50% | 3.50% |
| | PEG-45 stearate | 2.00% | 2.00% | 2.00% |
| | DPG | 20.00% | 20.00% | 20.00% |
| | Phenoxyethanol | 0.50% | 0.50% | 0.50% |
| B | Methyl gluceth-10 | 5.00% | 5.00% | 5.00% |
| | Carbomer | 0.40% | 0.40% | 0.40% |
| | Purified water | 43.86% | 42.86% | 40.86% |
| C | Purified water | 10.00% | 10.00% | 10.00% |
| | Potassium hydroxide | 0.14% | 0.14% | 0.14% |
| | EDTA-2Na | 0.10% | 0.10% | 0.10% |
| D | ES-01 premix | 0.00% | 1.00% | 3.00% |
| | | 100.00% | 100.00% | 100.00% |

[Formulation Example 2] Liquid Crystal Cream

According to compositions shown in Table 10, A, B, C, D, and E were prepared and mixed to thereby formulate liquid crystal creams.

Table 10

| Ingredient | | (1) | (2) | (3) |
|---|---|---|---|---|
| A | NIKKOL Nikkomulese LC-EF | 5.00% | 5.00% | 5.00% |
| | Stearyl alcohol | 2.50% | 2.50% | 2.50% |
| | NIKKOL Ceralipid 2 | 0.20% | 0.20% | 0.20% |
| | Glyceryl tri(caprylate/caprate) | 5.00% | 5.00% | 5.00% |
| | Squalane | 4.00% | 4.00% | 4.00% |
| | Triethylhexanoin | 3.00% | 3.00% | 3.00% |
| | Dimethicone | 2.00% | 2.00% | 2.00% |
| | Cyclopentasiloxane | 4.00% | 4.00% | 4.00% |
| B | Purified water | 48.79% | 47.79% | 45.79% |
| | BG | 5.00% | 5.00% | 5.00% |
| | Glycerin | 3.00% | 3.00% | 3.00% |
| | Methylparaben | 0.20% | 0.20% | 0.20% |
| | EDTA-2Na | 0.05% | 0.05% | 0.05% |
| | PEMULEN TR-1 | 0.06% | 0.06% | 0.06% |
| | 2% Aqueous carbomer | 10.00% | 10.00% | 10.00% |
| C | Arginine | 0.20% | 0.20% | 0.20% |
| | Purified water | 3.00% | 3.00% | 3.00% |
| D | 1% Aqueous biohyaluronic acid Na | 2.00% | 2.00% | 2.00% |
| | Purified water | 2.00% | 2.00% | 2.00% |
| E | ES-01 premix | 0.00% | 1.00% | 3.00% |
| Total | | 100.00% | 100.00% | 100.0% |

[Formulation Example 3] UV Milk (O/W Emulsion)

According to compositions shown in Table 11, A, B, C, and D were prepared and mixed to thereby formulate UV milks (O/W emulsions).

Table 11

| Ingredient | | (1) | (2) | (3) |
|---|---|---|---|---|
| A | NIKKOL Nikkomulese 41 | 2.00% | 2.00% | 2.00% |
| | SSQP40TJ | 20.00% | 20.00% | 20.00% |
| | NIKKOL NATURAL OILS | 1.00% | 1.00% | 1.00% |
| B | BG | 5.00% | 5.00% | 5.00% |
| | Purified water | 44.10% | 43.10% | 41.10% |
| | Stearoyl methyl taurine Na | 0.50% | 0.50% | 0.50% |
| | 2% Aqueous xanthan gum | 10.00% | 10.00% | 10.00% |
| | 1% Aqueous hydroxyethyl cellulose | 10.00% | 10.00% | 10.00% |
| | Phenoxyethanol | 0.40% | 0.40% | 0.40% |

(continued)

| Ingredient | | (1) | (2) | (3) |
|---|---|---|---|---|
| C | FUCOGEL 1.5P | 1.00% | 1.00% | 1.00% |
| | 1% Aqueous biohyaluronic acid Na | 1.00% | 1.00% | 1.00% |
| | Purified water | 5.00% | 5.00% | 5.00% |
| D | ES-01 premix | 0.00% | 1.00% | 3.00% |
| Total | | 100.00% | 100.0% | 100.0% |

[Formulation Example 4] O/W Foundation

According to compositions shown in Table 12, A, B, C, D, and E were prepared and mixed to thereby formulate O/W foundations.

Table 12

| Ingredient | | (1) | (2) | (3) | (4) |
|---|---|---|---|---|---|
| A | NIKKOL Nikkomulese 41 | 2.00% | 2.00% | 2.00% | 2.00% |
| | NIKKOL NATURAL OILS | 7.00% | 7.00% | 7.00% | 7.00% |
| | NIKKOL Nikkoguard 88 | 0.50% | 0.50% | 0.50% | 0.50% |
| B | Nylon-12 | 1.00% | 1.00% | 1.00% | 1.00% |
| | Silica (Sunsphere L-51) | 1.00% | 1.00% | 1.00% | 1.00% |
| C | Fresh Color Base AQUA (pigment) | 15.00% | 15.00% | 15.00% | 15.00% |
| | Purified water | 41.20% | 40.20% | 38.20% | 31.20% |
| | Stearoyl methyl taurine Na | 0.10% | 0.10% | 0.10% | 0.10% |
| | Lecithin | 0.10% | 0.10% | 0.10% | 0.10% |
| | EDTA-2Na | 0.10% | 0.10% | 0.10% | 0.10% |
| | 1% Aqueous hydroxyethyl cellulose | 20.00% | 20.00% | 20.00% | 20.00% |
| | 2% Aqueous xanthan gum | 10.00% | 10.00% | 10.00% | 10.00% |
| D | NIKKOL NET-813-1 | 2.00% | 2.00% | 2.00% | 2.00% |
| E | ES-01 premix | 0.00% | 1.00% | 3.00% | 10.00% |
| Total | | 100.0% | 100.0% | 100.0% | 100.0% |

[Formulation Example 5] Non-Silicon Treatment (Leave-on Type)

According to compositions shown in Table 13, A, B, C, and D were prepared and mixed to thereby formulate non-silicon treatments (leave-on type).

Table 13

| Ingredient | | (1) | (2) | (3) |
|---|---|---|---|---|
| A | Pentylene glycol | 1.50% | 1.50% | 1.50% |
| | BG | 3.50% | 3.50% | 3.50% |
| | Glycerin | 1.00% | 1.00% | 1.00% |
| | Methylparaben | 0.20% | 0.20% | 0.20% |
| | Carbomer | 0.20% | 0.20% | 0.20% |
| | EDTA-2Na | 0.10% | 0.10% | 0.10% |
| | Purified water | 79.80% | 78.80% | 76.80% |
| B | NIKKOL Nikkomulese LC-EF | 4.00% | 4.00% | 4.00% |
| | Methylheptyl laurate | 3.50% | 3.50% | 3.50% |
| | Squalane | 0.50% | 0.50% | 0.50% |
| | Stearyl alcohol | 1.50% | 1.50% | 1.50% |
| | Macadamia nut oil | 0.50% | 0.50% | 0.50% |
| | Avocado oil | 0.50% | 0.50% | 0.50% |
| | Shea butter | 0.50% | 0.50% | 0.50% |
| | Propylparaben | 0.10% | 0.10% | 0.10% |
| C | Purified water | 2.45% | 2.45% | 2.45% |
| | Arginine | 0.15% | 0.15% | 0.15% |
| D | ES-01 premix | 0.00% | 1.00% | 3.00% |
| Total | | 100.00% | 100.0% | 100.0% |

[Formulation Example 6] Shampoo

According to compositions shown in Table 14, A, B, C, and D were prepared and mixed to thereby formulate shampoos.

Table 14

| Ingredient | | (1) | (2) | (3) |
|---|---|---|---|---|
| A | Polyquaternium-10 | 0.50% | 0.50% | 0.50% |
| | Citric acid | 0.65% | 0.65% | 0.65% |
| | EDTA-2Na | 0.10% | 0.10% | 0.10% |
| | Methylparaben | 0.50% | 0.50% | 0.50% |
| | Phenoxyethanol | 0.50% | 0.50% | 0.50% |
| | BG | 4.00% | 4.00% | 4.00% |
| | Purified water | 28.25% | 27.25% | 25.25% |
| B | Aqueous cocoyl methyl taurine Na | 20.00% | 20.00% | 20.00% |
| | Aqueous lauroyl methyl alanine Na | 15.00% | 15.00% | 15.00% |
| | Aqueous cocamidopropyl betaine | 15.00% | 15.00% | 15.00% |
| | Cocamide DEA | 2.00% | 2.00% | 2.00% |
| | Aqueous (caprylyl/capryl) glycoside | 10.00% | 10.00% | 10.00% |

(continued)

| Ingredient | | (1) | (2) | (3) |
|---|---|---|---|---|
| C | Behenamidopropyldimethylamine | 0.50% | 0.50% | 0.50% |
| | NIKKOL NIKKOMIX SR | 3.00% | 3.00% | 3.00% |
| D | ES-01 premix | 0.00% | 1.00% | 3.00% |
| Total | | 100.0% | 100.0% | 100.0% |

[Example 11 and Comparative Example 7] Evaluation of Finger Passage through Hair after Treatment with Conditioner

In Example 11, a composition having the same formulation as the composition of Example 10 was prepared according to Table 8 described above. In Comparative Example 7, a composition having the same formulation as the composition of Comparative Example 6 was prepared.

Firstly, 10 g of the composition (conditioner) of Example 11 or Comparative Example 7 was uniformly applied to the entirety of a human hair bundle (black hair, 1 g, 10 cm) (available from Ketabaya). After the elapse of five minutes, the applied conditioner was washed off. The hair bundle was allowed to stand still at room temperature overnight, and then the frictional resistance of the hair bundle was measured with Tactile Meter Type 33 (available from Shinto Scientific Co., Ltd.). The frictional resistance was used as an index of finger passage.

FIG. 8 shows frictional resistances in Example 11 and Comparative Example 7. In Referential Example 1, the frictional resistance of a human hair bundle not treated with the conditioner was measured under the same conditions as described above.

According to the results shown in FIG. 8, the hair treated with the conditioner exhibited low frictional resistance (Example 11 and Comparative Example 7) as compared with the hair not treated with the conditioner. In particular, the hair treated with the conditioner containing a lipidic peptide compound exhibited considerably low frictional resistance; i.e., the conditioner achieved smooth finger passage through hair (Example 11).

Thus, the coating film-forming composition of the present invention successfully formed a coating film exhibiting low frictional resistance; i.e., a coating film achieving smooth finger passage or smooth combing.

[Example 12; Preparation of O/W Liquid Foundation]

An O/W liquid foundation containing a homogeneously dispersed pigment (17% by mass) was prepared as shown in Table 15 below. In Table 15, the amount of each ingredient corresponds to "% by mass (wt%)" relative to the total mass of the O/W liquid foundation.

In a 200 mL beaker (available from HARIO Co., Ltd.), 34.0 g of Fresh Color Base AQUA was mixed with 140.6 g of purified water, 0.2 g of stearoyl methyl taurine Na (NIKKOL SMT), 0.2 g of LPA (lecithin), 0.2 g of disodium edetate (EDTA-2Na), 0.4 g of hydroxypropyl cellulose, and 0.4 g of xanthan gum (KELTROL CG-SFT). The resultant mixture was stirred under heating at 75°C for 10 minutes (phase B). In a 200 mL beaker (available from HARIO Co., Ltd.), 4.0 g of NIKKOL Nikkomulese 41 was added to 14.0 g of NIKKOL NATURAL OILS and 1.0 g of NIKKOL Nikkoguard 88. The resultant mixture was stirred under heating at 75°C for 10 minutes (phase A). Phase A was slowly added to phase B with stirring, and the resultant mixture was subjected to emulsification treatment at 75°C with a homomixer (QUICK HOMO MIXER LR-1A available from MIZUHO INDUSTRIAL CO., LTD.) at 5,000 rpm for three minutes. Subsequently, 1.0 g of ES-01 premix heated to 75°C (phase D) was added to the emulsified mixture, and the mixture was stirred under heating at 75°C for two minutes. Thereafter, the mixture was cooled at room temperature with stirring until the temperature reached 40°C. After confirmation that the temperature reached 40°C, 4.0 g of NIKKOL NET-813-1 (phase C) was added to the mixture, and the resultant mixture was cooled with stirring until the temperature reached 35°C. In all the aforementioned processes, the stirring was performed at 200 rpm.

[Comparative Example 8; Preparation of O/W Liquid Foundation]

An O/W liquid foundation homogeneously containing a pigment (17% by mass) was prepared as shown in Table 15 below.

In a 200 mL beaker (available from HARIO Co., Ltd.), 34.0 g of Fresh Color Base AQUA was mixed with 141.6 g of purified water, 0.2 g of stearoyl methyl taurine Na (NIKKOL SMT), 0.2 g of LPA (lecithin), 0.2 g of disodium edetate (EDTA-2Na), 0.4 g of hydroxypropyl cellulose, and 0.4 g of xanthan gum (KELTROL CG-SFT). The resultant mixture was stirred under heating at 75°C for 10 minutes (phase B). In a 200 mL beaker (available from HARIO Co., Ltd.), 4.0 g of NIKKOL Nikkomulese 41 was added to 14.0 g of NIKKOL NATURAL OILS and 1.0 g of NIKKOL Nikkoguard 88.

The resultant mixture was stirred under heating at 75°C for 10 minutes (phase A). Phase A was slowly added to phase B with stirring, and the resultant mixture was subjected to emulsification treatment at 75°C with a homomixer (QUICK HOMO MIXER LR-1A available from MIZUHO INDUSTRIAL CO., LTD.) at 5,000 rpm for three minutes. Thereafter, the mixture was cooled at room temperature with stirring until the temperature reached 40°C. Subsequently, 4.0 g of NIKKOL NET-813-1 (phase C) was added to the mixture, and the resultant mixture was cooled with stirring until the temperature reached 35°C. In all the aforementioned processes, the stirring was performed at 200 rpm.

Table 15

| Ingredient (wt%) | | Example 12 | Comparative Example 8 |
|---|---|---|---|
| Phase A | NIKKOL Nikkomulese 41*1 | 2.0 | 2.0 |
| | NIKKOL NATURAL OILS*1 | 7.0 | 7.0 |
| | NIKKOL Nikkoguard 88*1 | 0.5 | 0.5 |
| Phase B | NIKKOL Fresh Color Base AQUA (pigment)*1 | 17.0 | 17.0 |
| | Purified water | 70.3 | 70.8 |
| | Stearoyl methyl taurine Na*1 | 0.1 | 0.1 |
| | LPA (lecithin)*1 | 0.1 | 0.1 |
| | EDTA-2Na*2 | 0.1 | 0.1 |
| | Hydroxypropyl cellulose*3 | 0.2 | 0.2 |
| | Xanthan gum*4 | 0.2 | 0.2 |
| Phase C | NIKKOL NET-813-1*1 | 2.0 | 2.0 |
| Phase D | ES-01 premix | 0.5 | 0 |
| Total | | 100 | 100 |

*1: available from Nikko Chemicals Co., Ltd.
*2: available from JUNSEI CHEMICAL CO., LTD.
*3: available from Nippon Soda Co., Ltd. [trade name: NISSO HPC SSL]
*4: available from Sansho Co., Ltd.

[Example 13 and Comparative Example 9] Measurement of Reflected Light Intensity

For measurement of reflected light intensity, the O/W liquid foundation of Example 12 was used in Example 13, and the O/W liquid foundation of Comparative Example 8 was used in Comparative Example 9.

The O/W liquid foundation of Example 13 or the O/W liquid foundation of Comparative Example 9 was applied onto BIOSKIN (available from Beaulax Co., Ltd.) so as to achieve 10 $mg/cm^2$, followed by drying in a thermostatic chamber at 35°C. The reflected light intensity of the resultant coating film at 45° (incident light: -45°) was measured with Goniophotometer GP-5 (available from MURAKAMI COLOR RESEARCH LABORATORY). The results of measurement are shown in FIG. 9.

According to the results shown in FIG. 9, strong reflected light was detected at the BIOSKIN to which the O/W liquid foundation of Comparative Example 9 was applied, whereas reflected light intensity was reduced by about 40% at the BIOSKIN to which the O/W liquid foundation containing a lipidic peptide compound was applied (Example 13).

Thus, the coating film-forming composition of the present invention successfully formed a coating film capable of reducing light reflection.

[Example 14 and Comparative Example 10] Evaluation of Makeup Deterioration

The O/W liquid foundation of Example 12 was used as a composition of Example 14, and the O/W liquid foundation of Comparative Example 8 was used as a composition of Comparative Example 10.

Firstly, 0.5 g of the O/W liquid foundation of Example 14 or the O/W liquid foundation of Comparative Example 10 was applied onto human skin, and the thus-applied liquid foundation was uniformly spread. Thereafter, 0.5 g of artificial sebum was added dropwise onto the liquid foundation, and then allowed to stand still at room temperature for 30 minutes. FIG. 10 shows appearance photographs in Example 14 and Comparative Example 10 after being allowed

to stand still at room temperature for 30 minutes.

As shown in the results of FIG. 10, dropwise addition of artificial sebum onto the O/W liquid foundation of Comparative Example 10 caused association between oil in the O/W liquid foundation and the artificial sebum, leading to observation of makeup deterioration (Comparative Example 10). In contrast, dropwise addition of artificial sebum onto the O/W liquid foundation containing a lipidic peptide compound resulted in no observation of makeup deterioration (Example 14).

Thus, the coating film-forming composition of the present invention successfully formed a coating film capable of reducing association of an oil ingredient and sebum to thereby prevent makeup deterioration.

[Example 15 and Comparative Example 11] Preparation of Conditioner Containing Malic Acid

A foundation containing malic acid in an amount of 1.5% by mass (wt%) was prepared as shown in Table 16 below.

Phase A was heated with stirring in a 200 mL beaker (available from HARIO Co., Ltd.) until the temperature reached 75°C. Phase B and phase C were mixed in a 200 mL beaker, and the mixture was heated with stirring until the temperature reached 75°C. Phase A was added to the mixture of phase B and phase C, and the resultant mixture was subjected to emulsification treatment at 75°C with a homomixer (QUICK HOMO MIXER LR-1A available from MIZUHO INDUSTRIAL CO., LTD.) at 5,000 rpm for five minutes. Subsequently, phase D heated to 75°C was added to the emulsified mixture, and the mixture was stirred under heating at 75°C for five minutes. Thereafter, the mixture was cooled with stirring until the temperature reached 40°C. In all the aforementioned processes, the stirring was performed at 200 rpm.

Table 16

| | Ingredient (wt%) | Example 15 | Comparative Example 11 |
|---|---|---|---|
| Phase A | NIKKOL CA-2580*1 | 6.0 | 6.0 |
| | Behenyl alcohol*2 | 5.0 | 5.0 |
| | NIKKOL Nikkomulese 41*3 | 5.0 | 5.0 |
| | Oleyl alcohol*4 | 1.0 | 1.0 |
| | NIKKOL LM Wax*5 | 1.0 | 1.0 |
| | Tocopherol*6 | 0.5 | 0.5 |
| | Olive oil*7 | 5.0 | 5.0 |
| | Dimethicone*8 | 5.0 | 5.0 |
| | White Vaseline*9 | 6.0 | 6.0 |
| Phase B | Propylene glycol*10 | 4.0 | 4.0 |
| | Dipropylene glycol*11 | 5.0 | 5.0 |
| | Phenoxyethanol*12 | 0.1 | 0.1 |
| | Malic acid*13 | 1.5 | 1.5 |
| Phase C | Hydroxyethyl cellulose*14 | 0.2 | 0.2 |
| | Polyethylene glycol 400*15 | 0.08 | 0.08 |
| | Potassium hydroxide*16 | 0.05 | 0.05 |
| | Purified water | 54.07 | 54.57 |
| Phase D | ES-01 premix | 0.5 | 0 |

(continued)

| | Ingredient (wt%) | Example 15 | Comparative Example 11 |
|---|---|---|---|
| Total | | 100 | 100 |

*1: available from Nikko Chemicals Co., Ltd.
*2: available from KOKYU ALCOHOL KOGYO CO., LTD.
*3: available from Nikko Chemicals Co., Ltd.
*4: available from KOKYU ALCOHOL KOGYO CO., LTD.
*5: available from Nikko Chemicals Co., Ltd.
*6: RIKEN VITAMIN E OIL 700 available from RIKEN VITAMIN CO., LTD.
*7: available from Nikko Chemicals Co., Ltd.
*8: KF-96A-500CS available from Shin-Etsu Chemical Co., Ltd.
*9: available from JUNSEI CHEMICAL CO., LTD.
*10: available from FUJIFILM Wako Pure Chemical Corporation
*11: available from KOYO FINE CHEMICAL CORP.
*12: available from Tokyo Chemical Industry Co., Ltd.
*13: available from JUNSEI CHEMICAL CO., LTD.
*14: available from NACALAI TESQUE, INC.
*15: available from JUNSEI CHEMICAL CO., LTD.
*16: available from Wako Pure Chemical Industries, Ltd.

[Example 16 and Comparative Example 12] Moisturizing Test of Human Hair

(1) Preparation Procedure of Damaged Hair Sample (Damaged Hair)

One set of Gatsby EX High Bleach (available from Mandom Corporation) (mixture of 18 g of powder, 70 mL of water, and 35 g of cream) was applied to 50 bundles (about 10 cm and about 1 g for each bundle) of human black hair (BS-B-A, available from Beaulax Co., Ltd.), and then allowed to stand at room temperature for one hour, to thereby bleach the hair. Subsequently, the hair was washed with distilled water, and then dried with a constant-temperature dryer (OF-300B, available from AS ONE CORPORATION) set at 65°C for 30 minutes. The aforementioned operation was repeated three times in total. Thereafter, the hair was immersed in 1 L of 1% by mass aqueous solution of sodium dodecyl sulfate (available from FUJIFILM Wako Pure Chemical Corporation) in distilled water, and then washed with distilled water. Excess water was removed with Kaydry (available from NIPPON PAPER CRECIA Co., LTD.), and then the hair was dried at room temperature overnight, to thereby prepare a damaged hair sample (bleached hair).

(2) Treatment of Hair with Conditioner

The above-prepared damaged hair sample was wetted with distilled water, and then excess water was removed with Kaydry (available from NIPPON PAPER CRECIA Co., LTD.). Subsequently, 1 g of the sample prepared in Example 15 or Comparative Example 11 was applied to the hair with hands, and then allowed to stand still for five minutes. Thereafter, the hair was washed with shaking in a 300 mL tall beaker charged with 300 mL of water. Excess water was then removed with Kaydry (available from NIPPON PAPER CRECIA Co., LTD.), and the hair was dried at room temperature overnight.

(3) Moisturizing Test of Hair

The aforementioned hair sample was cut into a length of 1 cm, and then 0.7 g of the sample was weighed in a weighing dish. The hair sample was dried with a constant-temperature dryer (OF-300B, available from AS ONE CORPORATION) set at 65°C for 40 minutes, and then the mass of the sample was measured again. A reduction in the mass of the hair was regarded as a reduction in the amount of water, and the rate of moisture loss from the hair was calculated by use of the following formula (Table 17).

$$\text{Rate of moisture loss (\%)} = (1 - (\text{the mass of hair after drying})/(\text{the mass of hair before drying})) \times 100$$

Table 17

| | | Rate of moisture loss (%) |
|---|---|---|
| Referential Example 2 | Untreated black hair | 5.95 |
| Referential Example 3 | Bleached hair | 6.17 |
| Comparative Example 12 | Black hair treated with the sample of Comparative Example 11 | 5.90 |
| Example 16 | Black hair treated with the sample of Example 15 | 5.69 |

According to the results shown in Table 17, the black hair treated with the conditioner exhibited a low rate of moisture loss (Example 16 and Comparative Example 12) as compared with the black hair not treated with the conditioner (Referential Examples 2 and 3). In particular, the black hair treated with the conditioner containing a lipidic peptide compound exhibited a lower rate of moisture loss; i.e., the effect of moisturizing human hair was improved.

[Example 17 and Comparative Example 13] Test for Evaluation of Recovery of Human Hair Gloss

Black drawing paper PI-N86D (available from Maruai Corporation) was attached onto Micro Slide Glass S1111 (available from Matsunami Glass Ind., Ltd.) with NICETACK (registered trademark) NW-10 (available from NICHIBAN CO., LTD.). The hair samples used in the aforementioned moisturizing test were arranged in parallel on the drawing paper with no space between the samples, and then the samples were attached to the drawing paper with NICETACK (registered trademark) NW-10 (available from NICHIBAN CO., LTD.). Reflected light distribution was measured with Goniophotometer GP-5 (available from MURAKAMI COLOR RESEARCH LABORATORY). Reflected light intensity was measured at 45° (incident light for measurement: -45°).

Table 18

| | | Reflected light intensity at 40° |
|---|---|---|
| Referential Example 3 | Untreated black hair | 84.80 |
| Referential Example 4 | Bleached hair | 40.36 |
| Comparative Example 13 | Black hair after the moisturizing test in Comparative Example 12 | 46.38 |
| Example 17 | Black hair after the moisturizing text in Example 16 | 53.48 |

According to the results shown in Table 18, the black hair treated with the conditioner (Example 17 and Comparative Example 13) exhibited reflected light intensity higher than that of the black hair not treated with the conditioner; i.e., the black hair treated with the conditioner was provided with a gloss (recovery of gloss). In particular, the black hair treated with the conditioner containing a lipidic peptide compound exhibited very high reflected light intensity, and the gloss of the hair was further improved (further recovery of gloss).

[Example 18 and Comparative Example 14] Quantification of Malic Acid in Human Hair after Treatment with Conditioner

For quantification of malic acid in human hair, the human hair treated in (2) of Example 16 was used in Example 18, and the human hair treated in (2) of Comparative Example 12 was used in Comparative Example 14.

The quantification of malic acid was performed with EnzyChrom™ Malate Assay Kit (EMAL-100) available from BioAssay Systems.

Specifically, 200 mg of the human hair of Example 18 or Comparative Example 14 was weighed in a 50 mL sample vial, and 4 mL of purified water was added to the vial, followed by ultrasonic treatment for 60 minutes for extraction of malic acid. Subsequently, 20 μL of each extract, 74 μL of an assay buffer, 1 μL of Enzyme A, 1 μL of Enzyme B, and 8 μL of NAD/MTT reagent were added to a 96 well plate. After the elapse of 15 minutes, absorbance (565 nm) in the well prepared through the aforementioned procedure was measured with a plate reader (available from TECAN), and net absorbance was calculated by subtracting the absorbance of the sample itself from the measured absorbance. The test results are shown in FIG. 11.

As shown in the results of FIG. 11, the amount of malic acid extracted from the human hair of Example 18 was

about three times that of malic acid extracted from the human hair of Comparative Example 14.

Thus, the coating film-forming composition of the present invention successfully formed a coating film capable of sufficiently retaining an active ingredient (i.e., a coating film capable of containing a large amount of an active ingredient).

[Example 19 and Comparative Example 15; SEM Measurement of Hair Treated with Conditioner]

In each of Example 19 and Comparative Example 15, 1 g of each of the conditioners prepared in Example 10 and Comparative Example 6 was applied to damaged hair bundles (10 cm, 50 bundles) prepared by the same procedure as in (1) of Example 16, and then the hair was allowed to stand still for five minutes. Thereafter, the hair was washed with shaking in a 300 mL tall beaker charged with 300 mL of purified water. Excess purified water was then removed with Kaydry (available from NIPPON PAPER CRECIA Co., LTD.), and the hair was dried at room temperature overnight. The surface of the above-treated hair was observed with a scanning electron microscope (SEM) [Miniscope (registered trademark) TM3000 (available from Hitachi High-Technologies Corporation)]. The results are shown in FIG. 12. The results of observation indicated that the surface of the hair of Example 19 was smoother as compared with the surface of the bleached (damaged) hair or the surface of the hair of Comparative Example 15.

[Example 20 and Comparative Example 16; Evaluation of Adsorption or Desorption of Moisture on Hair Treated with Conditioner]

In Example 20, one damaged hair bundle (about 10 cm, about 1 g) prepared by the same procedure as in (1) of Example 16 was subjected to the same treatment as in (2) of Example 16, and the treated hair bundle was sheared to a length of 1 cm. Thereafter, 16 mg of the hair was weighed, and the amount of adsorbed/desorbed moisture was evaluated with a moisture adsorption/desorption measuring device IGAsorp (available from Hiden Isochema). The wet weight of each sample was measured at a temperature of 25°C and a humidity of 40%, and then the sample was dried at a temperature of 25°C and a humidity of 0%. The drying was continued until the rate of change in sample weight was lower than 0.0001 mg/min. The relative humidity was increased stepwise from 0% to 90% in increments of 10%, and the moisture content of the hair was calculated from the balancing weight of the sample at each relative humidity. Also, the moisture content of the hair was calculated from the balancing weight of the sample at each relative humidity when the relative humidity was decreased stepwise from 90% to 0% in increments of 10%. The maximum balancing time was set to 360 minutes at each humidity. The aforementioned procedure was automatically performed by a program, and the balancing weight of the sample was determined by the asymptotic method based on an LDF (linear driving force approximation) model for the purpose of calculation of the weight value in a 99% relaxation state. In the case where the LDF model was not applied, the balancing weight of the sample was determined by calculation through moving averaging of values after 180-minute measurement. The results of determined moisture contents are shown in Table 19 (during humidity increase) and Table 20 (during humidity decrease). Table 19 also shows the results of Comparative Example 16 (i.e., the results of the human hair treated in (2) of Comparative Example 12) and the results of damaged hair. As compared with the damaged hair not treated with the conditioner, the treated hair of Comparative Example 16 exhibited a tendency to reduce adsorption of moisture, and the treated hair of Example 19 exhibited a tendency to further reduce adsorption of moisture.

Table 19

| | Damaged hair | | Comparative Example 16 | | Example 20 | |
|---|---|---|---|---|---|---|
| %RH | Moisture content (%Wt) | Balancing weight (mg) | Moisture content (%Wt) | Balancing weight (mg) | Moisture content (%Wt) | Balancing weight (mg) |
| 0 | 0 | 14.8578 | 0 | 14.0294 | 0 | 15.222 |
| 10 | 3.151206774 | 15.326 | 2.868975152 | 14.4319 | 2.776244909 | 15.6446 |
| 20 | 5.22823029 | 15.6346 | 4.835559611 | 14.7078 | 4.720798844 | 15.9406 |
| 30 | 7.014430131 | 15.89999 | 6.555519124 | 14.9491 | 6.377611352 | 16.1928 |
| 40 | 8.651348113 | 16.1432 | 8.04952457 | 15.1587 | 7.846537906 | 16.4164 |
| 50 | 10.22291322 | 16.3767 | 9.51573125 | 15.3644 | 9.235317304 | 16.6278 |
| 60 | 12.26695742 | 16.6804 | 11.41388798 | 15.6307 | 11.02286165 | 16.8999 |
| 70 | 14.96385737 | 17.0811 | 13.94713958 | 15.9861 | 13.51530679 | 17.2793 |
| 80 | 18.70465345 | 17.6369 | 17.2530543 | 16.4499 | 16.7225069 | 17.7675 |
| 90 | 25.40618396 | 18.6326 | 23.37448501 | 17.3087 | 22.68624359 | 18.6753 |

Table 20

| | Damaged hair | | Comparative Example 16 | | Example 20 | |
|---|---|---|---|---|---|---|
| %RH | Moisture content (%Wt) | Balancing weight (mg) | Moisture content (%Wt) | Balancing weight (mg) | Moisture content (%Wt) | Balancing weight (mg) |
| 90 | 25.40618396 | 18.6326 | 23.37448501 | 17.3087 | 22.68624359 | 18.6753 |
| 80 | 19.67047611 | 17.7804 | 18.18181818 | 16.5802 | 17.64682696 | 17.9082 |
| 70 | 16.2561079 | 17.2731 | 15.20378633 | 16.1624 | 14.77269741 | 17.4707 |
| 60 | 14.17302696 | 16.9636 | 13.34625857 | 15.9018 | 12.9437656 | 17.1923 |
| 50 | 12.40829733 | 16.7014 | 11.67191754 | 15.6669 | 11.24425174 | 16.9336 |
| 40 | 10.63347198 | 16.4377 | 9.897073289 | 15.4179 | 9.614373932 | 16.6855 |
| 30 | 8.608946143 | 16.1369 | 8.033130426 | 15.1564 | 7.772303245 | 16.4051 |
| 20 | 6.511731212 | 15.8253 | 6.055854135 | 14.879 | 5.806070162 | 16.1058 |
| 10 | 4.15943141 | 15.4758 | 3.742141503 | 14.5544 | 3.592169229 | 15.7688 |
| 0 | 0.045767206 | 14.8646 | -0.076981197 | 14.0186 | -0.185915123 | 15.1937 |

[Example 21 and Comparative Example 17; Dynamic Friction Coefficient of Hair Treated with Conditioner]

In Example 21, one damaged hair bundle (about 10 cm, about 1 g) prepared by the same procedure as in (1) of Example 16 was subjected to the same treatment as in (2) of Example 16, and the dynamic friction coefficient of the surface of the treated hair bundle was measured. The dynamic friction coefficient was used as an index of finger passage. The dynamic friction coefficient was measured with KES-SE friction tester (available from KATO TECH CO., LTD.) by using a silicon sensor having dimensions of 10 mm × 10 mm. In Comparative Example 17, the dynamic friction coefficient was measured for the hair bundle treated in the same manner as in (2) of Comparative Example 12. FIG. 13 shows the results of Example 21 and Comparative Example 17. The surface of the hair used in Example 21 exhibited a dynamic friction coefficient lower than that of the surface of the hair used in Comparative Example 17.

[Example 22 and Comparative Example 18; Preparation of Conditioner Containing Succinic Acid]

A conditioner containing succinic acid in an amount of 1.5% by mass was prepared as shown in Table 21 below. Phase A was heated with stirring in a 200 mL beaker (available from HARIO Co., Ltd.) until the temperature reached 75°C. Phase B and phase C were mixed in a 200 mL beaker, and the mixture was heated with stirring until the temperature reached 75°C. Phase A was added to phase B + phase C, and the resultant mixture was subjected to emulsification treatment at 75°C with a homomixer (QUICK HOMO MIXER LR-1A available from MIZUHO INDUSTRIAL CO., LTD.) at 5,000 rpm for five minutes. Subsequently, phase D heated to 75°C was added to the emulsified mixture, and the mixture was stirred under heating at 75°C for five minutes. Thereafter, the mixture was cooled with stirring until the temperature reached 40°C. In all the aforementioned processes, the stirring was performed at 200 rpm.

Table 21

| | | Example 22 | Comparative Example 18 |
|---|---|---|---|
| A | NIKKOL CA-2580*1 | 6 | 6 |
| | Behenyl alcohol*2 | 5 | 5 |
| | NIKKOL Nikkomulese 41*3 | 5 | 5 |
| | Oleyl alcohol*4 | 1 | 1 |
| | NIKKOL LM Wax*5 | 1 | 1 |
| | Tocopherol*6 | 0.5 | 0.5 |
| | Olive oil*7 | 5 | 5 |
| | Dimethicone*8 | 5 | 5 |
| | White Vaseline*9 | 6 | 6 |

(continued)

| | | Example 22 | Comparative Example 18 |
|---|---|---|---|
| B | Propylene glycol*10<br>Dipropylene glycol*11<br>Phenoxyethanol*12<br>Succinic acid*13 | 4<br>5<br>0.1<br>1.5 | 4<br>5<br>0.1<br>1.5 |
| C | Hydroxyethyl cellulose*14<br>Polyethylene glycol 400*15<br>Potassium hydroxide*16<br>Purified water | 0.2<br>0.08<br>0.05<br>54.07 | 0.2<br>0.08<br>0.05<br>54.57 |
| D | ES-01 premix | 0.5 | 0 |
| Total | | 100 | 100 |

*1: available from Nikko Chemicals Co., Ltd.
*2: available from KOKYU ALCOHOL KOGYO CO., LTD.
*3: available from Nikko Chemicals Co., Ltd.
*4: available from KOKYU ALCOHOL KOGYO CO., LTD.
*5: available from Nikko Chemicals Co., Ltd.
*6: RIKEN VITAMIN E OIL 700 available from RIKEN VITAMIN CO., LTD.
*7: available from Nikko Chemicals Co., Ltd.
*8: KF-96A-500CS available from Shin-Etsu Chemical Co., Ltd.
*9: available from JUNSEI CHEMICAL CO., LTD.
*10: available from FUJIFILM Wako Pure Chemical Corporation
*11: available from KOYO FINE CHEMICAL CORP.
*12: available from Tokyo Chemical Industry Co., Ltd.
*13: available from JUNSEI CHEMICAL CO., LTD.
*14: available from NACALAI TESQUE, INC.
*15: available from JUNSEI CHEMICAL CO., LTD.
*16: available from Wako Pure Chemical Industries, Ltd.

[Example 23 and Comparative Example 19; Evaluation of Amount of Succinic Acid in Hair Treated with Conditioner]

In each of Example 23 and Comparative Example 19, 1 g of each of the conditioners prepared in Example 22 and Comparative Example 18 was applied with hands to one damaged hair bundle (about 10 cm, about 1 g) prepared by the same procedure as in (1) of Example 16, and then the hair was allowed to stand still for five minutes. Thereafter, the hair was washed with shaking in a 300 mL tall beaker charged with 300 mL of water. Excess water was then removed with Kaydry (available from NIPPON PAPER CRECIA Co., LTD.), and the hair was dried at room temperature overnight. The aforementioned treatment was performed once, twice, and three times, to thereby prepare three types of samples. Each of the thus-prepared damaged hair samples was cut into a length of 1 cm. The quantification of succinic acid was performed with Succinate colorimetric Assay Kit (available from Megazyme). Specifically, 200 mg of the human hair treated in each of Example 23 and Comparative Example 19 was weighed in a 50 mL sample vial, and 4 mL of purified water was added to the sample vial, followed by ultrasonic treatment for 60 minutes for extraction of succinic acid. Subsequently, 10 μL of each extract, 210 μL of purified water, 20 μL of Buffer, 20 μL of NADH, 20 μL of ATP/PEP/CoA, and 2 μL of PK/L-LDH were added to a 96 well plate, and the mixture was allowed to stand still for three minutes, followed by addition of 2 μL of SCS. Absorbance (340 nm) in the well prepared through the aforementioned procedure was measured with a plate reader (available from TECAN), and net absorbance was calculated by subtracting the absorbance of the sample itself from the measured absorbance. The test results are shown in FIG. 14. The amount of succinic acid extracted from the hair increased in a manner dependent on the number of treatment days. The amount of succinic acid extracted from the human hair of Example 23 was about twice that of succinic acid extracted from the human hair of Comparative Example 19.

[Example 24 and Comparative Example 20; Evaluation of Amount of Succinic Acid and Lipidic Peptide Permeating into Human Hair]

In each of Example 24 and Comparative Example 20, 1 g of each of the conditioners prepared in Example 22 and Comparative Example 18 was applied with hands to one damaged hair bundle (about 10 cm, about 1 g) prepared by the same procedure as in (1) of Example 16, and then the hair was allowed to stand still for five minutes. Thereafter, the hair was washed with shaking in a 300 mL tall beaker charged with 300 mL of water. Excess water was then removed with Kaydry (available from NIPPON PAPER CRECIA Co., LTD.), and the hair was dried at room temperature overnight. The aforementioned treatment was repeated for three days. Subsequently, succinic acid and the lipidic peptide contained in ES-01 premix were analyzed with a time-of-flight secondary ion mass spectrometer TOF.SIMS5 (available from Hitachi High-Tech Corporation). A portion around the center of a hair of each hair sample was collected, and a cross section of the portion was prepared. Bi was selected as a primary ion source. FIG. 15 shows the results (i.e., hair cross sections). High succinic acid intensity was determined in the cross section of the hair of Example 24 as compared with the case of Comparative Example 20. In Example 24, the lipidic peptide contained in ES-01 premix was found to localize at the hair surface.

[Example 25 and Comparative Example 21; Measurement of Zeta Potential on Surface of Hair Treated with Conditioner]

In each of Example 25 and Comparative Example 21, 1 g of each of the conditioners prepared in Example 22 and Comparative Example 18 was applied with hands to one damaged hair bundle (about 10 cm, about 1 g) prepared by the same procedure as in (1) of Example 16, and then the hair was allowed to stand still for five minutes. Thereafter, the hair was washed with shaking in a 300 mL tall beaker charged with 300 mL of water. Excess water was then removed with Kaydry (available from NIPPON PAPER CRECIA Co., LTD.), and the hair was dried at room temperature overnight. The surface zeta potential of the aforementioned hair sample was evaluated with a solid surface zeta potential meter SurPASS (available from Anton-Paar). 1 mM KCl was prepared as an electrolyte, and the zeta potential was measured at a temperature of 23°C. The results are shown in Table 22. In Comparative Example 21, the surface potential was found to be cationic as compared with the case of the damaged (bleached) hair. In Example 25, the surface potential was found to be more cationic.

Table 22

| | (mV) |
|---|---|
| Damaged hair | -5.8 ± 0.458 |
| Comparative Example 21 | -1.547 ± 0.35 |
| Example 25 | 2.013 ± 0.25 |

[Example 26 and Comparative Example 22; Evaluation of Hardness of Surface of Hair Treated with Conditioner]

In each of Example 26 and Comparative Example 22, 1 g of each of the conditioners prepared in Example 22 and Comparative Example 18 was applied with hands to one damaged hair bundle (about 10 cm, about 1 g) prepared by the same procedure as in (1) of Example 16, and then the hair was allowed to stand still for five minutes. Thereafter, the hair was washed with shaking in a 300 mL tall beaker charged with 300 mL of water. Excess water was then removed with Kaydry (available from NIPPON PAPER CRECIA Co., LTD.), and the hair was dried at room temperature overnight. The hardness of the surface of each hair sample described above was measured with Hysitron TI980 Triboindenter (available from Bruker). The pushing depth was set to 1 μm, and a Berkovich indenter was used. The measurement was performed 10 times, and the average value was calculated. The results are shown in FIG. 16. The damaged (bleached) hair was found to have a harder surface than the untreated black hair. Also, the hair of Comparative Example 22 was found to have a softer surface as compared with the damaged hair, and the hair of Example 26 was found to have an even softer surface.

[Example 27 and Comparative Example 23; Evaluation of Adsorption or Desorption of Moisture on Hair Treated with Conditioner]

In each of Example 27 and Comparative Example 23, 1 g of each of the conditioners prepared in Example 22 and Comparative Example 18 was applied with hands to one damaged hair bundle (about 10 cm, about 1 g) prepared by the same procedure as in (1) of Example 16, and then the hair was allowed to stand still for five minutes. Thereafter,

the hair was washed with shaking in a 300 mL tall beaker charged with 300 mL of water. Excess water was then removed with Kaydry (available from NIPPON PAPER CRECIA Co., LTD.), and the hair was dried at room temperature overnight. The thus-treated sample was sheared to a length of 1 cm, and then 3 mg of the sample was weighed. The amount of moisture adsorbed/desorbed on the human hair was evaluated with a moisture adsorption/desorption measuring device IGAsorp (available from Hiden Isochema). The hair was dried at a relative humidity of 0% for two days. Thereafter, the relative humidity was rapidly increased to 90%, and the rate of change in mass was measured. The aforementioned procedure was automatically performed by a program. The results are shown in FIG. 17. As compared with the damaged hair not treated with the conditioner, the treated hair of Comparative Example 23 exhibited a tendency to reduce adsorption of moisture, and the treated hair of Example 27 exhibited a tendency to further reduce adsorption of moisture.

[Example 28 and Comparative Example 24; Time-Course Change in Shine by Sebum Floating]

For evaluation of time-course change in shine by sebum floating, the O/W liquid foundation of Example 12 was used in Example 28, and the O/W liquid foundation of Comparative Example 8 was used in Comparative Example 24. Each of the O/W liquid foundations of Example 12 and Comparative Example 8 was applied to the face of a subject, and the subject was allowed to live as usual. From one hour to six hours after application of each foundation, the surface gloss of the face was measured every hour with Glossymeter GL200 (available from Courage+khazaka electronic). The results are shown in FIG. 18. As compared with the foundation of Comparative Example 24, the foundation of Example 28 reduced time-course shine caused by sebum floating even after the elapse of six hours.

[Example 29 and Comparative Example 25; Preparation of Beauty Essence]

A beauty essence containing hyaluronic acid in an amount of 0.005% was prepared as shown in Table 23 below. Phase A was heated with stirring in a 200 mL beaker (available from HARIO Co., Ltd.) until the temperature reached 75°C. Phase B, phase C, and phase D were mixed in a 200 mL beaker, and the mixture was heated with stirring until the temperature reached 75°C. Phase A was added to phase B + phase C + phase D, and the resultant mixture was stirred at 75°C for five minutes. Subsequently, the mixture was cooled with stirring until the temperature reached 45°C. Thereafter, phase E was added to the mixture, and the mixture was cooled with stirring until the temperature reached 35°C. In all the aforementioned processes, the stirring was performed at 200 rpm.

Table 23

| Phase | | Comparative Example 25 | Example 29 |
|---|---|---|---|
| A | Purified water | 20 (g) | 20 (g) |
| | ES-01 premix | 0 | 0.5 |
| B | Purified water | 70.905 | 70.405 |
| | Concentrated glycerin*1 | 1 | 1 |
| C | 1,3-Butanediol*2 | 4 | 4 |
| | Methylparaben*3 | 0.05 | 0.05 |
| | Polyoxyethylene monolaurate*4 | 0.05 | 0.05 |
| D | 1,3-Butanediol*2 | 3.97 | 3.97 |
| | Xanthan gum*5 | 0.02 | 0.02 |
| E | Hyaluronic acid FCH150*6 | 0.005 | 0.005 |
| Total | | 100 | 100 |

*1: available from Sakamoto Yakuhin Kogyo Co., Ltd.
*2: available from Daicel Corporation
*3: available form Maruzen Chemicals Co., Ltd.
*4: available from Kao Corporation
*5:KELTROL CG-SFT available from Sansho Co., Ltd.
*6: available from Kikkoman Biochemifa Company

[Example 30 and Comparative Example 26; Moisturizing Effect of Beauty Essence]

For evaluation of moisturizing effect, the beauty essence of Example 29 was used in Example 30, and the beauty essence of Comparative Example 25 was used in Comparative Example 26. Each of the beauty essences of Example 29 and Comparative Example 25 was applied to the inside of the forearm of a subject. From one hour to seven hours after application of the beauty essence, the moisture content of the corneum of the forearm was measured every one hour at a humidity of 50% and a temperature of 24°C with Corneometer CM825 (available from Courage+khazaka electronic). The moisture content of the corneum was measured at 10 points randomly selected from the beauty-essence-applied forearm, and the average of measured values was calculated. The results are shown in FIG. 19. As compared with the beauty essence of Comparative Example 26, the beauty essence of Example 30 was found to retain a higher moisture content of the corneum (i.e., a moisturizing effect) even after the elapse of seven hours.

[Example 31, Example 32, and Comparative Example 27; Preparation of Conditioner Containing Keratin]

A conditioner containing keratin in an amount of 1.5% by mass was prepared as shown in Table 24 below. Phase A was heated with stirring in a 200 mL beaker (available from HARIO Co., Ltd.) until the temperature reached 75°C. Phase B and phase C were mixed in a 200 mL beaker, and the mixture was heated with stirring until the temperature reached 75°C. Phase A was added to phase B + phase C, and the resultant mixture was subjected to emulsification treatment at 75°C with a homomixer (QUICK HOMO MIXER LR-1A available from MIZUHO INDUSTRIAL CO., LTD.) at 5,000 rpm for five minutes. Subsequently, phase D heated to 75°C was added to the emulsified mixture, and the mixture was stirred under heating at 75°C for five minutes. Thereafter, the mixture was cooled with stirring until the temperature reached 40°C. In all the aforementioned processes, the stirring was performed at 200 rpm.

Table 24

| | | Example 31 | Example 32 | Comparative Example 27 |
|---|---|---|---|---|
| A | NIKKOL CA-2580*1 | 6 (g) | 6 (g) | 6 (g) |
| | Behenyl alcohol*2 | 5 | 5 | 5 |
| | NIKKOL Nikkomulese 41*3 | 5 | 5 | 5 |
| | Oleyl alcohol*4 | 1 | 1 | 1 |
| | NIKKOL LM Wax*5 | 1 | 1 | 1 |
| | Tocopherol*6 | 0.5 | 0.5 | 0.5 |
| | Olive oil*7 | 5 | 5 | 5 |
| | Dimethicone*8 | 5 | 5 | 5 |
| | White Vaseline*9 | 6 | 6 | 6 |
| B | Propylene glycol*10 | 4 | 4 | 4 |
| | Dipropylene glycol*11 | 5 | 5 | 5 |
| | Phenoxyethanol*12 | 0.1 | 0.1 | 0.1 |
| | Keratin*13 | 1.5 | 1.5 | 1.5 |
| C | Hydroxyethyl cellulose*14 | 0.2 | 0.2 | 0.2 |
| | Polyethylene glycol 400*15 | 0.08 | 0.08 | 0.08 |
| | Potassium hydroxide*16 | 0.05 | 0.05 | 0.05 |
| | Purified water | 54.47 | 54.07 | 54.57 |
| D | ES-01 premix | 0.1 | 0.5 | 0 |

(continued)

| | | Example 31 | Example 32 | Comparative Example 27 |
|---|---|---|---|---|
| Total | | 100 | 100 | 100 |
| *1: available from Nikko Chemicals Co., Ltd.<br>*2: available from KOKYU ALCOHOL KOGYO CO., LTD.<br>*3: available from Nikko Chemicals Co., Ltd.<br>*4: available from KOKYU ALCOHOL KOGYO CO., LTD.<br>*5: available from Nikko Chemicals Co., Ltd.<br>*6: RIKEN VITAMIN E OIL 700 available from RIKEN VITAMIN CO., LTD.<br>*7: available from Nikko Chemicals Co., Ltd.<br>*8: KF-96A-500CS available from Shin-Etsu Chemical Co., Ltd.<br>*9: available from JUNSEI CHEMICAL CO., LTD.<br>*10: available from FUJIFILM Wako Pure Chemical Corporation<br>*11: available from KOYO FINE CHEMICAL CORP.<br>*12: available from Tokyo Chemical Industry Co., Ltd.<br>*13: CASHMERE COAT available from ICHIMARU PHARCOS Co., Ltd.<br>*14: available from NACALAI TESQUE, INC.<br>*15: available from JUNSEI CHEMICAL CO., LTD.<br>*16: available from Wako Pure Chemical Industries, Ltd. | | | | |

[Example 33, Example 34, and Comparative Example 28; Evaluation of Keratin Content of Hair Treated with Conditioner]

In each of Example 33, Example 34, and Comparative Example 28, 1 g of each of the conditioners prepared in Example 31, Example 32, and Comparative Example 27 was applied with hands to one damaged hair bundle (about 10 cm, about 1 g) prepared by the same procedure as in (1) of Example 16, and then the hair was allowed to stand still for five minutes. Thereafter, the hair was washed with shaking in a 300 mL tall beaker charged with 300 mL of methanol (available from JUNSEI CHEMICAL CO., LTD.). Excess methanol was then removed with Kaydry (available from NIPPON PAPER CRECIA Co., LTD.), and the hair was dried at room temperature overnight. Each of the thus-prepared damaged hair samples was cut into a length of 1 cm. The quantification of keratin was performed with Keratin Orange $\alpha$-Keratin Assay Kit (available from Biocolor). Specifically, 5 mg of the human hair treated in each of the Example and Comparative Example was weighed in a 1.5 mL microtube (available from Eppendorf), and 1 mL of Digestion reagent was added to the microtube, followed by mixing with a vortex mixer for one hour. Reference standard ($\alpha$-Keratin 5.0 mg/mL) was diluted with Digestion reagent, to thereby prepare a calibration curve sample (0, 1, 2, 3, 4, 5 mg/mL). 200 $\mu$L of Neutralising solution (1M HCl) was added to 200 $\mu$L of each of the hair sample and the calibration curve sample, to thereby neutralize the pH of each solution. 50 $\mu$L of Dye reagent was added to the sample and mixed with a vortex mixer, and then the mixture was allowed to stand still for 30 minutes, to thereby allow reaction to proceed. For salting out of keratin extracted from each hair sample, 450 $\mu$L of Saturated solution ($(NH_4)_2SO_4$) was added to the sample. For removal of unbound Orange G, the sample was subjected to centrifugation at 12,000 rpm for 10 minutes. Subsequently, the supernatant was discarded, and 50 $\mu$L of Digestion reagent was added, followed by sufficient mixing with a vortex mixer for solubilization of keratin. Thereafter, 200 $\mu$L of each of the prepared samples was added to a 96 well plate, and absorbance (480 nm) was measured with a plate reader (available from TECAN). Net absorbance was calculated by subtracting the absorbance of the sample itself from the measured absorbance. The test results are shown in FIG. 20. The amount of keratin extracted from the hair of each of Example 33 and Example 34 was greater than that of keratin extracted from the hair of Comparative Example 28.

[Example 35 and Comparative Example 29; Preparation of Conditioner Containing 18-MEA]

A conditioner containing 18-methyl eicosanoic acid (18-MEA) in an amount of 1.5% by mass was prepared as shown in Table 25 below. Phase A was heated with stirring in a 200 mL beaker (available from HARIO Co., Ltd.) until the temperature reached 75°C. Phase B and phase C were mixed in a 200 mL beaker, and the mixture was heated with stirring until the temperature reached 75°C. Phase A was added to phase B + phase C, and the resultant mixture was subjected to emulsification treatment at 75°C with a homomixer (QUICK HOMO MIXER LR-1A available from MIZUHO INDUSTRIAL CO., LTD.) at 5,000 rpm for five minutes. Subsequently, phase D heated to 75°C was added to the emulsified mixture, and the mixture was stirred under heating at 75°C for five minutes. Thereafter, the mixture was cooled with stirring until the temperature reached 40°C. In all the aforementioned processes, the stirring was performed at 200

rpm.

Table 25

| | | Example 35 | Comparative Example 29 |
|---|---|---|---|
| A | NIKKOL CA-2580*1<br>Behenyl alcohol*2<br>NIKKOL Nikkomulese 41*3<br>Oleyl alcohol*4<br>NIKKOL LM Wax*5<br>Tocopherol*6<br>Olive oil*7<br>Dimethicone*8<br>White Vaseline*9 | 6 (g)<br>5<br>5<br>1<br>1<br>0.5<br>5<br>5<br>6 | 6 (g)<br>5<br>5<br>1<br>1<br>0.5<br>5<br>5<br>6 |
| B | Propylene glycol*10<br>Dipropylene glycol*11<br>Phenoxyethanol*12<br>18-MEA*13 | 4<br>5<br>0.1<br>1.5 | 4<br>5<br>0.1<br>1.5 |
| C | Hydroxyethyl cellulose*14<br>Polyethylene glycol 400*15<br>Potassium hydroxide*16<br>Purified water | 0.2<br>0.08<br>0.05<br>54.07 | 0.2<br>0.08<br>0.05<br>54.57 |
| D | ES-01 premix | 0.5 | 0 |
| Total | | 100 | 100 |

*1: available from Nikko Chemicals Co., Ltd.
*2: available from KOKYU ALCOHOL KOGYO CO., LTD.
*3: available from Nikko Chemicals Co., Ltd.
*4: available from KOKYU ALCOHOL KOGYO CO., LTD.
*5: available from Nikko Chemicals Co., Ltd.
*6: RIKEN VITAMIN E OIL 700 available from RIKEN VITAMIN CO., LTD.
*7: available from Nikko Chemicals Co., Ltd.
*8: KF-96A-500CS available from Shin-Etsu Chemical Co., Ltd.
*9: available from JUNSEI CHEMICAL CO., LTD.
*10: available from FUJIFILM Wako Pure Chemical Corporation
*11: available from KOYO FINE CHEMICAL CORP.
*12: available from Tokyo Chemical Industry Co., Ltd.
*13: Cutissential 18-MEA40 available from Croda Japan KK
*14: available from NACALAI TESQUE, INC.
*15: available from JUNSEI CHEMICAL CO., LTD.
*16: available from Wako Pure Chemical Industries, Ltd.

[Example 36 and Comparative Example 30; SEM Measurement of Hair Treated with Conditioner]

In each of Example 36 and Comparative Example 30, 1 g of each of the conditioners prepared in Example 35 and Comparative Example 29 was applied with hands to one damaged hair bundle (about 10 cm, about 1 g) prepared by the same procedure as in (1) of Example 16, and then the hair was allowed to stand still for five minutes. Thereafter, the hair was washed with shaking in a 300 mL tall beaker charged with 300 mL of purified water. Excess purified water was then removed with Kaydry (available from NIPPON PAPER CRECIA Co., LTD.), and the hair was dried at room temperature overnight. This operation was repeated 10 times, and then the surface of each of the above-prepared hair samples was observed with a Schottky field emission scanning electron microscope JSM-7800F (available from JEOL Ltd.). The sample was fixed with carbon tape, and the measurement was performed at an acceleration voltage of 0.7 kV. The results are shown in FIG. 21 together with the results of the damaged hair. As shown in the results of FIG. 21, the structure of a cuticle surface was observed in the damaged hair or Comparative Example 30, whereas a cuticle surface was covered with a coating film in Example 36.

## Claims

1. A lipidic peptide compound composed of a lipidic peptide or a pharmaceutically usable salt thereof, wherein the lipidic peptide compound is a compound prepared by bonding of a peptide moiety having an amino acid repeating structure to a lipidic moiety having a $C_{9-23}$ branched chain aliphatic group.

2. The lipidic peptide compound according to claim 1, **characterized in that** the lipidic peptide compound is selected from compounds of the following Formulae (1) to (3) or pharmaceutically usable salts of the compounds:

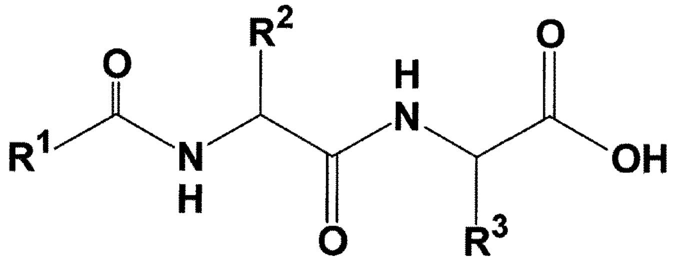

(1)

(wherein $R^1$ is a $C_{9-23}$ branched chain aliphatic group; $R^2$ is a hydrogen atom or a $C_{1-4}$ alkyl group optionally having a $C_1$ or $C_2$ branched chain; and $R^3$ is a $-(CH_2)_n-X$ group, wherein n is a number of 1 to 4, and X is an amino group, a guanidino group, a $-CONH_2$ group, or a 5-membered ring or a 6-membered ring optionally having one to three nitrogen atoms or a fused heterocyclic ring composed of the 5-membered ring and the 6-membered ring);

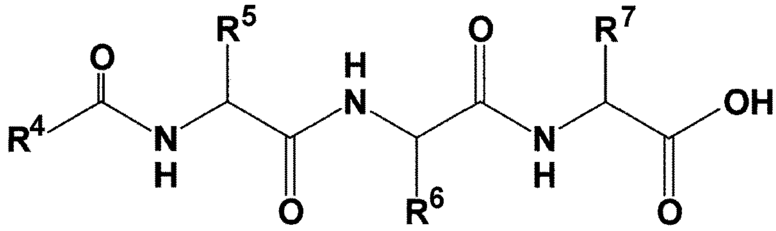

(2)

(wherein $R^4$ is a $C_{9-23}$ branched chain aliphatic group; and $R^5$ to $R^7$ are each independently a hydrogen atom, a $C_{1-4}$ alkyl group optionally having a $C_1$ or $C_2$ branched chain, or a $-(CH_2)_n-X$ group, wherein n is a number of 1 to 4, and X is an amino group, a guanidino group, a $-CONH_2$ group, or a 5-membered ring or a 6-membered ring optionally having one to three nitrogen atoms or a fused heterocyclic ring composed of the 5-membered ring and the 6-membered ring); and

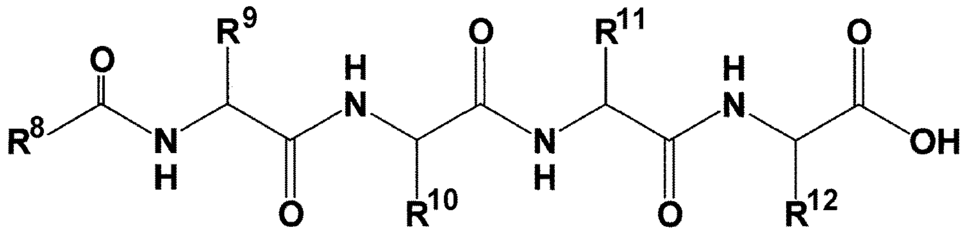

(3)

(wherein $R^8$ is a $C_{9-23}$ branched chain aliphatic group; and $R^9$ to $R^{12}$ are each independently a hydrogen atom, a $C_{1-4}$ alkyl group optionally having a $C_1$ or $C_2$ branched chain, or a $-(CH_2)_n-X$ group, wherein n is a number of 1 to 4, and X is an amino group, a guanidino group, a $-CONH_2$ group, or a 5-membered ring or a 6-membered ring optionally having one to three nitrogen atoms or a fused heterocyclic ring composed of the 5-membered ring and the 6-membered ring).

3. A composition **characterized by** comprising at least one lipidic peptide compound composed of a lipidic peptide or a pharmaceutically usable salt thereof, wherein the lipidic peptide compound is a compound prepared by bonding of a peptide moiety having an amino acid repeating structure to a lipidic moiety having a $C_{9-23}$ branched chain aliphatic group.

4. The composition according to claim 3, **characterized in that** the lipidic peptide compound is composed of at least one of compounds of the following Formulae (1) to (3) or pharmaceutically usable salts of the compounds:

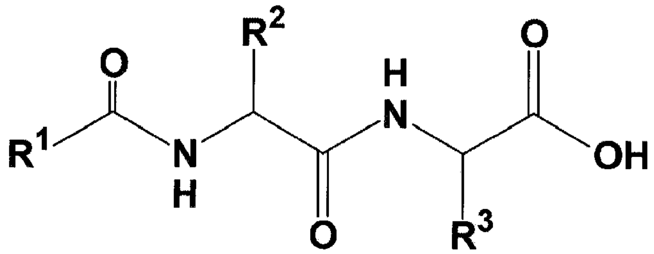

(1)

(wherein $R^1$ is a $C_{9-23}$ branched chain aliphatic group; $R^2$ is a hydrogen atom or a $C_{1-4}$ alkyl group optionally having a $C_1$ or $C_2$ branched chain; and $R^3$ is a $-(CH_2)_n-X$ group, wherein n is a number of 1 to 4, and X is an amino group, a guanidino group, a $-CONH_2$ group, or a 5-membered ring or a 6-membered ring optionally having one to three nitrogen atoms or a fused heterocyclic ring composed of the 5-membered ring and the 6-membered ring);

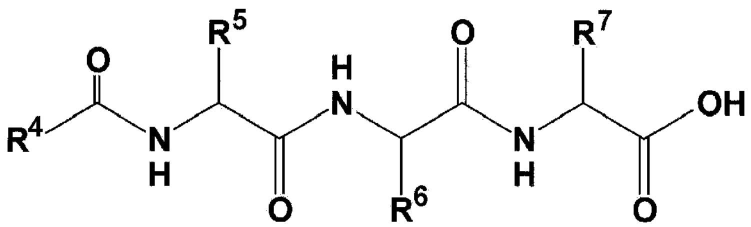

(2)

(wherein $R^4$ is a $C_{9-23}$ branched chain aliphatic group; and $R^5$ to $R^7$ are each independently a hydrogen atom, a $C_{1-4}$ alkyl group optionally having a $C_1$ or $C_2$ branched chain, or a $-(CH_2)_n-X$ group, wherein n is a number of 1 to 4, and X is an amino group, a guanidino group, a $-CONH_2$ group, or a 5-membered ring or a 6-membered ring optionally having one to three nitrogen atoms or a fused heterocyclic ring composed of the 5-membered ring and the 6-membered ring); and

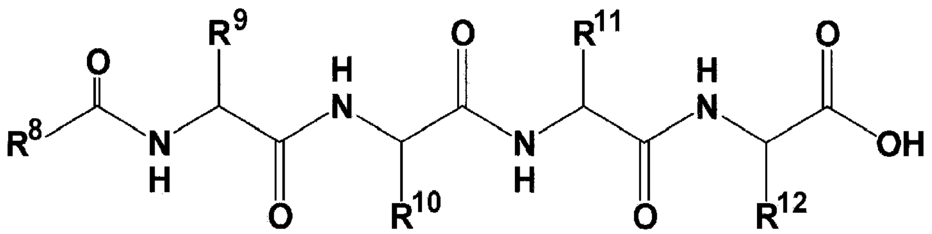

(3)

(wherein $R^8$ is a $C_{9-23}$ branched chain aliphatic group; and $R^9$ to $R^{12}$ are each independently a hydrogen atom, a $C_{1-4}$ alkyl group optionally having a $C_1$ or $C_2$ branched chain, or a $-(CH_2)_n-X$ group, wherein n is a number of 1 to 4, and X is an amino group, a guanidino group, a $-CONH_2$ group, or a 5-membered ring or a 6-membered ring optionally having one to three nitrogen atoms or a fused heterocyclic ring composed of the 5-membered ring and the 6-membered ring).

5. The composition according to claim 3 or 4, **characterized by** further containing a polyhydric alcohol.

6. The composition according to any one of claims 3 to 5, **characterized by** further containing a surfactant.

7. The composition according to any one of claims 3 to 6, that is used for coating film formation.

8. A method for producing a coating film, the method being **characterized by** comprising using the composition according to any one of claims 3 to 6.

9. A coating film formed from the coating film-forming composition according to any one of claims 3 to 6.

FIG. 1

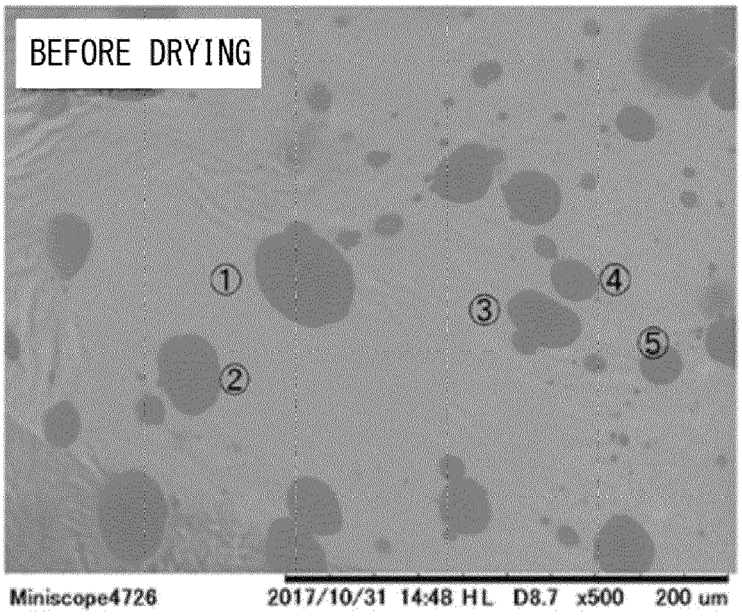
BEFORE DRYING
①
②
③
④
⑤
Miniscope4726
2017/10/31 14:48 HL D8.7 x500 200 um

(a)

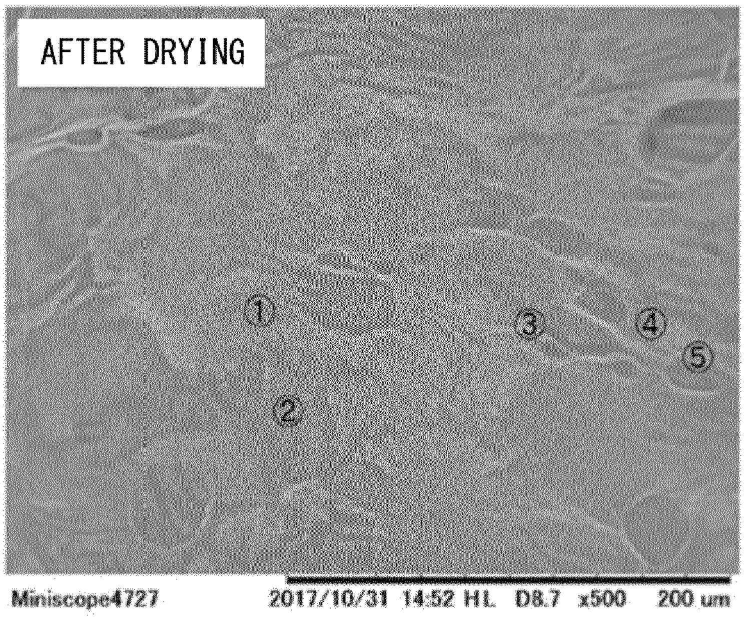
AFTER DRYING
①
②
③
④
⑤
Miniscope4727
2017/10/31 14:52 HL D8.7 x500 200 um

(b)

FIG. 2

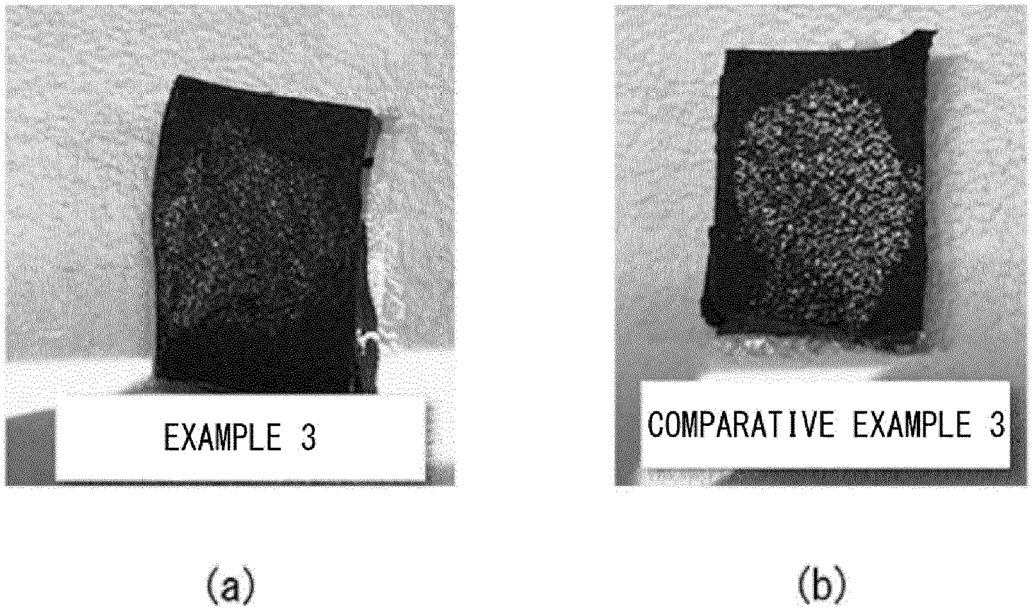

FIG. 3

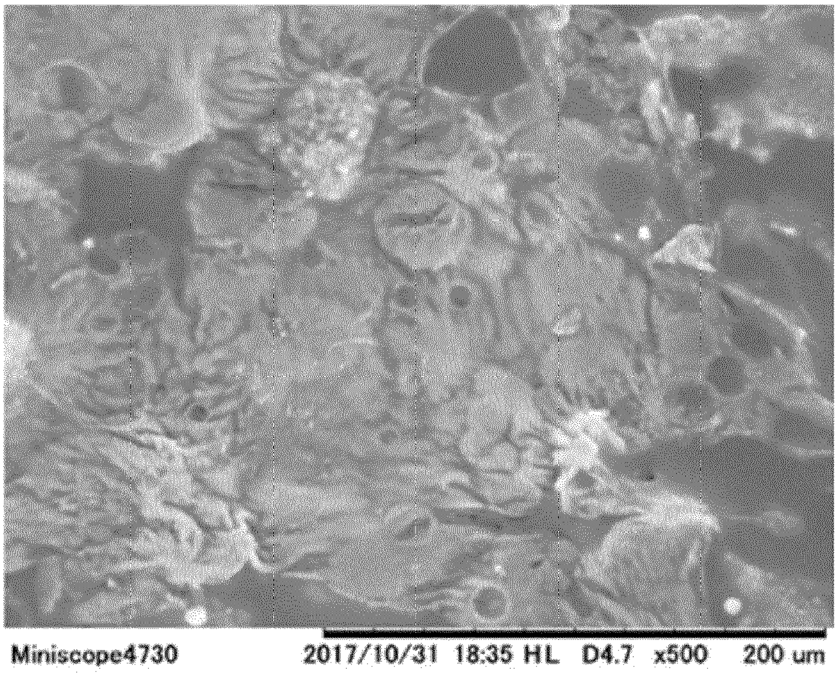
Miniscope4730
2017/10/31 18:35 HL D4.7 x500 200 um

(a)

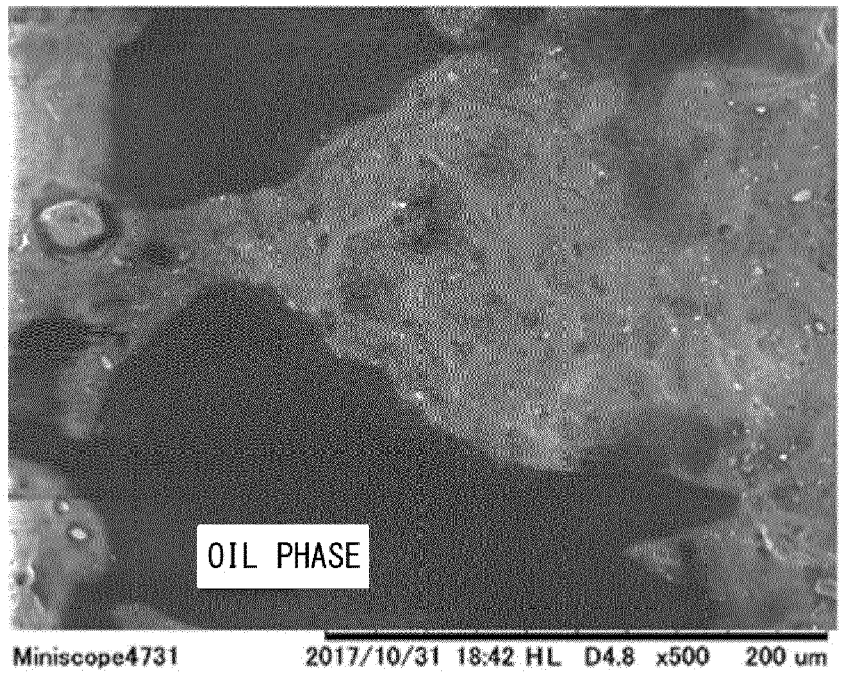
OIL PHASE
Miniscope4731
2017/10/31 18:42 HL D4.8 x500 200 um

(b)

FIG. 4

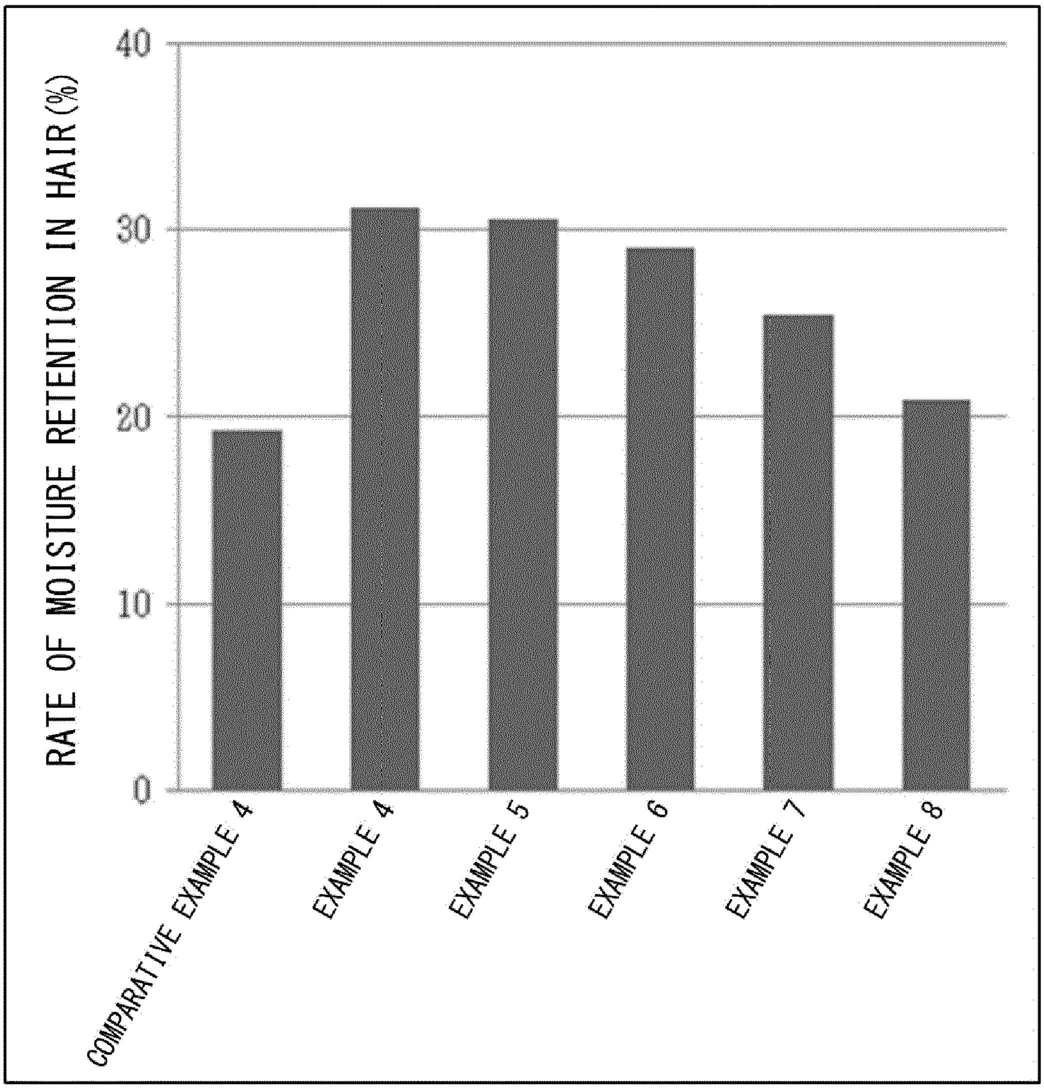
RATE OF MOISTURE RETENTION IN HAIR (%)
40
30
20
10
0
COMPARATIVE EXAMPLE 4
EXAMPLE 4
EXAMPLE 5
EXAMPLE 6
EXAMPLE 7
EXAMPLE 8

FIG. 5

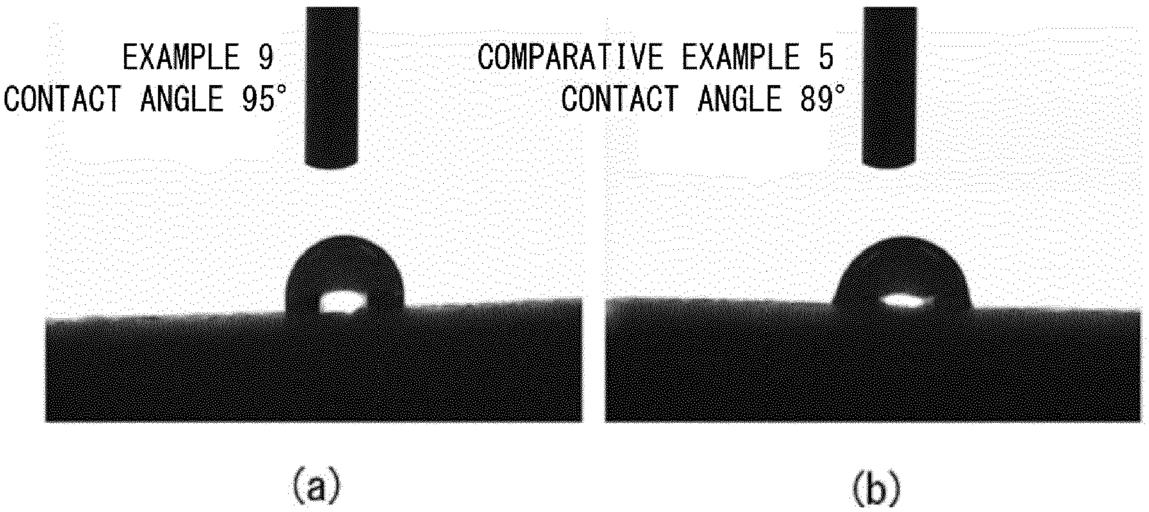
EXAMPLE 9
CONTACT ANGLE 95°
COMPARATIVE EXAMPLE 5
CONTACT ANGLE 89°
(a)
(b)

FIG. 6

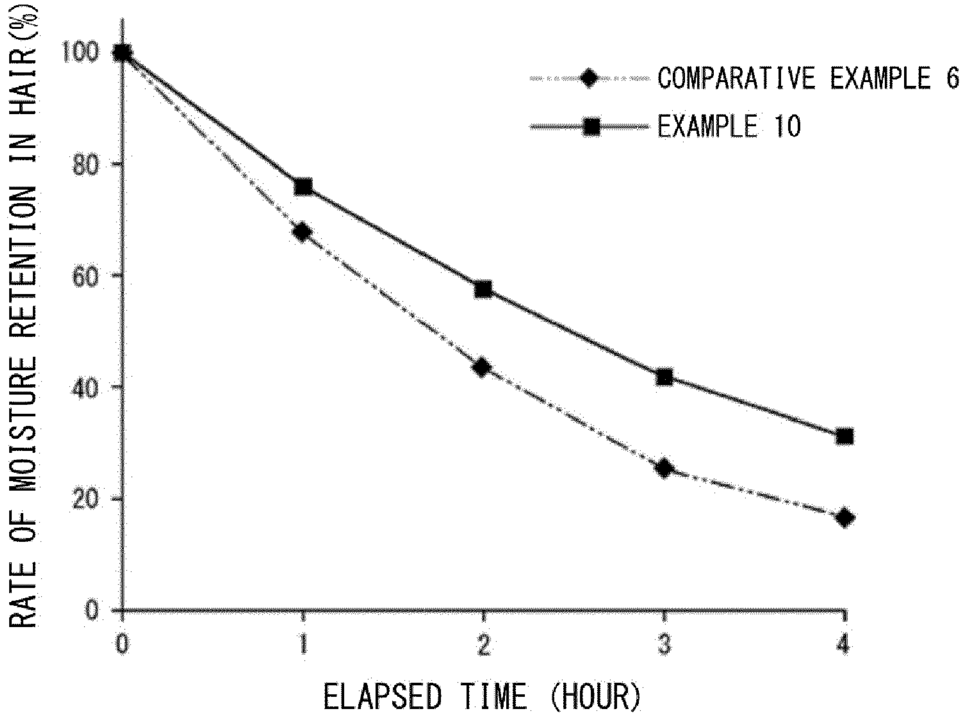
RATE OF MOISTURE RETENTION IN HAIR (%)
100
80
60
40
20
0
0
1
2
3
4
ELAPSED TIME (HOUR)
COMPARATIVE EXAMPLE 6
EXAMPLE 10

FIG. 7

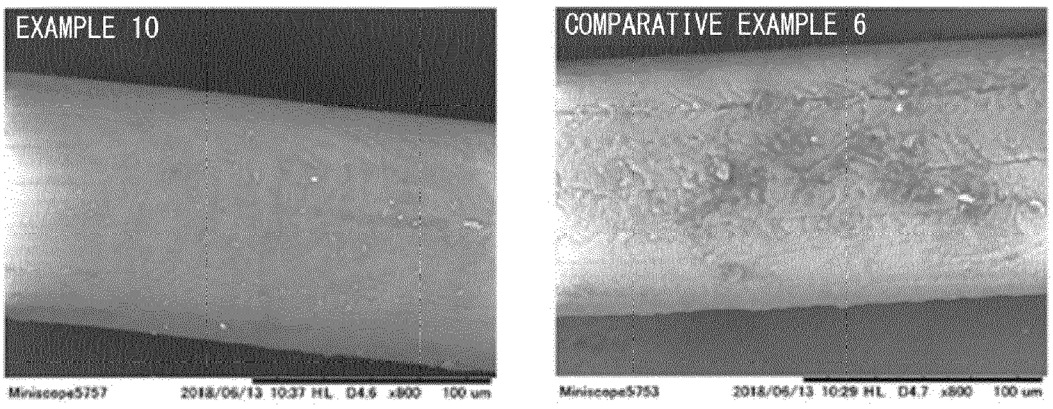
EXAMPLE 10
COMPARATIVE EXAMPLE 6

(a) (b)

FIG. 8

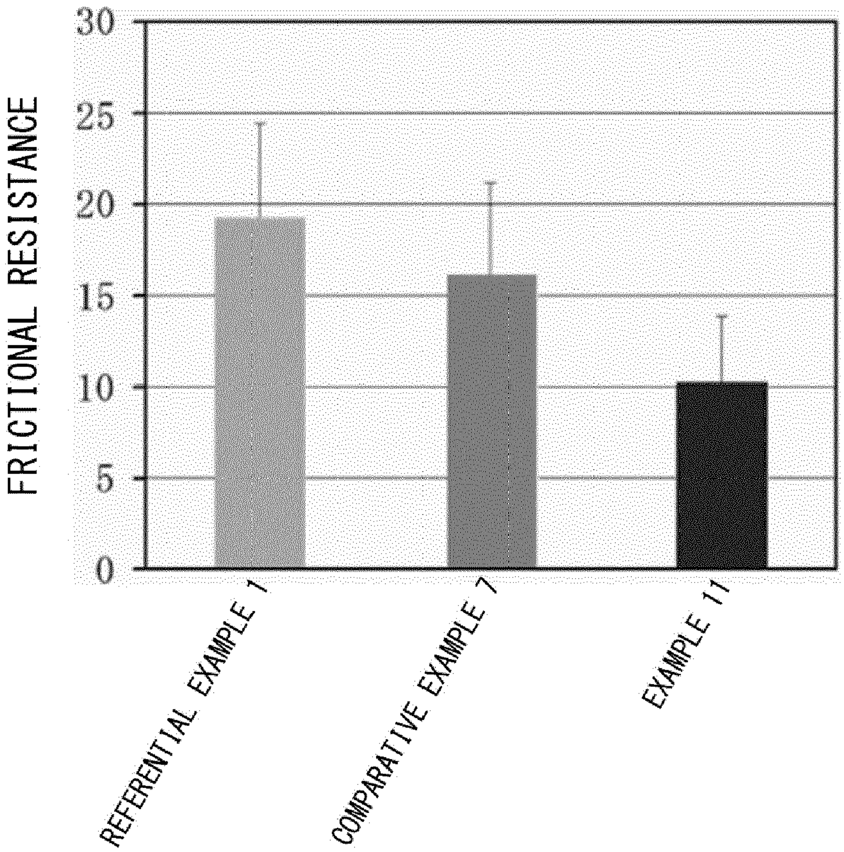
FRICTIONAL RESISTANCE
30
25
20
15
10
5
0
REFERENTIAL EXAMPLE 1
COMPARATIVE EXAMPLE 7
EXAMPLE 11

FIG. 9

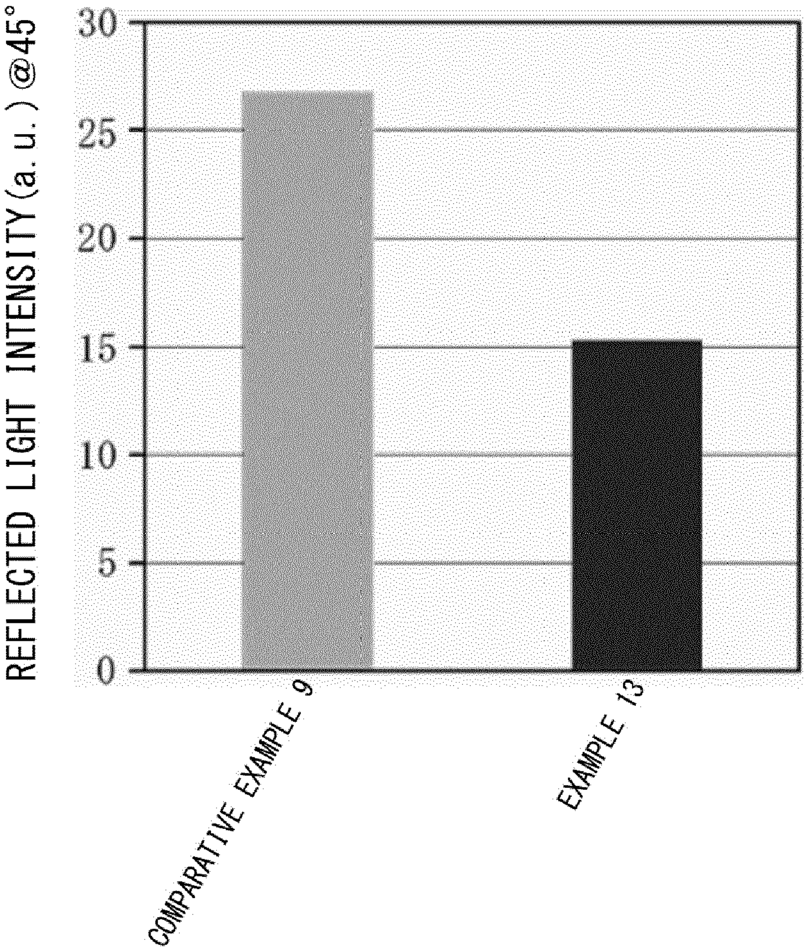


FIG. 10

COMPARATIVE EXAMPLE 10

EXAMPLE 14

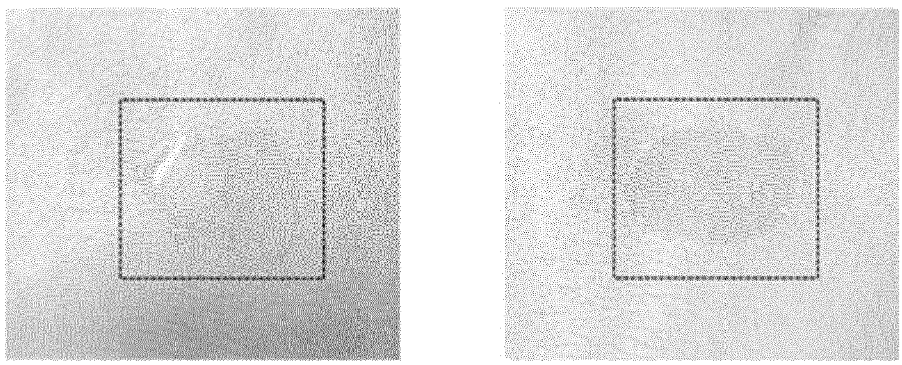

(a)

(b)

FIG. 11

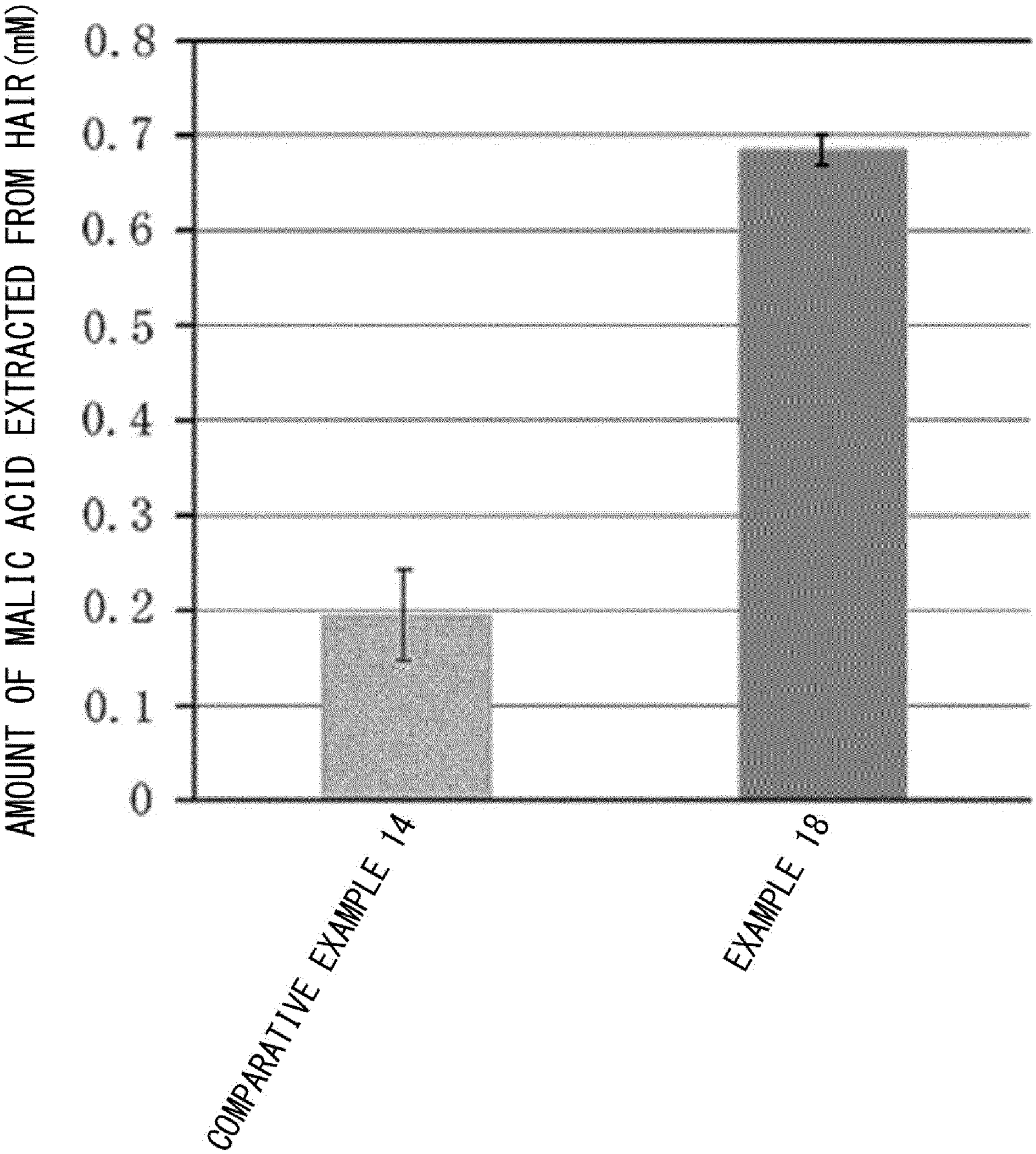
AMOUNT OF MALIC ACID EXTRACTED FROM HAIR (mM)
0.8
0.7
0.6
0.5
0.4
0.3
0.2
0.1
0
COMPARATIVE EXAMPLE 14
EXAMPLE 18

FIG. 12

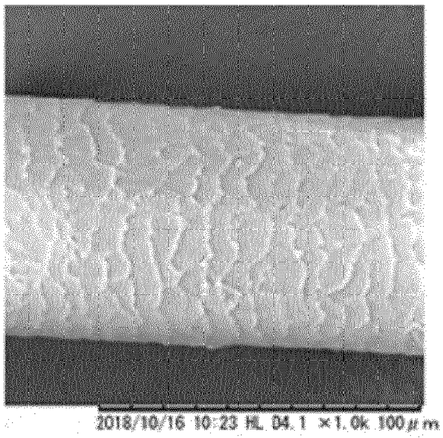


(a)
DAMAGED HAIR

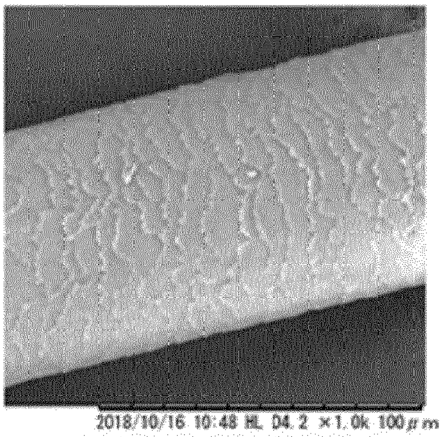


(b)
EXAMPLE 19

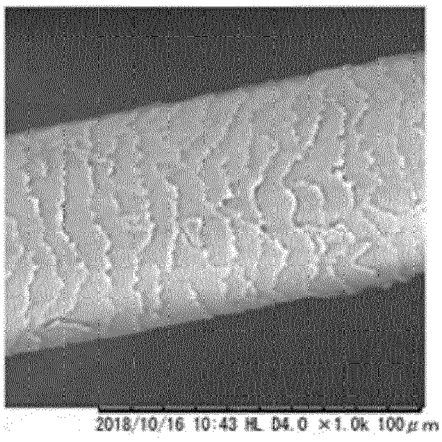


(c)
COMPARATIVE EXAMPLE 15

FIG. 13

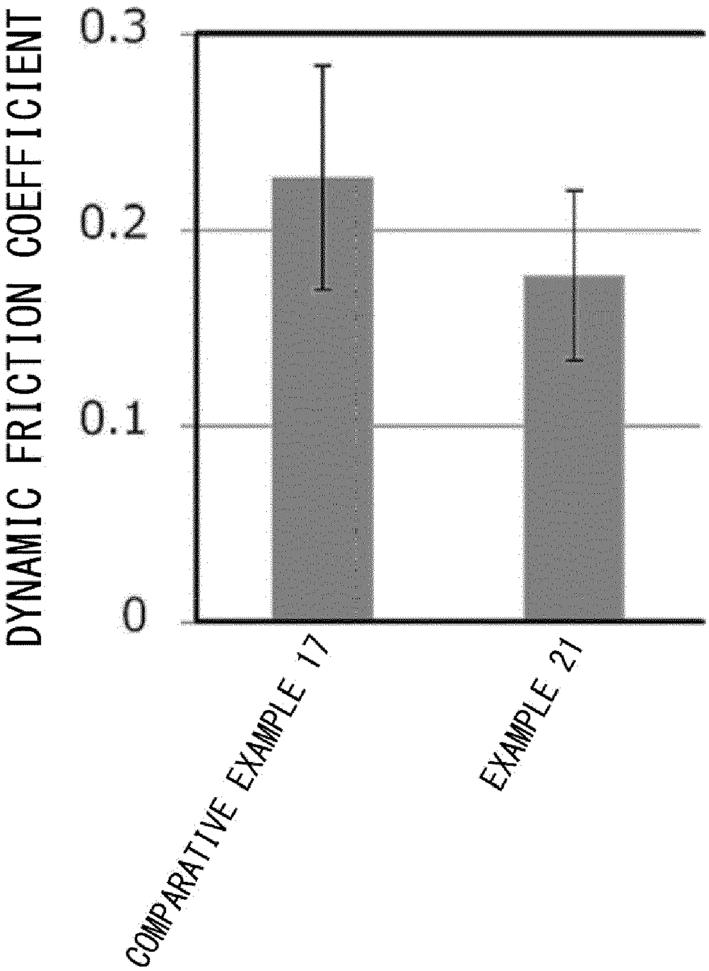
DYNAMIC FRICTION COEFFICIENT
0.3
0.2
0.1
0
COMPARATIVE EXAMPLE 17
EXAMPLE 21

FIG. 14

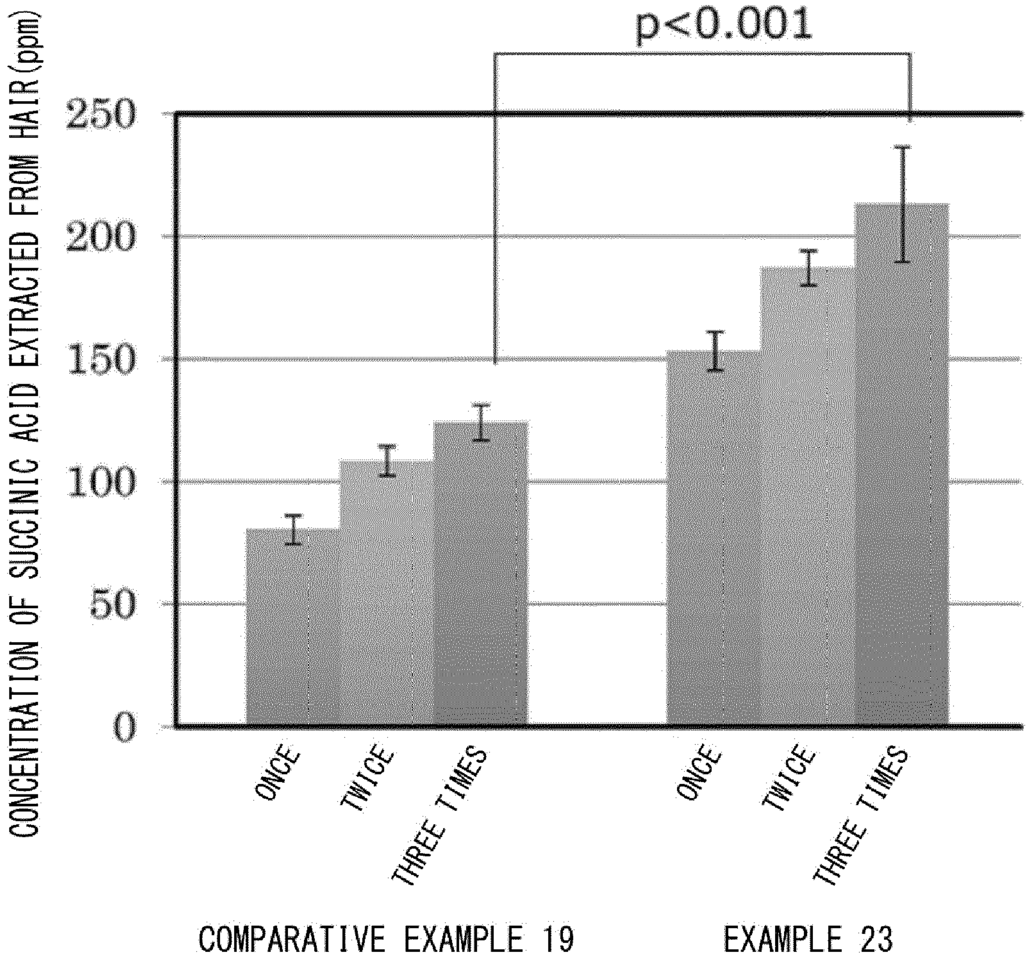
p<0.001
CONCENTRATION OF SUCCINIC ACID EXTRACTED FROM HAIR (ppm)
250
200
150
100
50
0
ONCE
TWICE
THREE TIMES
ONCE
TWICE
THREE TIMES
COMPARATIVE EXAMPLE 19
EXAMPLE 23

FIG. 15

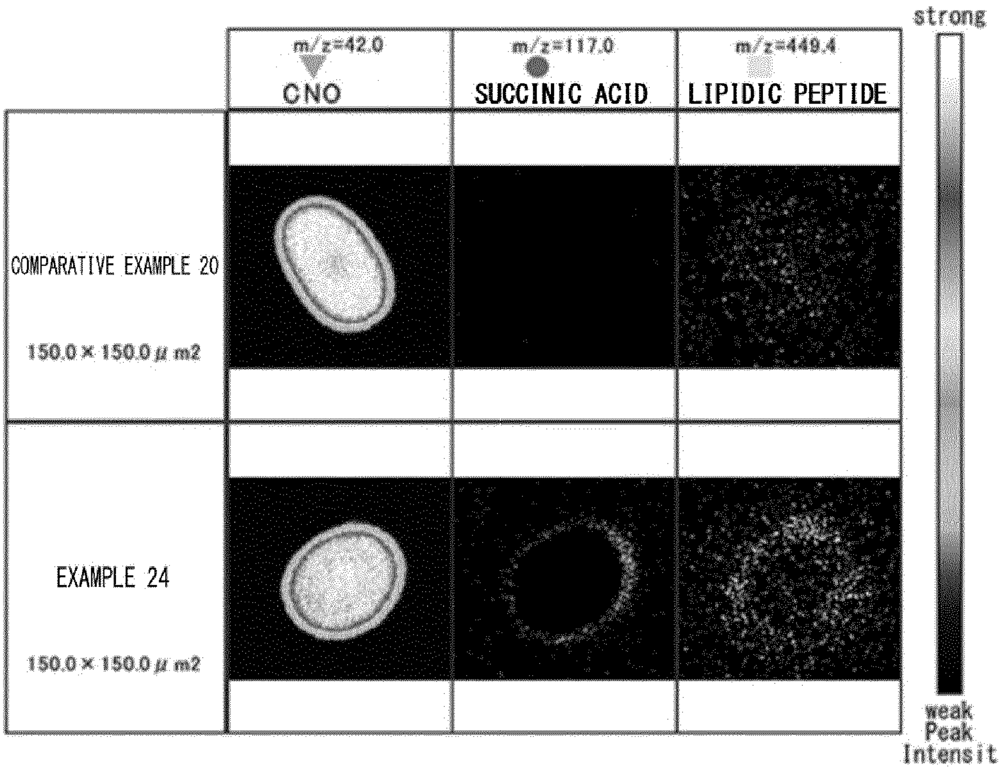
m/z=42.0
CNO
m/z=117.0
SUCCINIC ACID
m/z=449.4
LIPIDIC PEPTIDE
strong
COMPARATIVE EXAMPLE 20
150.0 × 150.0 μ m2
EXAMPLE 24
150.0 × 150.0 μ m2
weak
Peak
Intensit

FIG. 16

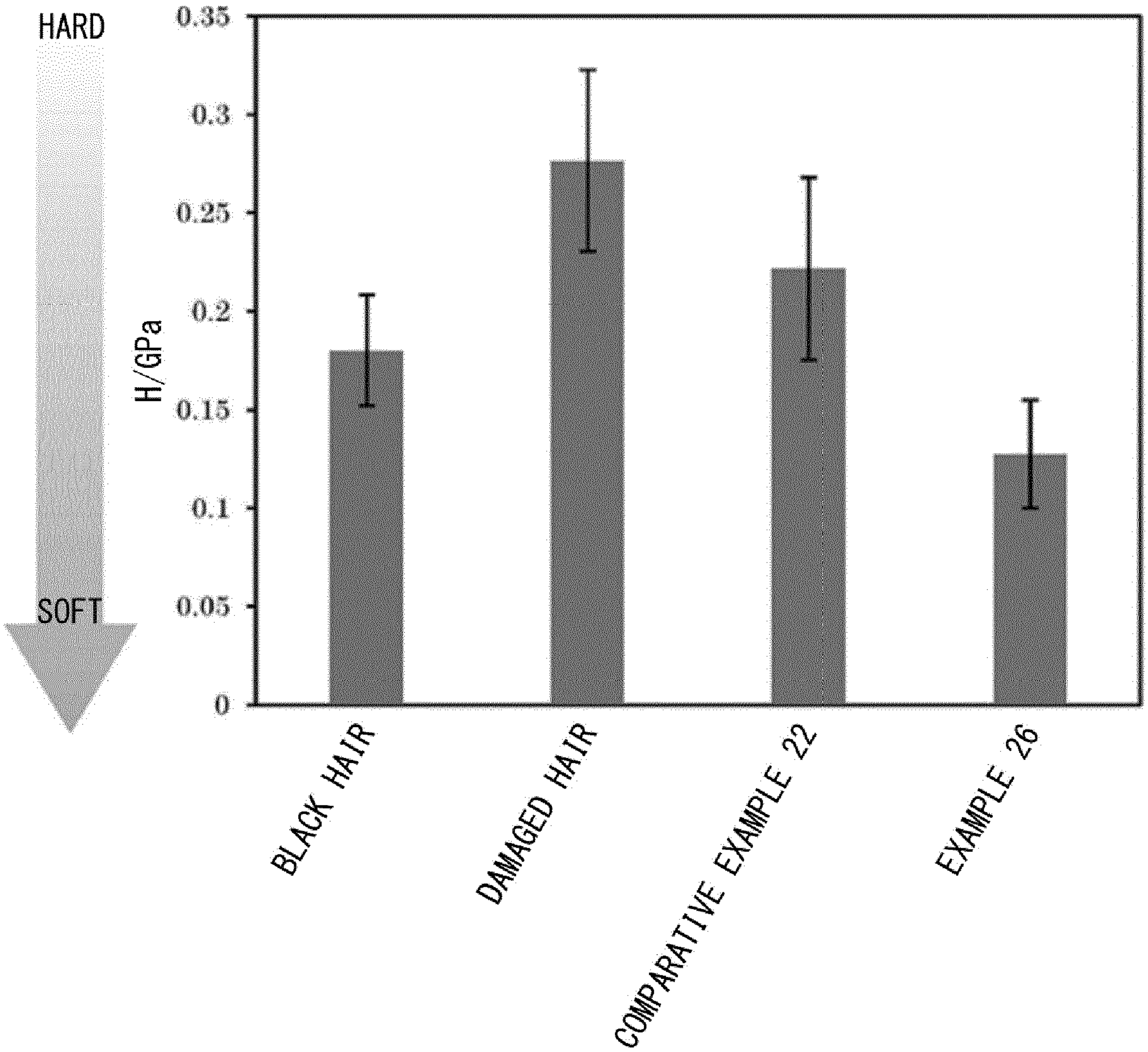
HARD
SOFT
H/GPa
0.35
0.3
0.25
0.2
0.15
0.1
0.05
0
BLACK HAIR
DAMAGED HAIR
COMPARATIVE EXAMPLE 22
EXAMPLE 26

FIG. 17

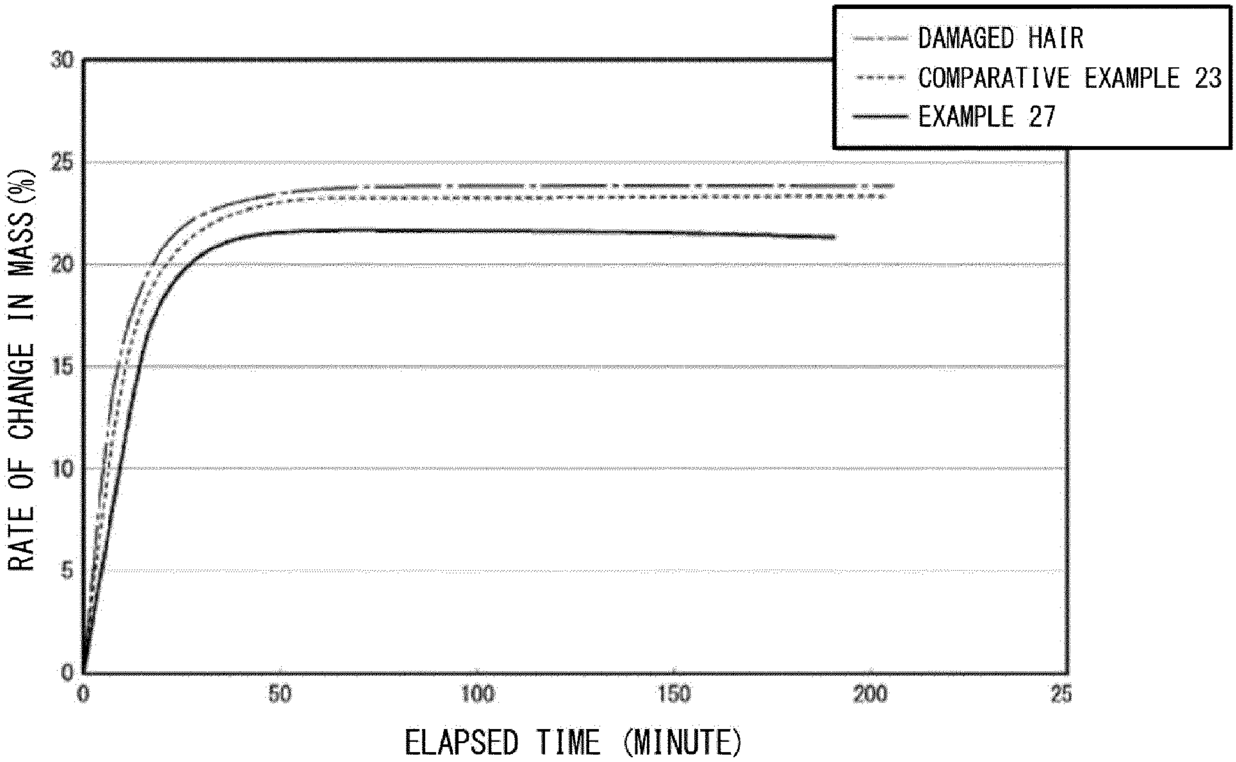
DAMAGED HAIR
COMPARATIVE EXAMPLE 23
EXAMPLE 27
RATE OF CHANGE IN MASS (%)
0
5
10
15
20
25
30
0
50
100
150
200
25
ELAPSED TIME (MINUTE)

FIG. 18

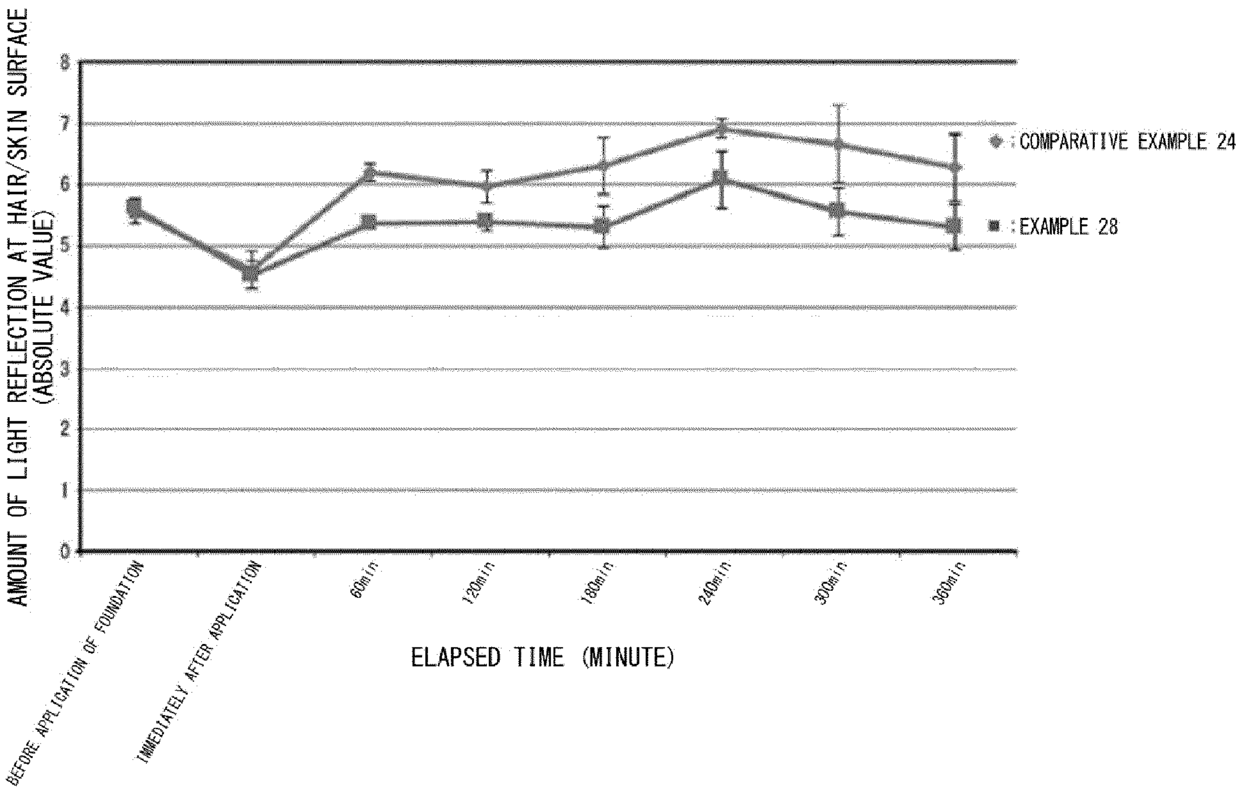
AMOUNT OF LIGHT REFLECTION AT HAIR/SKIN SURFACE (ABSOLUTE VALUE)
0
1
2
3
4
5
6
7
8
◆ : COMPARATIVE EXAMPLE 24
■ : EXAMPLE 28
BEFORE APPLICATION OF FOUNDATION
IMMEDIATELY AFTER APPLICATION
60min
120min
180min
240min
300min
360min
ELAPSED TIME (MINUTE)

FIG. 19

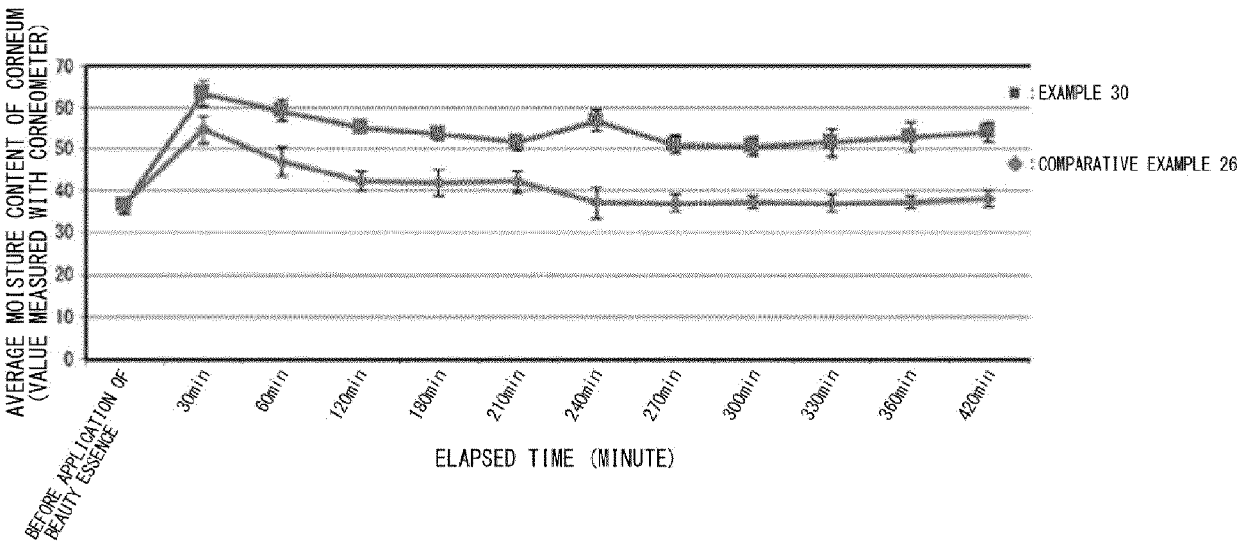
AVERAGE MOISTURE CONTENT OF CORNEUM (VALUE MEASURED WITH CORNEOMETER)
70
60
50
40
30
20
10
0
■ : EXAMPLE 30
◆ : COMPARATIVE EXAMPLE 26
BEFORE APPLICATION OF BEAUTY ESSENCE
30min
60min
120min
180min
210min
240min
270min
300min
330min
360min
420min
ELAPSED TIME (MINUTE)

FIG. 20

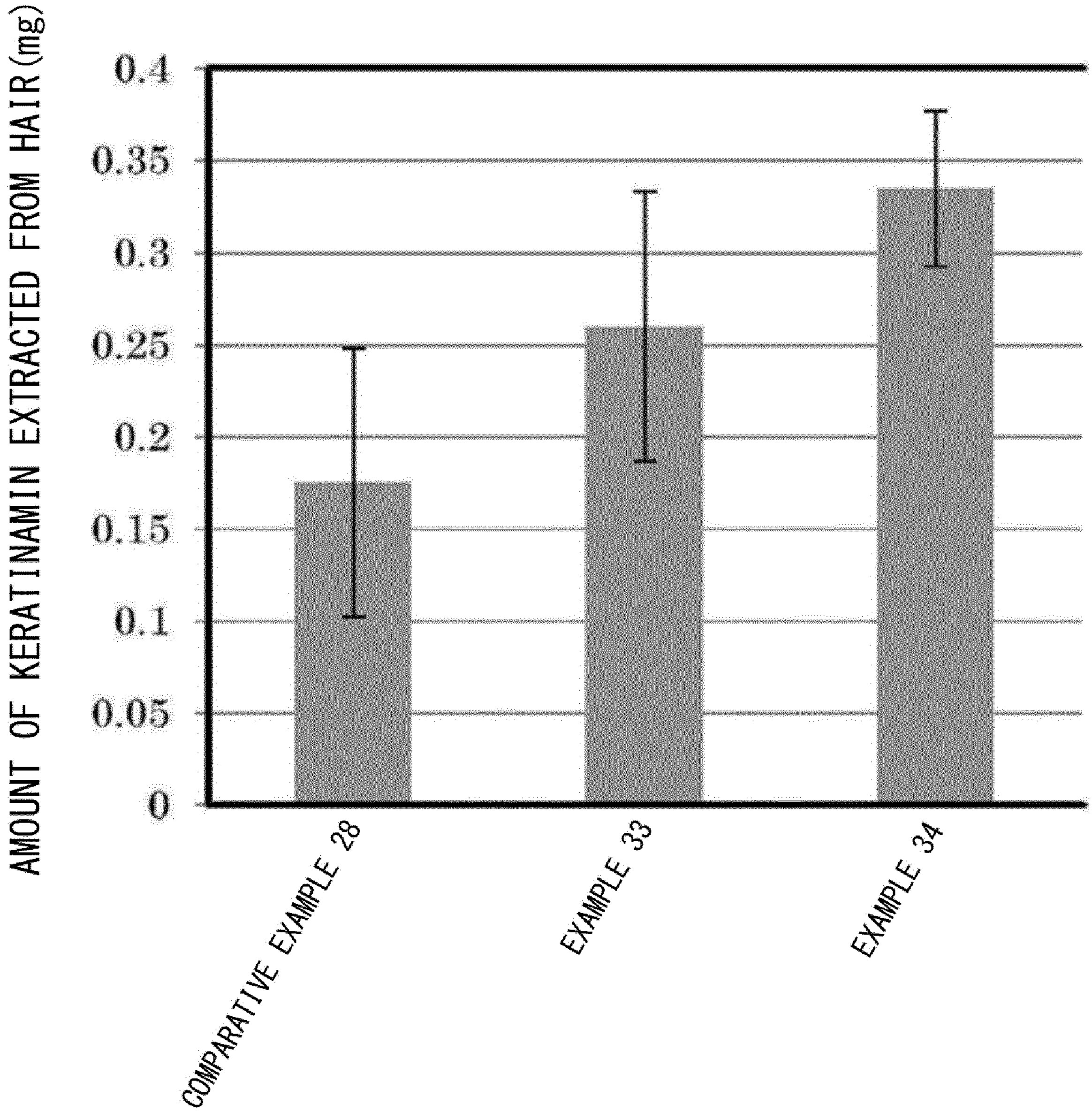
AMOUNT OF KERATINAMIN EXTRACTED FROM HAIR (mg)
0.4
0.35
0.3
0.25
0.2
0.15
0.1
0.05
0
COMPARATIVE EXAMPLE 28
EXAMPLE 33
EXAMPLE 34

FIG. 21

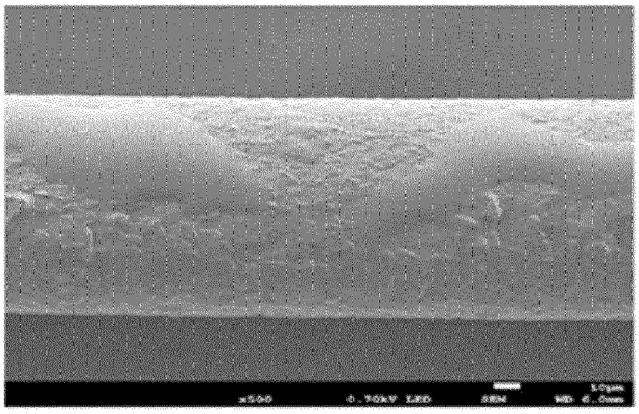

(a)
EXAMPLE 36

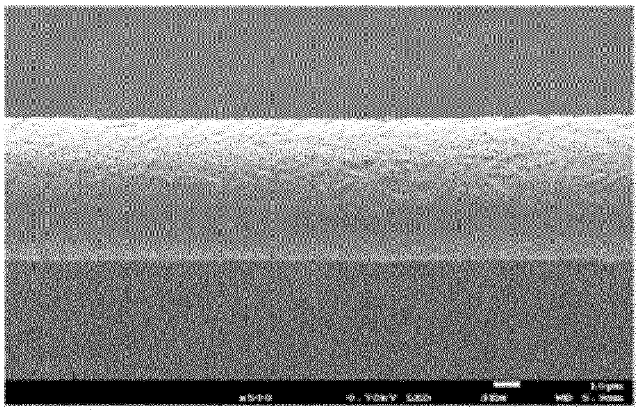

(b)
COMPARATIVE EXAMPLE 30

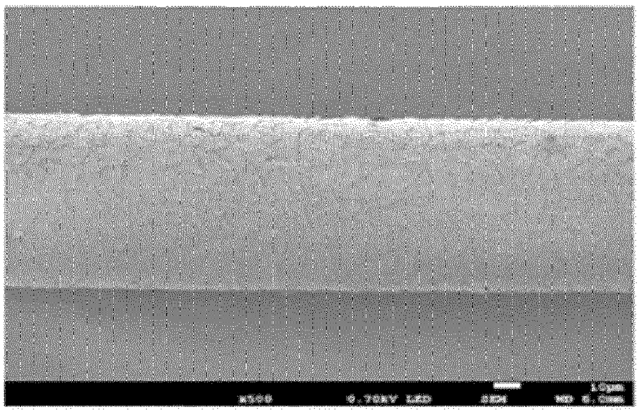

(c)
DAMAGED HAIR

Europäisches Patentamt
European Patent Office
Office européen des brevets

# EUROPEAN SEARCH REPORT

Application Number
EP 22 15 5607

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | EP 2 410 031 A1 (NISSAN CHEMICAL IND LTD [JP]; UNIV KYUSHU [JP]) 25 January 2012 (2012-01-25) * formulae (1) and (2); claims 11-13; examples * ----- | 1-9 | INV. A61K8/64 A61Q5/12 A61Q19/00 A61K8/36 A61K8/362 A61K8/365 A61Q1/02 A61Q5/02 A61Q5/06 A61Q19/10 C07K5/06 C09D177/04 |
| A | EP 2 319 894 A1 (NISSAN CHEMICAL IND LTD [JP]) 11 May 2011 (2011-05-11) * formulae (1) to (3); claim 24; examples * ----- | 1-9 | |
| A | US 2017/319462 A1 (MARCHANT NANCY S [US] ET AL) 9 November 2017 (2017-11-09) * claims 1,50,53,58 * ----- | 1-9 | |
| A | US 2008/181974 A1 (CAUCHARD JEAN-HUBERT [FR] ET AL) 31 July 2008 (2008-07-31) * example 5e * ----- | 1-9 | |
| A | CN 106 109 358 A (ANDIMEI BIOTECHNOLOGY CO LTD) 16 November 2016 (2016-11-16) * claims * ----- | 1-9 | TECHNICAL FIELDS SEARCHED (IPC) |
| X | ALMEIDA CARINA S. ET AL: "Tailoring Cellular Uptake of Conjugated Polymer Nanoparticles Using Modular Amphiphilic Peptide Capping Ligands", CHEMISTRY OF MATERIALS, vol. 27, no. 19, 17 September 2015 (2015-09-17), pages 6879-6889, XP055920794, US ISSN: 0897-4756, DOI: 10.1021/acs.chemmater.5b03337 * page 6881, column 2, paragraph 2nd; figure 1B * ----- | 1,3,7-9 | A61K A61Q C07K C09J C09D |

The present search report has been drawn up for all claims

1

| Place of search | Date of completion of the search | Examiner |
|---|---|---|
| Munich | 13 May 2022 | Werner, Stefan |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT ON EUROPEAN PATENT APPLICATION NO. EP 22 15 5607

This annex lists the patent family members relating to the patent documents cited in the above-mentioned European search report. The members are as contained in the European Patent Office EDP file on
The European Patent Office is in no way liable for these particulars which are merely given for the purpose of information.

13-05-2022

| Patent document cited in search report | Publication date | Patent family member(s) | Publication date |
|---|---|---|---|
| EP 2410031 A1 | 25-01-2012 | CN 102361951 A | 22-02-2012 |
| | | EP 2410031 A1 | 25-01-2012 |
| | | JP 5587290 B2 | 10-09-2014 |
| | | JP WO2010106981 A1 | 20-09-2012 |
| | | KR 20110127279 A | 24-11-2011 |
| | | TW 201100531 A | 01-01-2011 |
| | | US 2012035108 A1 | 09-02-2012 |
| | | WO 2010106981 A1 | 23-09-2010 |
| EP 2319894 A1 | 11-05-2011 | CN 102105553 A | 22-06-2011 |
| | | EP 2319894 A1 | 11-05-2011 |
| | | JP 5500071 B2 | 21-05-2014 |
| | | JP 5954548 B2 | 20-07-2016 |
| | | JP 6065245 B2 | 25-01-2017 |
| | | JP 2014080428 A | 08-05-2014 |
| | | JP 2016138247 A | 04-08-2016 |
| | | JP WO2010013555 A1 | 12-01-2012 |
| | | KR 20110055593 A | 25-05-2011 |
| | | KR 20170019488 A | 21-02-2017 |
| | | TW 201016832 A | 01-05-2010 |
| | | TW 201446947 A | 16-12-2014 |
| | | TW 201446948 A | 16-12-2014 |
| | | US 2011183913 A1 | 28-07-2011 |
| | | US 2013267609 A1 | 10-10-2013 |
| | | US 2013267610 A1 | 10-10-2013 |
| | | WO 2010013555 A1 | 04-02-2010 |
| US 2017319462 A1 | 09-11-2017 | BR 112017008810 A2 | 20-02-2018 |
| | | CA 2965525 A1 | 06-05-2016 |
| | | CN 107108837 A | 29-08-2017 |
| | | CR 20170179 A | 20-07-2017 |
| | | EP 3212688 A2 | 06-09-2017 |
| | | KR 20170077215 A | 05-07-2017 |
| | | SG 11201703214S A | 30-05-2017 |
| | | TW 201628599 A | 16-08-2016 |
| | | US 2017319462 A1 | 09-11-2017 |
| | | WO 2016069396 A2 | 06-05-2016 |
| US 2008181974 A1 | 31-07-2008 | DE 102008006404 A1 | 14-08-2008 |
| | | FR 2911779 A1 | 01-08-2008 |
| | | GB 2446260 A | 06-08-2008 |
| | | JP 2008189669 A | 21-08-2008 |
| | | KR 20080071517 A | 04-08-2008 |
| | | US 2008181974 A1 | 31-07-2008 |
| CN 106109358 A | 16-11-2016 | NONE | |

EPO FORM P0459

For more details about this annex : see Official Journal of the European Patent Office, No. 12/82

page 1 of 2

## ANNEX TO THE EUROPEAN SEARCH REPORT ON EUROPEAN PATENT APPLICATION NO.

EP 22 15 5607

This annex lists the patent family members relating to the patent documents cited in the above-mentioned European search report. The members are as contained in the European Patent Office EDP file on
The European Patent Office is in no way liable for these particulars which are merely given for the purpose of information.

13-05-2022

| Patent document cited in search report | Publication date | Patent family member(s) | Publication date |
|---|---|---|---|
| ------------------------------------------------------------------------ | | | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2016006030 A
- JP H1077210 A
- JP 10077210 A
- JP 2002308756 A